(19) Europäisches Patentamt
European Patent Office
Office européen des brevets

(11) Numéro de publication: 0 241 395
A2

(12) # DEMANDE DE BREVET EUROPEEN

(21) Numéro de dépôt: 87420095.9

(22) Date de dépôt: 07.04.87

(51) Int. Cl.³: C 07 C 129/00
A 23 L 1/236

(30) Priorité: 10.04.86 FR 8605320
07.11.86 FR 8615788
23.12.86 FR 8618233

(43) Date de publication de la demande:
14.10.87 Bulletin 87/42

(84) Etats contractants désignés:
AT BE CH DE ES FR GB GR IT LI LU NL SE

(71) Demandeur: UNIVERSITE CLAUDE BERNARD - LYON 1
43, boulevard du 11 Novembre 1918
F-69622 Villeurbanne Cédex(FR)

(72) Inventeur: Nofre, Claude
119 cours Albert Thomas
F-69003 Lyon(FR)

(72) Inventeur: Tinti, Jean-Marie
5 avenue de Grenoble
F-69330 Meyzieu(FR)

(72) Inventeur: Chatzopoulos-Ouar Farroudja
14 rue Raoul Follereau
F-42100 Saint Etienne(FR)

(74) Mandataire: Laurent, Michel et al,
Cabinet Michel Laurent 20, rue Louis Chirpaz B.P. 32
F-69131 Ecully Cédex(FR)

(54) **Agents édulcorants dérivés des acides n-phénylguanidinoacétique et n-phényléthanamidinoacétique et compositions contenant de tels agents édulcorants.**

(57) Agents édulcorants de formule générale :

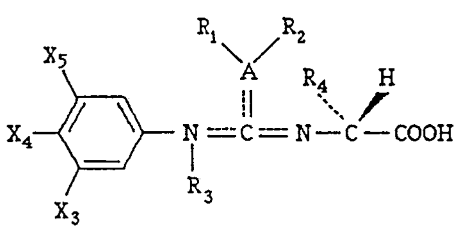

dans laquelle, dans une forme de réalisation préférée, $X_3$ et $X_5$ sont H, $CF_3$, $CH_3$, ou un halogène, $X_4$ est H, CN ou F ; dans laquelle A est N ou C ; dans laquelle $R_1$ est, dans une forme de réalisation préférée, H ou $CH_3$ ; dans laquelle $R_2$ est un groupe hydrocarboné de 2 à 13 atomes de carbone, dans lequel jusqu'à 4 atomes de carbone peuvent être remplacés par des atomes d'azote, oxygène, soufre, chlore, brome et iode et jusqu'à 5 atomes d'hydrogène par des atomes de fluor ; dans laquelle, dans une forme de réalisation préférée, $R_3$ et $R_4$ sont H.

EP 0 241 395 A2

# AGENTS EDULCORANTS DERIVES DES ACIDES N-PHENYLGUANIDINOACETIQUE ET N-PHENYLETHANAMIDINOACETIQUE ET COMPOSITIONS CONTENANT DE TELS AGENTS EDULCORANTS.

La présente invention concerne de nouveaux agents édulcorants utiles, notamment, pour édulcorer les aliments, les boissons, les confiseries, les pâtisseries, le chewing gum, les produits d'hygiène, les cosmétiques, les articles de toilette, les produits pharmaceutiques et vétérinaires, et leurs équivalents. Elle concerne aussi des préparations et des compositions contenant de tels agents édulcorants.

Dans la demande de brevet européen EP-A-0 195 730 du Demandeur, on a décrit, comme nouveaux agents édulcorants, des composés de formule :

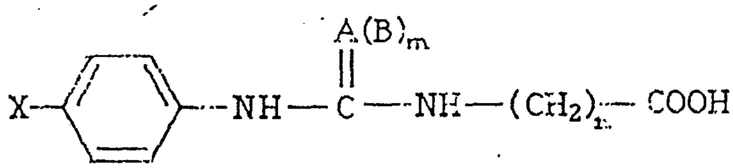

dans laquelle :

- A est :
  - N et
  - C ;

- m et n sont 1 ou 2 ;

- B est,
  - . quand n = 1 :
    - H,
    - CN,
    - $NO_2$,
    - $OCH_3$,
    - SOR,

$SO_2R$,

$SO_2NHR$ et

$SO_2N(R)_2$, R étant un groupe alkyle, cycloalkyle ou aryle ayant jusqu'à 10 atomes de carbone, 1 ou 2 atomes de carbone pouvant être remplacés par 1 ou 2 atomes de soufre ou d'oxygène,

. quand n = 2 :

H,

CN et

$OCH_3$ ;

- X, qui est en position 4, est :

CN et

$NO_2$, quand B est H, CN et $OCH_3$,

et est :

H,

$CF_3$,

CHO,

Cl,

CN,

$COCH_3$,

F et

$NO_2$, quand B est $NO_2$, SOR, $SO_2R$, $SO_2NHR$ et $SO_2N(R)_2$.

Ces composés ont pour principal inconvénient de présenter une stabilité assez faible dans des conditions acides de pH et/ou à la chaleur. Cet inconvénient empêche leur utilisation comme agents édulcorants dans certains aliments, soit ayant des valeurs basses de pH, soit préparés ou servis à des températures élevées. Les agents édulcorants de la présente invention pallient cet inconvénient.

Les agents édulcorants de la présente invention se caractérisent en ce qu'ils répondent à la formule générale :

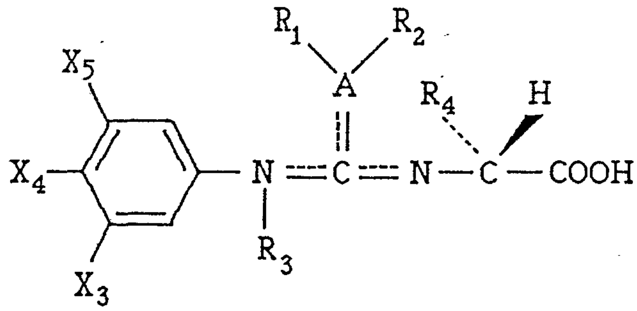

en incluant les formes tautomères et les sels physiologiquement acceptables,

- dans laquelle $X_3$, $X_4$ et $X_5$ sont choisis dans le groupe comprenant :

H,
Br,
$CF_3$,
$CF_2CF_3$,
$CH_2CF_3$,
$C_1$-$C_4$ alkyl,
$CH{=}NOCH_3$,
CH=NOH,
CHO,
$CH_2OCH_3$,
$CH_2OH$,
Cl,
CN,
$COCF_3$,
$COC_1$-$C_3$ alkyl,
$CONH_2$,
$CONHC_1$-$C_3$ alkyl,

$CON(C_1-C_3\ alkyl)_2$,
$COOC_1-C_3$ alkyl,
COOH,
F,
I,
$NH_2$,
$NHC_1-C_3$ alkyl,
$N(C_1-C_3\ alkyl)_2$,
NHCHO,
$NHCOCH_3$,
$NHCONH_2$,
$NHSO_2CH_3$,
$NO_2$,
$OC_1-C_3$ alkyl,
$OCOCH_3$,
OH,
$SC_1-C_3$ alkyl,
$SOC_1-C_3$ alkyl,
$SO_2C_1-C_3$ alkyl,
$SO_2NH_2$,
$SO_2NHC_1-C_3$ alkyl,
$SO_2N(C_1-C_3\ alkyl)_2$ et
$SO_3H$ ;

- dans laquelle A est choisi dans le groupe comprenant N et C ;

- dans laquelle $R_1$ est un atome d'hydrogène, ou un groupe hydrocarboné ou un groupe hydrocarboné modifié ayant jusqu'à 4 atomes de carbone, saturé ou insaturé, acyclique, cyclique ou mixte, et dans lequel, dans le groupe hydrocarboné modifié :

. 1 à 2 atomes de carbone peuvent être remplacés par 1 à 2 hétéroatomes, identiques ou différents, choisis dans le groupe comprenant les atomes d'azote, oxygène, soufre, chlore, brome et iode,

. et 1 à 3 atomes d'hydrogène peuvent être remplacés par 1 à 3 atomes de fluor ;

- dans laquelle $R_2$ est un groupe hydrocarboné ou un groupe hydrocarboné modifié, de 2 à 13 atomes de carbone, saturé ou insaturé, acyclique, cyclique ou mixte, et dans lequel, dans le groupe hydrocarboné modifié :

. 1 à 4 atomes de carbone peuvent être remplacés par 1 à 4 hétéroatomes, identiques ou différents, choisis dans le groupe comprenant les atomes d'azote, oxygène, soufre, chlore, brome et iode,

. et 1 à 5 atomes d'hydrogène peuvent être remplacés par 1 à 5 atomes de fluor ;

- dans laquelle $R_1$ et $R_2$ peuvent être fusionnés ;

- dans laquelle $R_3$ est choisi dans le groupe comprenant un atome d'hydrogène ou un groupe alkyle $C_1$-$C_3$ ;

- dans laquelle $R_4$ est choisi dans le groupe comprenant :

H et

$CH_3$ ;

à condition, quand $X_3$, $X_5$, $R_3$ et $R_4$ sont des atomes d'hydrogène,

. et quand $X_4$ est CN ou $NO_2$, que $R_1$ et $R_2$ ne soient pas CN ou $OCH_3$,

. et quand $X_4$ est H, $CF_3$, CHO, Cl, CN, $COCH_3$, F ou $NO_2$, que $R_1$ ne soit pas $NO_2$ ou $SOC_1$-$C_2$ alkyl et que $R_2$ ne soit pas $NO_2$, SOR, $SO_2R$, $SO_2NHR$ et $SO_2N(R)_2$, R étant un groupe alkyle, cycloalkyle ou aryle ayant jusqu'à 10 atomes de carbone, 1 ou 2 de ces atomes de carbone pouvant être remplacés par 1 ou 2 atomes de soufre ou d'oxygène.

Dans les définitions de $R_1$ et $R_2$ données ci-dessus, l'expression "groupe hydrocarboné modifié" désigne un groupe résultant du remplacement, dans le groupe hydrocarboné initial, d'au moins une unité carbonée par au moins une unité contenant un hétéroatome choisi dans le groupe comprenant N, O, S, Cl, Br et I, ou d'au moins un atome d'hydrogène par un atome de fluor. Par exemple : le groupe $CH(CH_3)C_6H_5$ peut être modifié en un groupe $N(CH_3)C_6H_5$ par remplacement du groupe CH par un atome d'azote (exemple 74) ; le groupe *c*-$C_3H_5$ peut être modifié en un groupe $NO_2$ par remplacement du groupe CH par un atome d'azote et des deux groupes $CH_2$ par des atomes d'oxygène (exemple 29) ; le groupe 1-cyclopropylphényle (1-*c*-$C_3H_4$-$C_6H_5$) peut être modifié en un groupe $SO_2C_6H_5$ par remplacement de l'atome de carbone du groupe cyclopropyle par un atome de soufre et des deux groupes $CH_2$ par des atomes d'oxygène (exemple 79). Par l'expression "$R_1$ et $R_2$ peuvent être fusionnés", il faut entendre que les groupes hydrocarbonés (ou hydrocarbonés modifiés) peuvent être reliés par l'intermédiaire d'une simple ou d'une double liaison (exemples 82 et 83). Enfin, dans la définition du groupe $R_2$, la limitation en $C_2$-$C_{13}$ du groupe hydrocarboné est essentiellement basée sur des considérations de solubilité ou de maintien de l'activité édulcorante.

Avantageusememt :

- $X_3$ et $X_5$ sont choisis dans le groupe constitué par :
  - H
  - Br,
  - $CF_3$,
  - $CH_3$,
  - Cl et
  - F ;

- $X_4$ est choisi dans le groupe constitué par :
  - H,
  - CN,
  - $COOCH_3$,
  - F et
  - $NO_2$ ;

- A est un atome d'azote ;

- $R_1$ est choisi dans le groupe constitué par H et $CH_3$ ;

- $R_2$ est choisi dans le groupe constitué par:
  - $C_2$-$C_{13}$ alk(én)(yn)yl normal,
  - $C_3$-$C_{13}$ alk(én)(yn)yl ramifié,
  - $C_3$-$C_{13}$ cycloalk(én)yl,
  - $C_4$-$C_{13}$ alk(én)yl cycloalk(én)yl,
  - $C_4$-$C_{13}$ cycloalk(én)yl alk(én)yl,
  - $C_5$-$C_{13}$ alk(én)yl cycloalk(én)yl alk(én)yl,
  - $C_7$-$C_{13}$ alk(én)yl bicycloalk(én)yl,
  - $C_7$-$C_{13}$ bicycloalk(én)yl fusionné,
  - $C_8$-$C_{13}$ alk(én)yl bicycloalk(én)yl fusionné,

$C_8$-$C_{13}$ bicycloalk(én)yl fusionné alk(én)yl,
$C_9$-$C_{13}$ alk(én)yl bicycloalk(én)yl fusionné alk(én)yl,
$C_{10}$-$C_{13}$ tricycloalk(én)yl fusionné,
$C_{11}$-$C_{13}$ alk(én)yl tricycloalk(én)yl fusionné,
$C_{11}$-$C_{13}$ tricycloalk(én)yl fusionné alk(én)yl,
et $C_{13}$ alk(én)yl tricycloalk(én)yl fusionné alk(én)yl,

dans lesquels, sous la forme d'un groupe hydrocarboné modifié :

. jusqu'à 4 atomes de carbone peuvent être remplacés par des hétéroatomes, identiques ou différents, choisis dans le groupe constitué par :

S pour remplacer C ou $CH_2$,
N pour remplacer CH,
NH et O pour remplacer $CH_2$, et
Cl, Br et I pour remplacer $CH_3$,

. et jusqu'à 5 atomes d'hydrogène peuvent être remplacés par des atomes de fluor ;

- $R_3$ est choisi dans le groupe constitué par H et $CH_3$ ;

- $R_4$ est un atome d'hydrogène.

Il doit être noté que dans la liste des groupes hydrocarbonés préférés pour $R_2$, les groupes cycloalkényle comprennent aussi les groupes aryle, un groupe phényle par exemple étant un groupe

1,3,5-cyclohexatriényle ; par les expressions "bicycloalkényl fusionné" et "tricycloalkényl fusionné", il faut entendre des groupes polycycliques, les cycles étant associés par l'intermédiaire de liaisons simples ou doubles communes.

Avantageusement, en pratique :

- le groupe hydrocarboné $R_2$ est choisi dans le groupe constitué par :

$n$-$C_5H_{11}$, $n$-$C_6H_{13}$, $n$-$C_7H_{15}$,

$CH(CH_3)(CH_2)_2CH_3$, $CH(CH_3)(CH_2)_3CH_3$, $(CH_2)_3CH(CH_3)_2$, $(CH_2)_4CH(CH_3)_2$, $(CH_2)_5CH(CH_3)_2$,

$C_6H_5$, $C_6$-$C_{10}$ cycloalkyl,

$CH_2C_6H_5$, $CH_2$-$c$- $C_6H_{11}$, $CH(CH_3)C_6H_5$, $CH(CH_3)$-$c$-$C_6H_{11}$, $(CH_2)_2C_6H_5$, $(CH_2)_2$-$c$-$C_6H_{11}$, $CH(CH_3)CH_2C_6H_5$, $CH(CH_3)CH_2$-$c$-$C_6H_{11}$,

$C_6H_4(CH_3)$, $C_6$-$C_{10}$ cycloalkyl$(CH_3)$,

$CH_2C_6H_4(CH_3)$, $CH_2$-$c$-$C_6H_{10}(CH_3)$, $CH(CH_3)C_6H_4(CH_3)$, $CH(CH_3)$-$c$-$C_6H_{10}(CH_3)$,

$(CH_2)_2CH(c$-$C_3H_5)_2$, $(CH_2)_3CH(c$-$C_3H_5)_2$, $CH(CH_3)CH_2CH$-$(c$-$C_3H_5)_2$, $CH(CH_3)(CH_2)_2CH(c$-$C_3H_5)_2$,

naphtyl, 5,6,7,8-tétrahydronaphtyl, perhydronaphtyl, indényl, indanyl,

naphtyl$(CH_3)$, 5,6,7,8-tétrahydronaphtyl$(CH_3)$, perhydronaphtyl$(CH_3)$, indényl$(CH_3)$, indanyl$(CH_3)$, fenchyl,

$CH_2$-naphtyl, $CH_2$-5,6,7,8-tétrahydronaphtyl, $CH_2$-perhydronaphtyl, $CH_2$-indényl, $CH_2$-indanyl,

$CH_2$-naphtyl$(CH_3)$, $CH_2$-5,6,7,8-tétrahydronaphtyl-$(CH_3)$, $CH_2$-perhydronaphtyl$(CH_3)$, $CH_2$-indényl$(CH_3)$, $CH_2$-indanyl$(CH_3)$,

adamantyl,

$CH_2$-adamantyl, $CH(CH_3)$adamantyl,

$CH_2$-adamantyl$(CH_3)$ ;

- le groupe hydrocarboné modifié $R_2$ est choisi dans le groupe constitué par :

$N(CH_3)C_6H_5$, pyridinyl, pipéridyl, homopipéridyl, indolyl, indolinyl, isoindolinyl, quinolyl, isoquinolyl, pyrazinyl, pyrimidyl, indazolyl, quinoxalinyl, quinazolinyl, purinyl,

$OCH_2C_6H_5$, pyranyl, benzofuranyl, méthoxyphényl, méthyloxycarbonylphényl, 3,4-méthylènedioxyphényl, morpholinyl, benzoxazolyl, éthanamidophényl, nitrile, nitro,

thiophényl, benzothiophényl, 2,2,4,4-tétraméthylthiacyclobut-3-yl, thiazolyl, isothiazolyl, $SO_2C_6H_5$, $SO_2C_6H_{11}$, $SO_2C_7H_{13}$,

chlorophényl,

fluorophényl, trifluorométhylphényl.

Les agents édulcorants préférés de l'invention répondent à la formule :

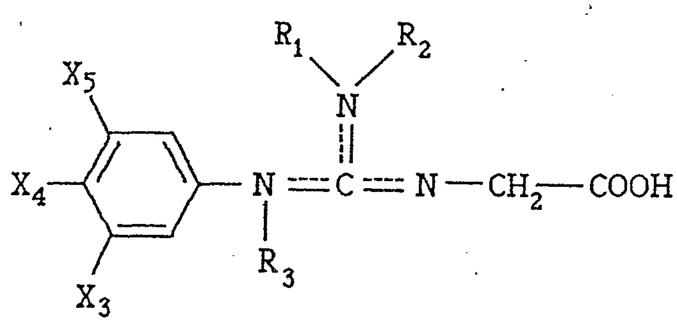

dans laquelle :

- $X_3$ et $X_5$ sont un atome d'hydrogène et $X_4$ est CN,

- $X_3$ et $X_5$ sont Cl, $CF_3$, $CH_3$ et F et $X_4$ est H ou CN,

- $R_1$ est H ou $CH_3$,

- $R_2$ est choisi dans le groupe constitué par $CH(CH_3)C_6H_5$, $CH_2C_6H_5$, $CH(CH_3)$-*c*-$C_6H_{11}$, *c*-$C_6H_{11}$, *c*-$C_7H_{13}$, *c*-$C_8H_{15}$, *c*-$C_9H_{17}$, *c*-$C_{10}H_{19}$, $SO_2C_6H_5$ et $SO_2C_7H_{13}$,

- $R_3$ est H.

Les agents édulcorants de l'invention se caractérisent aussi en ce qu'ils peuvent être salifiés par des acides ou des bases inorganiques ou organiques physiologiquement acceptables. Avantageusement, ces composés sont salifiés sous forme d'hydrochlorure ou de sels de sodium, potassium, ammonium, calcium et magnésium.

L'invention concerne également le procédé qui consiste à édulcorer les aliments, boissons,

confiseries, pâtisseries, chewing gum, produits d'hygiène, cosmétiques, articles de toilette, produits pharmaceutiques et vétérinaires, et leurs équivalents, en leur ajoutant une quantité adéquate d'un ou plusieurs agents édulcorants selon la présente invention. Par "quantité adéquate", on désigne une quantité d'agent édulcorant suffisante pour produire la perception d'une saveur sucrée.

L'invention concerne aussi les préparations édulcorées suivant le procédé de la présente invention.

L'invention concerne également les compositions édulcorantes caractérisées en ce qu'elles comprennent une quantité adéquate d'au moins un agent édulcorant selon la présente invention, et un support ou agent de charge approprié.

L'invention concerne aussi les compositions édulcorantes caractérisées en ce qu'elles comprennent une quantité adéquate d'un ou plusieurs agents édulcorants selon la présente invention, et au moins un autre agent édulcorant.

Les composés de la présente invention se distinguent, pour la plupart, des composés décrits dans la demande de brevet européen EP-A-0 195 730 citée dans le préambule par leur activité édulcorante beaucoup plus intense et par leur plus grande stabilité à la chaleur ou dans les solutions acides. Sur le plan structural, les composés de la présente invention se distinguent des composés de la demande EP-A-0 195 730 précédemment citée par la possibilité de substituer les positions 3 et 5 du noyau benzénique, cette substitution ayant l'avantage d'augmenter, souvent considérablement, et de manière tout à fait imprévisible, le pouvoir sucrant des composés dérivant des acides N-phénylguanidino et N-phényléthanamidinoacétiques. C'est ainsi par exemple

que les dérivés disubstitués par $CH_3$ ou Cl sur les positions 3 et 5 (méta) du noyau benzénique sont environ 40 fois plus sucrés, sur une base pondérale, que les dérivés correspondants substitués seulement en position 4 (para). Ceci est d'autant plus inattendu que, comme on le sait, toute modification, même légère, de la structure moléculaire d'un agent édulcorant peut entraîner une suppression de l'activité édulcorante, et ce d'autant que les relations entre la structure et l'activité édulcorante sont imprévisibles (voir par exemple M.G.J. BEETS, Structure-Activity Relationships in Human Chemoreception, Applied Science Pub., London, 1978, pp. 259-362). On a d'ailleurs observé que les dérivés substitués sur les positions 2 ou 6 (positions ortho) sont non sucrés ou très faiblement sucrés, ce qui montre, une fois encore, le caractère imprévisible qu'exerce, sur l'activité édulcorante, la position des substituants sur le cycle benzénique. Les composés de la présente invention se distinguent aussi des composés de la demande EP-A-0 195 730 par la possibilité d'obtenir, de manière totalement imprévisible, des activités édulcorantes extrêmement élevées en greffant sur l'atome A un groupe encombrant de caractère fortement hydrophobe (groupe $R_2$ des composés de la présente invention), en remplacant donc les groupes hydrophiles attracteurs d'électrons par des groupes très hydrophobes.

La présente invention a permis ainsi d'accéder à des agents édulcorants qui sont parmi les plus puissants obtenus jusqu'à ce jour, puisqu'on a pu obtenir un agent édulcorant qui est au moins 200 000 fois plus sucré que le saccharose, sans commune mesure donc avec le pouvoir sucrant des principaux agents édulcorants actuellement utilisés, proposés, développés ou en cours d'expérimentation, et qui sont rappelés dans le tableau I ci-après.

TABLEAU I

| Agents édulcorants | Pouvoir sucrant (saccharose = 1) |
|---|---|
| Xylitol | 1 |
| Cyclamate de sodium | 30 |
| Glycyrrhizine | 50 |
| Acésulfame-K | 200 |
| Aspartame | 200 |
| Stévioside | 300 |
| Saccharine | 400 |
| Néohespéridine dihydrochalcone | 1000 |
| Trichlorogalactosucrose | 2000 |
| Alitame | 2000 |
| Thaumatine | 2000 |
| Monelline | 2000 |

Un autre avantage très important des agents édulcorants de la présente invention est de donner une saveur sucrée très pure, pratiquement identique à celle du saccharose, sans arrière-goût de réglisse (comme c'est le cas par exemple pour la glycyrrhizine ou la thaumatine) et sans arrière-goût métallique ou amer (comme c'est le cas par exemple pour la saccharine ou l'acésulfame-K).

Les autres avantages que l'on peut enfin attendre des agents édulcorants de la présente invention sont directement liés à leur activité édulcorante élevée, à

savoir notamment leur faible prix de revient et leur sécurité d'utilisation due aux très faibles concentrations nécessaires pour obtenir une saveur sucrée.

Bref, les composés de l'invention répondent de manière inattendue aux exigences modernes que l'on recherche pour les édulcorants et que l'on n'avait pu obtenir jusqu'ici, à savoir :

. un pouvoir édulcorant élevé (jusqu'à mille fois celui de l'aspartame) ;
. une solubilité suffisante pour leur utilisation dans les boissons acides ;
. une bonne stabilité dans les milieux acides ou neutres et une bonne résistance thermique ;
. une innocuité potentielle, favorisée par les très faibles concentrations d'utilisation ;
. et enfin, un prix de revient très faible.

Les composés de la présente invention peuvent être préparés par des méthodes variées déjà décrites dans la littérature (J. Med. Chem., 1978, 21, 773-781 ; Chem. Ber., 1966, 99, 1252-1257 ; Brit. Pat. No. 1587 258 ; J. Org. Chem., 1970, 35, 2067-2069 ; J. Org. Chem., 1986, 51, 1882-1884 ; Chem. Ber., 1967, 100, 591-604 ; J. Für. Prakt. Chemie, 1977, 319, 149-157 ; The Chemistry of Amidines and Imidates, S. Patai ed., Wiley-Interscience, 1975, p. 283-348).

Le procédé pour la préparation de tels agents édulcorants consiste à condenser un dérivé thiouréido ou thioamido de formule générale :

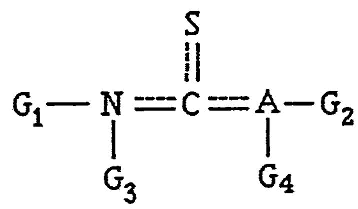

avec un composé de formule générale :

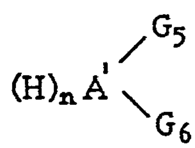

dans lesquelles :

- . $G_1$ est $(X_3X_4X_5)C_6H_2$ ou $HOOCCHR_4$,
- . $G_2$ est $R_2$ ou $HOOCCHR_4$,
- . $G_3$, $G_4$ et $G_5$ sont H, $R_1$ ou $R_3$,
- . $G_6$ est $(X_3X_4X_5)C_6H_2$, $R_2$ ou $HOOCCHR_4$,
- . A' est N ou C,
- . n est égal à 1 quand A' est N, et est égal à 2 quand A' est C ;

et dans lesquelles :

- . $G_1$, $G_2$ et $G_6$ ne sont pas simultanément identiques,
- . A et A' ne sont pas simultanément un atome de

carbone,

- A' est N et $G_3$, $G_4$ et $G_5$ sont H lorsque $G_1$ ou $G_2$ ou $G_6$ sont $HOOCCHR_4$ ;

et dans lesquelles $X_3$, $X_4$, $X_5$, $R_1$, $R_2$, $R_3$, $R_4$ et A correspondent aux définitions données précédemment.

Les dérivés thiouréido peuvent être avantageusement obtenus par réaction d'un isothiocyanate sur une amine suivant l'une des réactions :

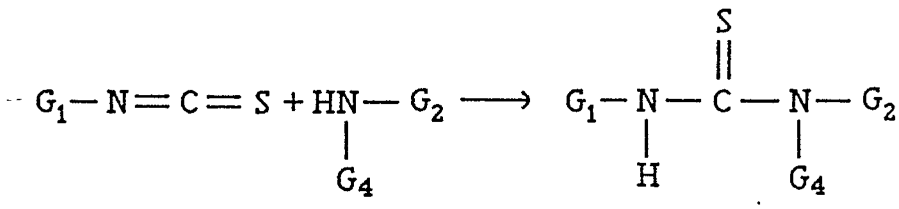

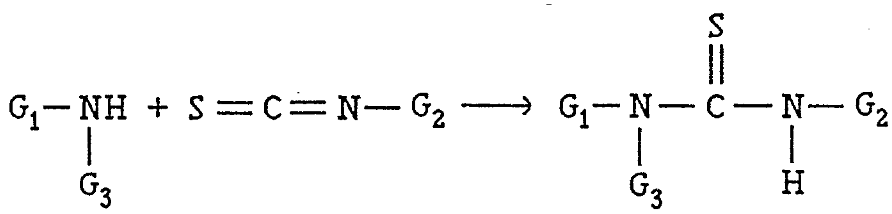

La condensation des dérivés thiouréido ou thioamido peut être avantageusement réalisée par activation de l'atome de soufre en le transformant en un groupe S-alkyle ou $SO_3H$, ou en le remplaçant par un groupe O-alkyl, O-$SO_2$aryl ou un atome d'halogène. En pratique, le dérivé thiouréido ou thioamido est avantageusement activé en le transformant en un dérivé S-méthylisothiouréido ou S-méthylisothioamido de formule générale :

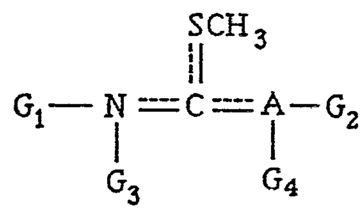

ceci par action d'un agent alkylant choisi dans le groupe comprenant l'iodure de méthyle et le diméthyl sulfate.

Le dérivé S-méthylisothiouréido préféré correspond à la formule générale :

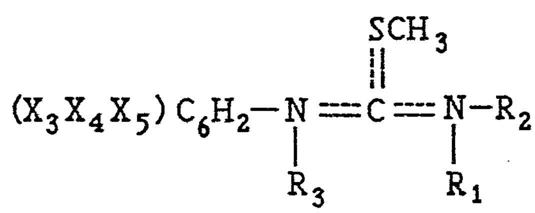

Lorsque A est N et lorsque $G_3$ et $G_4$ sont un atome d'hydrogène, la condensation peut être avantageusement réalisée en activant le dérivé thiouréido par transformation en un intermédiaire carbodiimide :

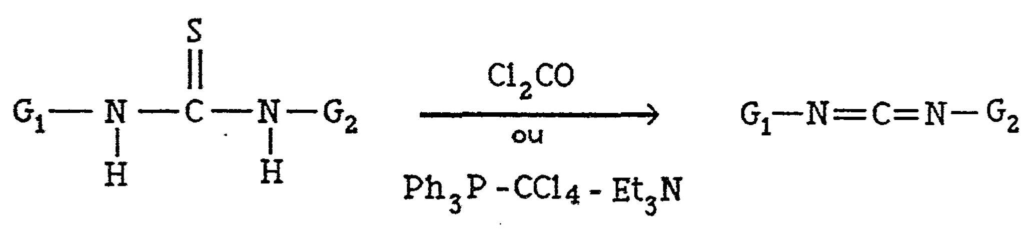


soit par traitement par le phosgène, soit par action d'un mélange équimoléculaire de $(C_6H_5)_3P$ - $CCl_4$ - $(C_2H_5)_3N$.

Enfin, lorsque A et A' sont N et que $G_3$ ou $G_4$ ou $G_5$ est H, la condensation du dérivé thiouréido avec l'amine peut avantageusement être réalisée par action de dicyclohexylcarbodiimide (DCC) comme agent de couplage, suivant la réaction :

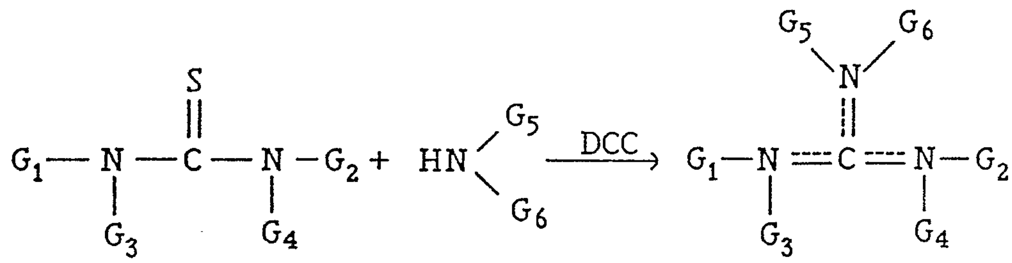

Avantageusement, en pratique :

L'une des méthodes de synthèse préférées pour obtenir les dérivés thiouréido consiste à faire réagir un isothiocyanate d'alkyle ou d'aryle avec une amine convenablement choisie. La réaction est conduite à température ambiante ou à ébullition suivant la réactivité des deux composés mis en présence, et elle est effectuée dans un solvant organique tel que l'éthanol, le méthanol, le chloroforme ou l'acétone. On peut ainsi réaliser les réactions suivantes :

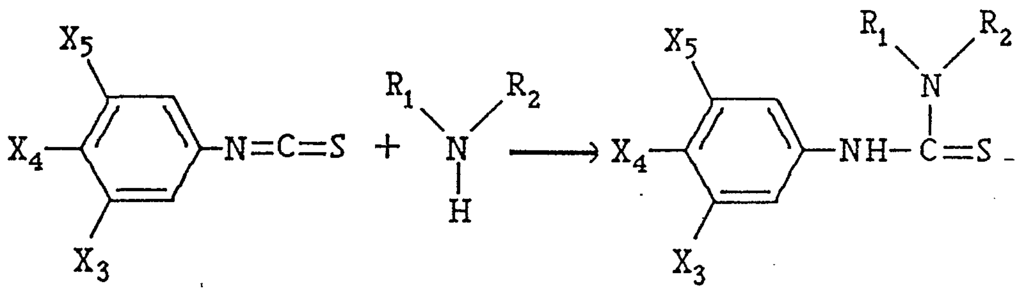

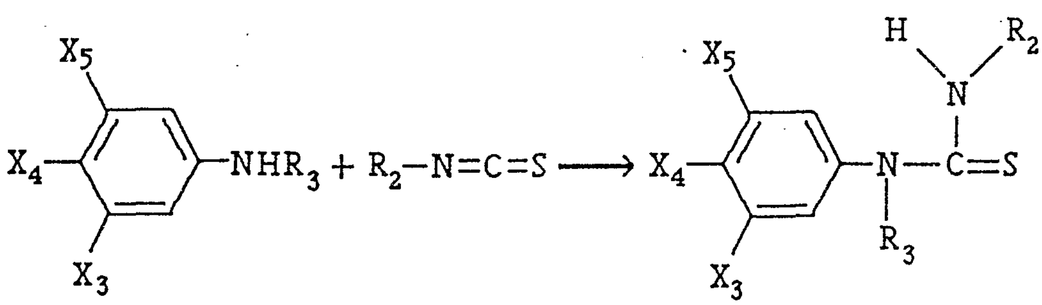

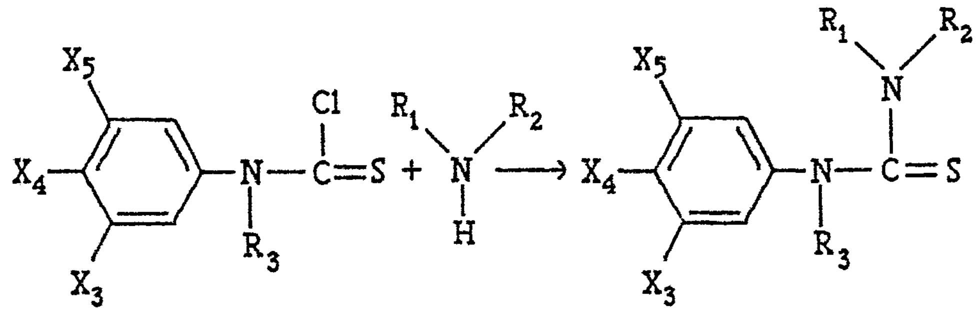

La condensation des dérivés thiouréido peut alors être avantageusement réalisée soit en remplaçant l'atome de soufre par un ligand activé (désigné par la lettre L dans les formules ci-dessous), soit en transformant le dérivé thiouréido en un intermédiaire carbodiimide.

Dans la première méthode d'activation, le ligand activé est choisi de préférence dans l'ensemble constitué par les groupes S-alkyl, $SO_3H$, O-alkyl, $OSO_2$aryl, et les halogènes. La condensation consiste alors à faire réagir l'intermédiaire ainsi activé avec une amine convenablement choisie, en mettant en contact :

(1) 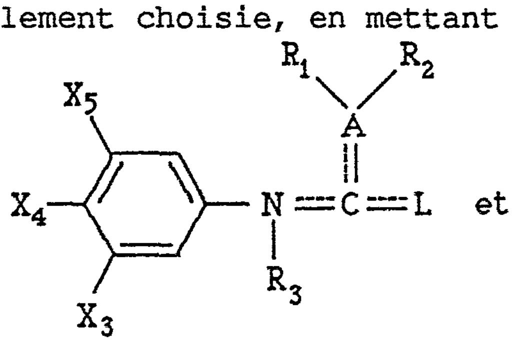 et $H_2N—CHR_4—COOH$ ,

(2) 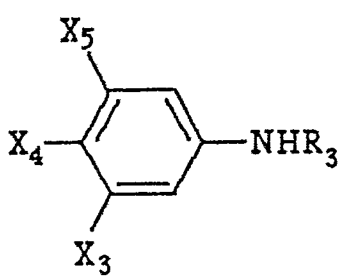 et 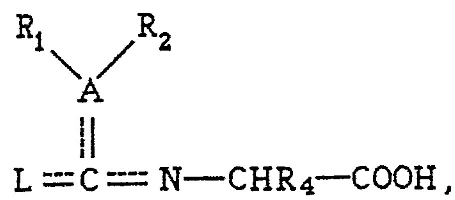

(3) 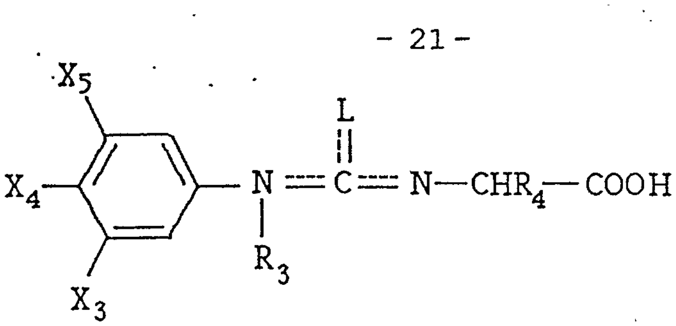 et 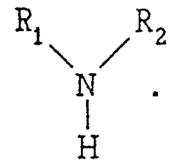 .

Dans une méthode de réalisation particulièrement avantageuse, le ligand activé L préféré est un groupe S-alkyle, de préférence un groupe $S-CH_3$, comme par exemple dans le dérivé S-méthylisothiouréido suivant :

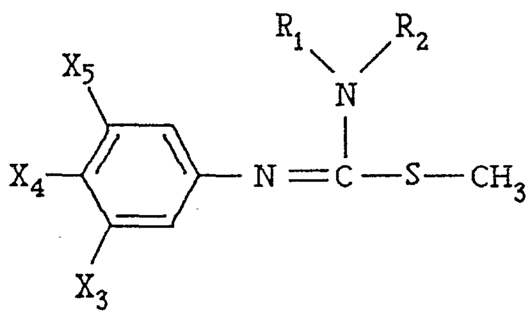

L'intermédiaire activé est avantageusement obtenu en traitant le dérivé thiouréido par un agent alkylant (iodure de méthyle, diméthyl sulfate) en solution dans un solvant organique comme l'acétone ou la 2-butanone, à une température comprise entre la température ambiante et l'ébullition. Le dérivé S-méthylisothiouréido est obtenu sous forme de sel (iodure, sulfate). Le sel est traité par une solution d'hydroxyde de sodium ou d'hydroxyde de potassium pour libérer la forme basique. La condensation avec l'α-amino acide est alors effectuée dans un mélange éthanol-eau, en présence d'une base telle que l'hydroxyde de sodium, l'hydroxyde de potassium ou une amine tertiaire (comme par exemple la triéthylamine), à des températures comprises entre la température ambiante et l'ébullition.

Dans la seconde méthode d'activation, l'intermédiaire carbodiimide est avantageusement obtenu par action du phosgène, ou par action d'un mélange équimoléculaire triphénylphosphine- tétrachlorure de carbone-amine tertiaire (triéthylamine par exemple) en solution dans un solvant organique tel que le tétrachlorure de carbone ou le dichlorométhane, à des températures comprises entre 0 °C et l'ébullition. La condensation consiste alors à faire réagir l'intermédiaire carbodiimide avec une amine convenablement choisie, en mettant en contact :

(4) 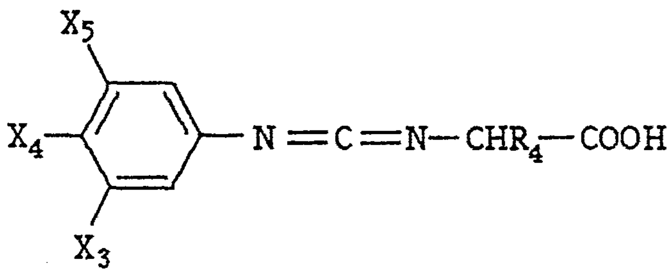 et 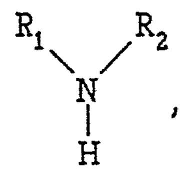 ,

(5) 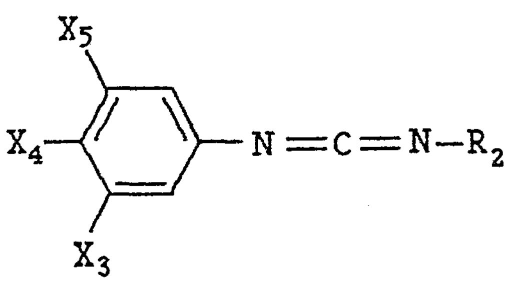 et $H_2N-CHR_4-COOH$ ,

(6) 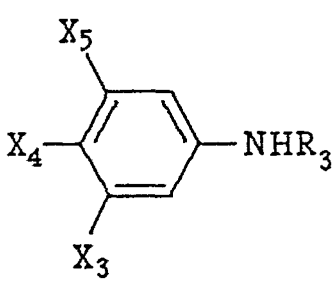 et $R_2-N=C=N-CHR_4-COOH$ .

Ces condensations peuvent être effectuées dans l'eau ou dans des solvants organiques tels que l'éthanol, le méthanol, l'acétone, le chloroforme, le tétrachlorure de carbone ou la pyridine, à une température pouvant varier

de la température ambiante jusqu'à l'ébullition.

Dans certaines réactions (réactions 2 à 6), il peut être avantageux de protéger au préalable le groupe carboxyle de l'α-amino acide, sous forme par exemple d'un ester (ester méthylique, éthylique, *tert*-butylique, benzylique). Il est alors nécessaire, pour obtenir les composés de l'invention, d'éliminer le groupe protecteur par le moyen le plus approprié qui peut être, par exemple, une saponification par une solution d'hydroxyde de sodium ou une hydrolyse par une solution de chlorure d'hydrogène.

Les composés de l'invention peuvent exister, suivant la nature de $R_1$, $R_2$, $R_3$ et A, sous forme de zwitterion ou sous forme acide. Ils peuvent donc être salifiés par des acides ou des bases inorganiques ou organiques physiologiquement acceptables. L'une des méthodes de choix pour préparer ces sels consiste à concentrer à sec sous vide un mélange, en solution aqueuse, d'un composé de l'invention et d'un équivalent d'un acide ou d'une base inorganique ou organique. Les sels préférés de l'invention sont les hydrochlorures et les sels de sodium, potassium, ammonium, calcium et magnésium.

La purification des composés de l'invention a été réalisée selon les techniques stantards telles que la recristallisation ou la chromatographie. Leur structure et leur pureté a été contrôlée par les techniques classiques (chromatographie sur couche mince, chromatographie liquide haute performance, spectrométrie infra-rouge, résonance magnétique nucléaire, analyse élémentaire).

Les présents agents édulcorants peuvent exister comme un mélange en équilibre de formes tautomères. C'est

ainsi par exemple, quand A est N, et $R_1$ et $R_3$ sont H, on a :

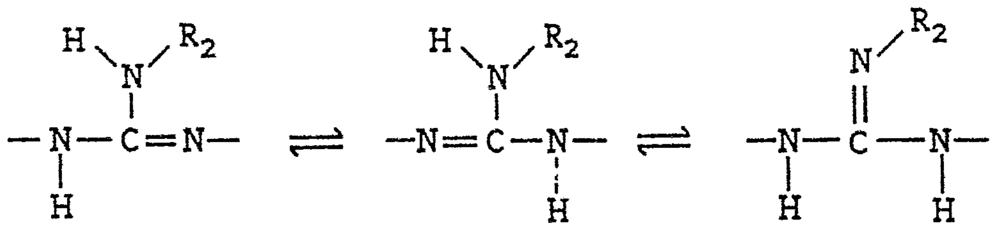

Quand A est N, $R_1$ est $CH_3$, et $R_3$ est H, on a :

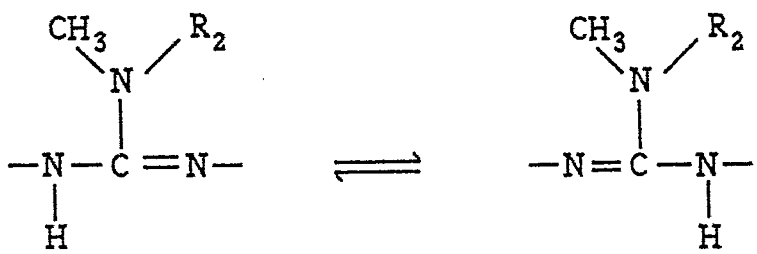

Quand A est N, $R_1$ est H, et $R_3$ est $CH_3$, on a :

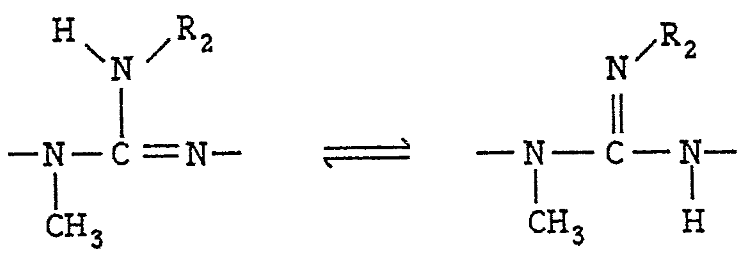

Quand A est C, et $R_3$ est H, on a :

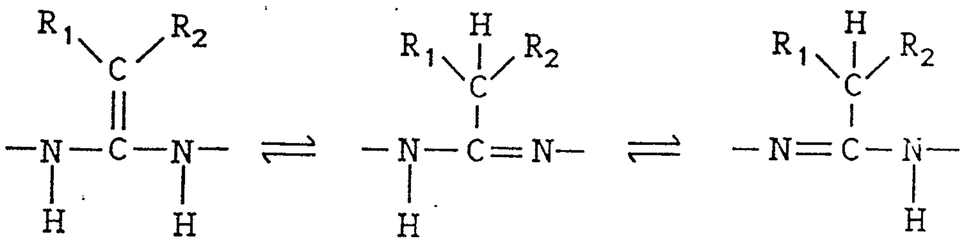

C'est la raison pour laquelle les agents édulcorants de la présente invention sont représentés, dans la formule générale, par un hybride de résonance , et, dans la partie descriptive, par une de leurs formes tautomères, tout en sachant que la forme tautomère figurée est immanquablement en équilibre avec les autres formes tautomères, les proportions respectives des tautomères variant suivant la nature des substituants $X_3$, $X_4$, $X_5$, $R_1$, $R_2$ et $R_3$, et suivant le pH.

Les agents édulcorants de la présente invention sont généralement stables. La stabilité est déterminée à pH 3 en solution dans un tampon phosphate 1mM à 70 °C. La concentration du composé restant est déterminée par chromatographie liquide haute pression (HPLC). Par exemple, après six jours dans de telles conditions, on retrouve encore la moitié du composé de l'exemple 14 dans lequel $X_4$ est CN, $X_3$, $X_5$, $R_1$, $R_3$ et $R_4$ sont H, A est N, et $R_2$ est (*S*)$CH(CH_3)C_6H_5$. On améliore la stabilité lorsque l'on remplace l'atome d'hydrogène de $R_1$ par $CH_3$. Ainsi, après sept jours dans les mêmes conditions , on retrouve encore 88 % du composé de l'exemple 31 dans lequel $X_3$ et $X_5$ sont Cl, $X_4$, $R_3$ et $R_4$ sont H, A est N, $R_1$ est $CH_3$, et $R_2$ est $CH_2C_6H_5$. De la même manière, toujours après sept jours et dans les mêmes conditions, on retrouve 86 % du composé de l'exemple 33 dans lequel $X_4$ est CN, $X_3$, $X_5$, $R_3$ et $R_4$ sont H, A est N, $R_1$ est $CH_3$, et $R_2$ est $CH_2C_6H_5$.

Les agents édulcorants de la présente invention ont pour utilité de pouvoir être ajoutés à tout produit comestible dans lequel on désire apporter un goût sucré, à condition qu'on les ajoute en proportions suffisantes pour atteindre le niveau d'édulcoration désiré. La concentration optimale d'utilisation de l'agent édulcorant dépendra de facteurs divers tels que, par exemple, le pouvoir sucrant de l'agent édulcorant, les conditions de stockage et d'utilisation des produits, les constituants particuliers des produits, le profil gustatif des produits comestibles et le niveau d'édulcoration désiré. Toute personne qualifiée peut facilement déterminer la proportion optimale d'agent édulcorant qui doit être employée pour l'obtention d'un produit comestible en réalisant des analyses sensorielles de routine. Les agents édulcorants de la présente invention sont, en général, ajoutés aux produits comestibles dans des proportions allant, suivant le pouvoir édulcorant du composé, de 0,1 mg à 500 mg d'agent édulcorant par kilogramme ou par litre de produit comestible. Les produits concentrés contiendront évidemment des quantités plus élevées d'agent édulcorant, et seront ensuite dilués suivant les intentions finales d'utilisation.

Les produits susceptibles d'être édulcorés par les agents édulcorants de la présente invention comprennent tous les produits pour lesquels on désire un composant de goût sucré, notamment, et ce de manière non limitative, les produits alimentaires (pour la consommation humaine ou animale), les boissons (boissons alcooliques, boissons non alcooliques, jus, boissons gazeuses), les confiseries, les pâtisseries, le chewing gum, les produits d'hygiène, les cosmétiques, les produits pharmaceutiques et vétérinaires, et leurs équivalents.

Les agents édulcorants de la présente invention peuvent être ajoutés sous forme pure aux produits comestibles pour leur communiquer un goût sucré. Toutefois, par suite du pouvoir sucrant élevé des présents agents édulcorants, ils sont généralement mélangés à un support ("carrier") ou à un agent de charge ("bulking agent") approprié. Avantageusement, les supports ou agents de charge appropriés sont choisis dans le groupe constitué par le polydextrose, l'amidon, les maltodextrines, la cellulose, la méthylcellulose, la carboxyméthylcellulose, l'hydroxyméthylcellulose, la cellulose microcristalline et autres dérivés de la cellulose, l'alginate de sodium, les pectines, les gommes, le lactose, le maltose, le glucose, la leucine, le glycérol, le mannitol, le sorbitol, le bicarbonate de sodium, les acides phosphorique, citrique, tartrique, fumarique, benzoïque, sorbique, propionique, et leurs sels de sodium, potassium et calcium, et leurs équivalents ainsi que leurs mélanges.

Les présents agents édulcorants peuvent, dans un produit comestible, être employés seuls, comme unique agent édulcorant, ou sous forme de mélanges de deux ou plusieurs agents édulcorants de la présente invention. Les présents agents édulcorants peuvent, en outre, être utilisés en combinaison avec d'autres agents édulcorants tels que le saccharose, le sirop de maïs, le fructose, les dérivés dipeptidiques sucrés (aspartame, alitame), la néohespéridine dihydrochalcone, l'isomaltulose hydrogéné, le stévioside, les sucres L, la glycyrrhizine, le xylitol, l'acésulfame-K, la saccharine et ses sels de sodium, potassium ou calcium, l'acide cyclamique et ses sels de sodium, potassium ou calcium, le trichlorogalactosucrose, la monelline, la thaumatine, et leurs équivalents, ainsi que leurs mélanges.

Le pouvoir édulcorant des composés préparés dans les exemples suivants a été évalué par un groupe de huit goûteurs expérimentés. Pour cela, les composés, en solution aqueuse à des concentrations variables, sont comparés, sur le plan gustatif, à une solution témoin de saccharose à 2 % et dans certains cas à 5 % et 10 %, c'est-à-dire à des concentrations correspondant à celles utilisées lors d'un usage courant. Le pouvoir sucrant des édulcorants de synthèse varie en effet suivant la concentration de la solution de saccharose utilisée comme référence. Le pouvoir édulcorant du composé testé par rapport au saccharose correspond alors au rapport pondéral qui existe entre le composé et le saccharose à égale intensité édulcorante, c'est-à-dire quand les saveurs sucrées de la solution du composé testé et de la solution témoin de saccharose sont considérées, par une majorité de goûteurs, avoir la même intensité édulcorante.

La manière dont l'invention peut être réalisée et les avantages qui en découlent ressortiront mieux des exemples de réalisation qui suivent, donnés à titre indicatif et non limitatif.

EXEMPLE 1 :

Synthèse de l'acide N-[N-(*S*)-α-méthylbenzylamino (phénylimino)méthyl]-2-aminoéthanoïque :

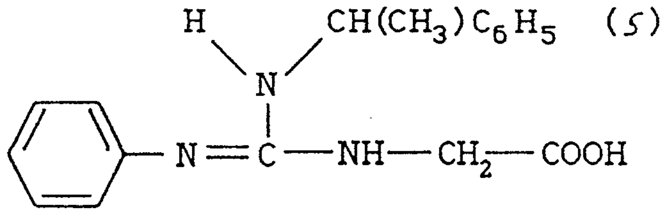

Ce composé est obtenu à partir de la glycine et de la N-(*S*)-α-méthylbenzyl-N'-phényl-S-méthylisothiourée en suivant le protocole expérimental décrit dans l'exemple 2 (rendement 53 %, point de fusion 209 °C).

Le pouvoir édulcorant de ce composé correspond approximativement, sur une base pondérale, à 5 000 (cinq mille) fois celui du saccharose (par comparaison avec une solution de saccharose à 2 %).

EXEMPLE 2 :

Synthèse de l'acide N-[N-(*S*)-α-méthylbenzylamino (3-bromophénylimino)méthyl]-2-aminoéthanoïque :

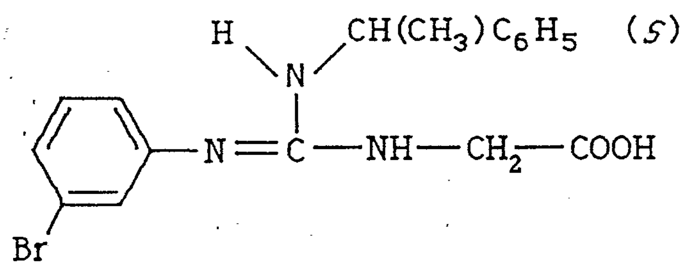

Etape 1 : Synthèse de la N-(*S*)-α-méthyl benzyl-N'-(3-bromophényl)thiourée :

5 g (41 mmol) de (*S*)-α-méthylbenzylamine et 8 g (37 mmol) de 3-bromophényl isothiocyanate sont mélangés dans 50 $cm^3$ d'éthanol à 95 %. Après 4 heures d'agitation à 20 °C, la solution est concentrée à sec sous vide. Après lavage par 200 $cm^3$ d'hexane, 11,6 g (rendement 97 %) du dérivé thiouréido (point de fusion 103 °C) sont obtenus.

Etape 2 : Synthèse de la N-(*S*)-α-méthylbenzyl-N'-(3-bromophényl)-S-méthylisothiourée :

Un mélange de 11,5 g (34 mmol) du composé obtenu précédemment et de 7,24 g (51 mmol) d'iodure de méthyle est agité dans 100 $cm^3$ de 2-butanone à 20 °C pendant 10 heures. Après que le mélange réactionnel ait été concentré à sec sous vide, le résidu obtenu est trituré dans 200 $cm^3$ d'une solution d'éther éthylique, puis est séparé par filtration. 15,1 g (rendement 92 %) du dérivé S-méthylisothiouréido sont obtenus sous forme d'hydroiodure. Ce sel est alors dissous dans 200 $cm^3$ d'une solution 1N d'hydroxyde de sodium. Le mélange alcalin résultant est extrait par du dichlorométhane (3 x 100 $cm^3$). Après séchage sur du sulfate de sodium anhydre et concentration à sec, 9,5 g (rendement 86 %) du dérivé isothiouréido sont obtenus, sous forme d'une huile incolore.

Etape 3 : Synthèse de l'acide N-[N-(*S*)-α-méthylbenzylamino(3-bromophénylimino)méthyl]-2-aminoéthanoïque :

Un mélange de 3 g (40,8 mmol) de glycine et de 1,6 g (40,8 mmol) d'hydroxyde de sodium dans 5 $cm^3$ d'eau est ajouté à une solution de 9,5 g (27,2 mmol) de N-(*S*)-α-méthylbenzyl-N'-(3-bromophényl)-S-méthylisothiourée dans 100 $cm^3$ d'éthanol à 95 %. Le mélange est chauffé à 70 °C durant 20 heures. Après refroidissement, la solution est concentrée à sec et le résidu est dissous dans 60 $cm^3$ d'eau. La solution résultante est lavée par du dichlorométhane (3 x 50 $cm^3$) puis acidifiée par une solution d'HCl 6N jusqu'à ce qu'un pH de 7 soit obtenu. Le dérivé guanidino désiré précipite et est alors isolé par filtration. 6,4 g (rendement 62 %) du produit sont obtenus (point de fusion 212 °C).

Le pouvoir édulcorant de ce composé correspond approximativement, sur une base pondérale, à 25 000 (vingt cinq mille) fois celui du saccharose (par comparaison avec une solution de saccharose à 2 %).

EXEMPLE 3 :

Synthèse de l'acide N-[N-(*S*)-α-méthylbenzylamino (3-trifluorométhylphénylimino)méthyl]-2-aminoéthanoïque :

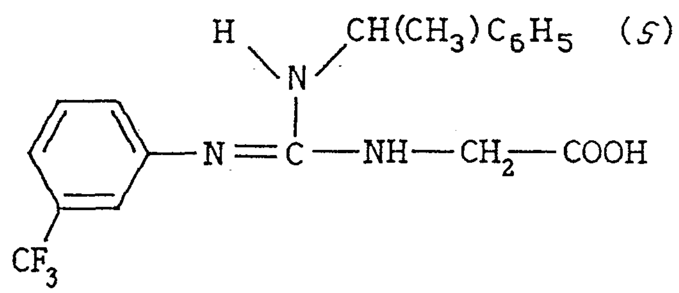

Ce composé est obtenu à partir de la glycine et de la N-(*S*)-α-méthylbenzyl-N'-(3-trifluorométhylphényl)-S-méthylisothiourée, en suivant le protocole expérimental décrit dans l'exemple 2 (rendement 34 %, point de fusion 158 °C).

Le pouvoir édulcorant de ce composé correspond approximativement, sur une base pondérale, à 7 500 (sept mille cinq cents) fois celui du saccharose (par comparaison avec une solution de saccharose à 2 %).

EXEMPLE 4 :

Synthèse de l'acide N-[N(*S*)-α-méthylbenzylamino (3-méthylphénylimino)méthyl]-2-aminoéthanoïque :

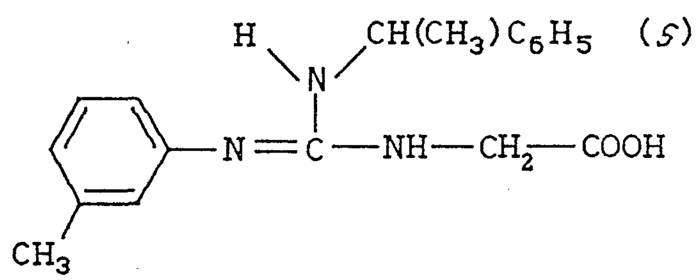

Ce composé est obtenu à partir de la glycine et de la N-(*S*)-α-méthylbenzyl-N'-(3-méthylphényl)-S-méthyl isothiourée, en suivant le protocole expérimental décrit dans l'exemple 2 (rendement 28 %, point de fusion 205 °C).

Le pouvoir édulcorant de ce composé correspond approximativement, sur une base pondérale, à 12 000 (douze mille) fois celui du saccharose (par comparaison avec une solution de saccharose à 2 %).

EXEMPLE 5 :

Synthèse de l'acide N-[N-(*S*)-α-méthylbenzylamino (3-éthylphénylimino)méthyl]-2-aminoéthanoïque :

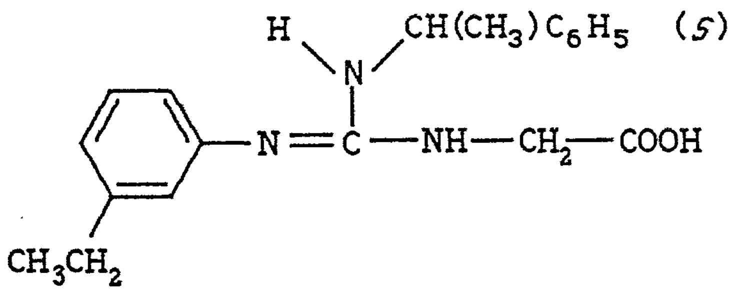

Ce composé est obtenu à partir de la glycine et de la N-(*S*)-α-méthylbenzyl-N'-(3-éthylphényl)-S-méthyl isothiourée, en suivant le protocole expérimental décrit dans l'exemple 2 (rendement 42 %, point de fusion 205 °C).

Le pouvoir édulcorant de ce composé correspond approximativement, sur une base pondérale, à 4 500 (quatre mille cinq cents) fois celui du saccharose (par comparaison avec une solution de saccharose à 2 %).

EXEMPLE 6 :

Synthèse de l'acide N-[N-(*S*)-α-méthylbenzylamino (3-chlorophénylimino)méthyl]-2-aminoéthanoïque :

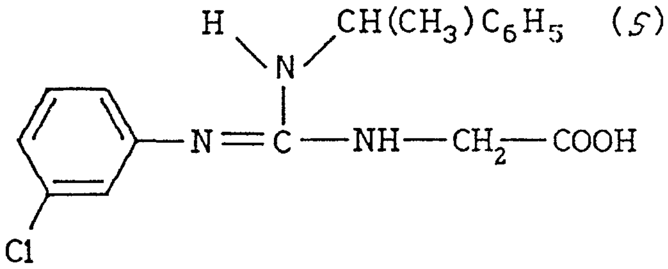

Ce composé est obtenu à partir de la glycine et de la N-(*S*)-α-méthylbenzyl-N'-(3-chlorophényl)-S-méthyl isothiourée, en suivant le protocole expérimental décrit dans l'exemple 2 (rendement 22 %, point de fusion 178 °C).

Le pouvoir édulcorant de ce composé correspond approximativement, sur une base pondérale, à 30 000 (trente mille) fois celui du saccharose (par comparaison avec une solution de saccharose à 2 %).

EXEMPLE 7 :

Synthèse de l'acide N-[N-(*S*)-α-méthylbenzylamino (3-cyanophénylimino)méthyl]-2-aminoéthanoïque :

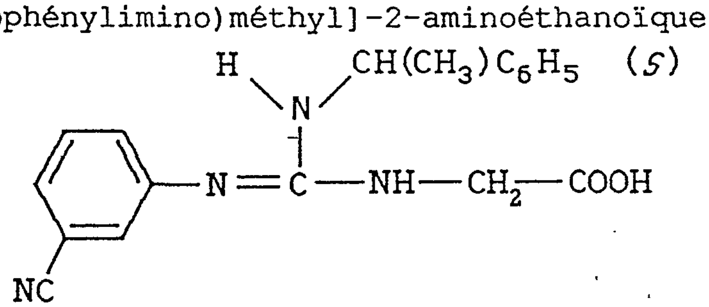

Ce composé est obtenu à partir de la glycine et de la N-(*S*)-α-méthylbenzyl-N'-(3-cyanophényl)-S-méthyl isothiourée, en suivant le protocole expérimental décrit dans l'exemple 2 (rendement 63 %, point de fusion 190 °C).

Le pouvoir édulcorant de ce composé correspond approximativement, sur une base pondérale, à 5 500 (cinq mille cinq cents) fois celui du saccharose (par comparaison avec une solution de saccharose à 2 %).

EXEMPLE 8 :

Synthèse de l'acide N-[N-(*S*)-α-méthylbenzylamino (3-nitrophénylimino)méthyl]-2-aminoéthanoïque :

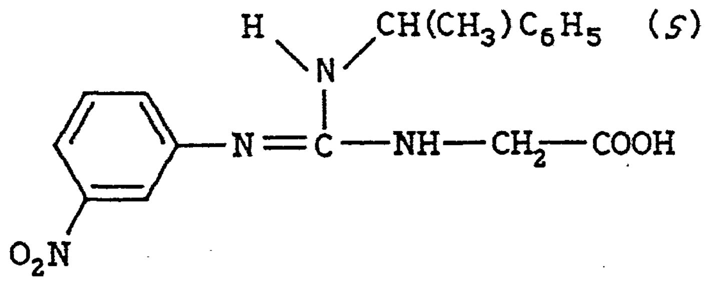

Ce composé est obtenu à partir de la glycine et la N-(*S*)-α-méthylbenzyl-N'-(3-nitrophényl)-S-méthylisothiourée, en suivant le protocole expérimental décrit dans l'exemple 2 (rendement 39 %, point de fusion 195 °C).

Le pouvoir édulcorant de ce composé correspond approximativement, sur une base pondérale, à 6 000 (six mille) fois celui du saccharose (par comparaison avec une solution de saccharose à 2 %).

EXEMPLE 9 :

Synthèse de l'acide N-[N-(*S*)-α-méthylbenzylamino (3-méthoxyphénylimino)méthyl]-2-aminoéthanoïque :

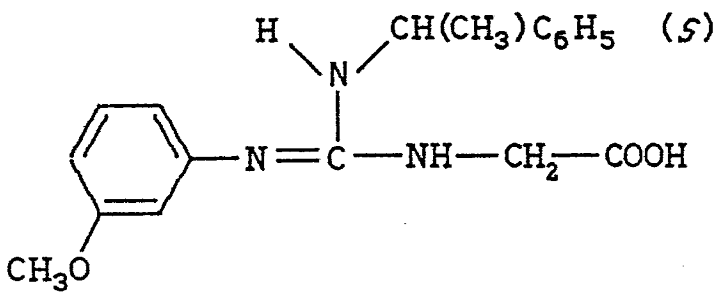

Ce composé est obtenu à partir de la glycine et de la N-(*S*)-α-méthylbenzyl-N'-(3-méthoxyphényl)-S-méthyl isothiourée, en suivant le protocole expérimental décrit dans l'exemple 2 (rendement 78 %, point de fusion 191 °C).

Le pouvoir édulcorant de ce composé correspond approximativement, sur une base pondérale, à 4 500 (quatre mille cinq cents) fois celui du saccharose (par comparaison avec une solution de saccharose à 2 %).

EXEMPLE 10 :

Synthèse de l'acide N-[N-(*S*)-α-méthylbenzylamino (3-méthylmercaptophénylimino)méthyl]-2-aminoéthanoïque :

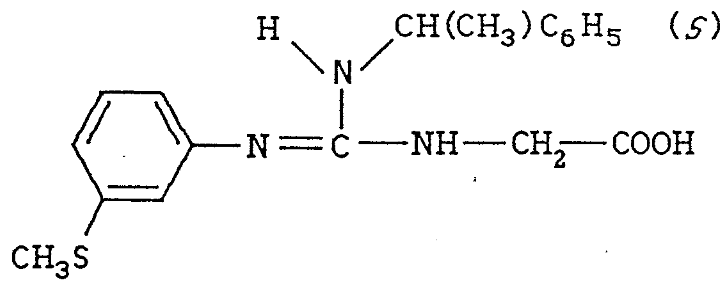

Ce composé est obtenu à partir de la glycine et de la N-(*S*)-α-méthylbenzyl-N'-(3-méthylmercaptophényl)-S-méthylisothiourée, en suivant le protocole expérimental décrit dans l'exemple 2 (rendement 59 %, point de fusion 186 °C).

Le pouvoir édulcorant de ce composé correspond approximativement, sur une base pondérale, à 5 500 (cinq mille cinq cents) fois celui du saccharose (par comparaison avec une solution de saccharose à 2 %).

EXEMPLE 11 :

Synthèse de l'acide N-[N-(*S*)-α-méthylbenzylamino (4-trifluorométhylphénylimino)méthyl]-2-aminoéthanoïque :

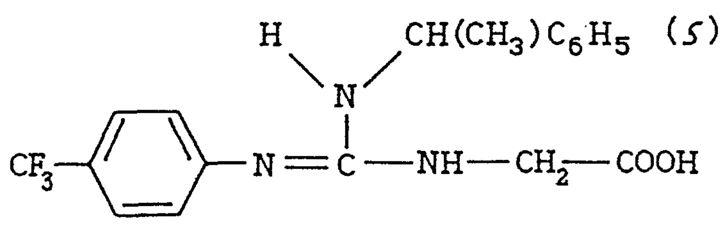

Ce composé est obtenu à partir de la glycine et de la N-(*S*)-α-méthylbenzyl-N'-(4-trifluorométhylphényl)-S-méthylisothiourée, en suivant le protocole expérimental décrit dans l'exemple 2 (rendement 9 %, point de fusion 168 °C).

Le pouvoir édulcorant de ce composé correspond approximativement, sur une base pondérale, à 500 (cinq cents) fois celui du saccharose (par comparaison avec une solution de saccharose à 2 %).

EXEMPLE 12 :

Synthèse de l'acide N-[N-(*S*)-α-méthylbenzylamino (4-méthylphénylimino)méthyl]-2-aminoéthanoïque :

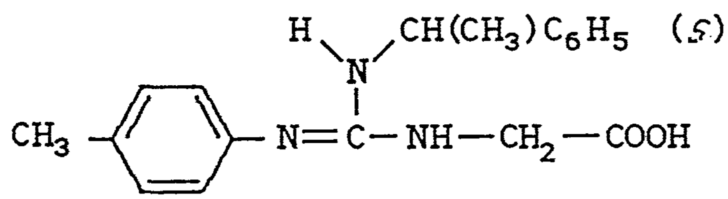

Ce composé est obtenu à partir de la glycine et de la N-(*S*)-α-méthylbenzyl-N'-(4-méthylphényl)-S-méthyl isothiourée, en suivant le protocole expérimental décrit dans l'exemple 2 (rendement 26 %, point de fusion 137 °C).

Le pouvoir édulcorant de ce composé correspond approximativement, sur une base pondérale, à 800 (huit cents) fois celui du saccharose (par comparaison avec une solution de saccharose à 2 %).

EXEMPLE 13 :

Synthèse de l'acide N-[N-(S)-α-méthylbenzylamino (4-chlorophénylimino)méthyl]-2-aminoéthanoïque :

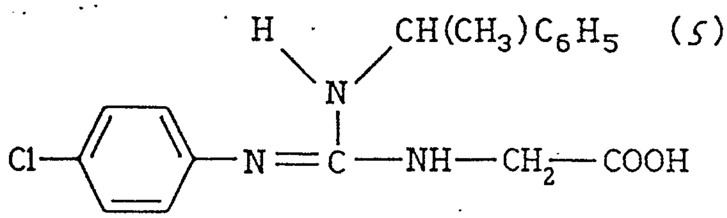

Ce composé est obtenu à partir de la glycine et de la N-(*S*)-α-méthylbenzyl-N'-(4-chlorophényl)-S-méthyl isothiourée, en suivant le protocole expérimental décrit dans l'exemple 2 (rendement 42 %, point de fusion 146 °C).

Le pouvoir édulcorant de ce composé correspond approximativement, sur une base pondérale, à 3 000 (trois mille) fois celui du saccharose (par comparaison avec une solution de saccharose à 2 %).

EXEMPLE 14 :

Synthèse de l'acide N-[N-(*S*)-α-méthylbenzylamino (4-cyanophénylimino)méthyl]-2-aminoéthanoïque :

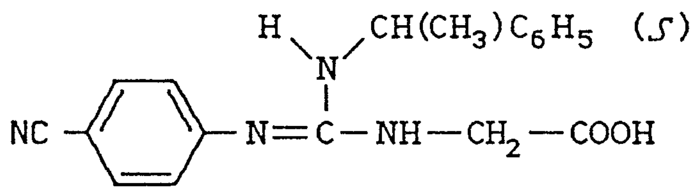

Ce composé est obtenu à partir de la glycine et de la N-(*S*)-α-méthylbenzyl-N'-(4-cyanophényl)-*S*-méthyl isothiourée, en suivant le protocole expérimental décrit dans l'exemple 2 [ rendement 35 %, $[\alpha]_D^{20}$ +112,5° (c = 1, HCl 0,5 N), point de fusion 206 °C ].

Le pouvoir édulcorant de ce composé correspond approximativement, sur une base pondérale, à 28 000 (vingt huit mille) fois celui du saccharose par rapport à une solution de saccharose à 2 %, à 20 000 (vingt mille) fois par rapport à une solution de saccharose à 5 %, à 10 000 (dix mille) fois par rapport à une solution de saccharose à 10 %.

EXEMPLE 15 :

Synthèse de l'acide N-[N-(*S*)-α-méthylbenzylamino (4-acétylphénylimino)méthyl]-2-aminoéthanoïque :

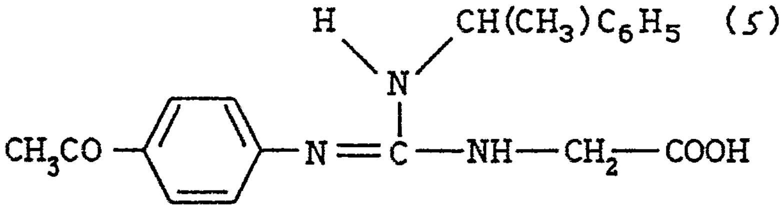

Ce composé est obtenu à partir de la glycine et de la N-(*S*)-α-méthylbenzyl-N'-(4-acétylphényl)-S-méthyl isothiourée, en suivant le protocole expérimental décrit dans l'exemple 2 (rendement 24 %, point de fusion 173 °C).

Le pouvoir édulcorant de ce composé correspond approximativement, sur une base pondérale, à 800 (huit cents) fois celui du saccharose (par comparaison avec une solution de saccharose à 2 %).

EXEMPLE 16 :

Synthèse de l'acide N-[N-(*S*)-α-méthylbenzylamino (4-méthoxycarbonylphénylimino)méthyl]-2-aminoéthanoïque :

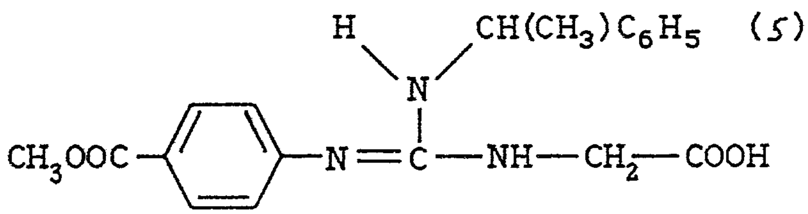

Ce composé est obtenu à partir de la glycine et de la N-(*S*)-α-méthylbenzyl-N'-(4-méthoxycarbonylphényl)-S-méthylisothiourée, en suivant le protocole expérimental décrit dans l'exemple 2 (rendement 28 %, point de fusion 163 °C).

Le pouvoir édulcorant de ce composé correspond approximativement, sur une base pondérale, à 700 (sept cents) fois celui du saccharose (par comparaison avec une solution de saccharose à 2 %).

EXEMPLE 17 :

Synthèse de l'acide N-[N-(*S*)-α-méthylbenzylamin (4-fluorophénylimino)méthyl]-2-aminoéthanoïque :

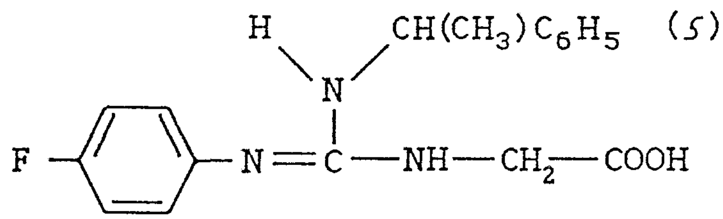

Ce composé est obtenu à partir de la glycine et de la N-(*S*)-α-méthylbenzyl-N'-(4-fluorophényl)-S-méthylisothiourée, en suivant le protocole expérimental décrit dans l'exemple 2 (rendement 15 %, point de fusion 166 °C).

Le pouvoir édulcorant de ce composé correspond approximativement, sur une base pondérale, à 4 000 (quatre mille) fois celui du saccharose (par comparaison avec une solution de saccharose à 2 %).

EXEMPLE 18 :

Synthèse de l'acide N-[N-(*S*)-α-méthylbenzylamino (4-nitrophénylimino)méthyl]-2-aminoéthanoïque :

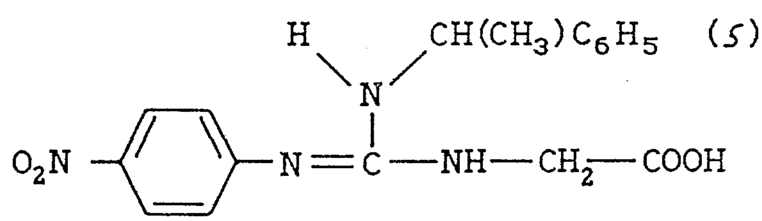

Ce composé est obtenu à partir de la glycine et de la N-(*S*)-α-méthylbenzyl-N'-(4-nitrophényl)-S-méthylisothiourée, en suivant le protocole expérimental décrit dans l'exemple 2 (rendement 85 %, point de fusion 198 °C).

Le pouvoir édulcorant de ce composé correspond approximativement, sur une base pondérale, à 7 000 (sept mille) fois celui du saccharose (par comparaison avec une solution de saccharose à 2 %).

EXEMPLE 19 :

Synthèse de l'acide N-[N-(*S*)-α-méthylbenzylamino (4-méthoxyphénylimino)méthyl]-2-aminoéthanoïque :

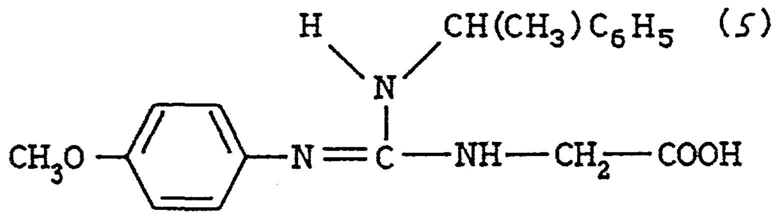

Ce composé est obtenu à partir de la glycine et de la N-(*S*)-α-méthylbenzyl-N'-(4-méthoxyphényl)-S-méthylisothiourée, en suivant le protocole expérimental décrit dans l'exemple 2 (rendement 22 %, point de fusion 195 °C).

Le pouvoir édulcorant de ce composé correspond approximativement, sur une base pondérale, à 2 100 (deux mille cent) fois celui du saccharose (par comparaison avec une solution de saccharose à 2 %).

EXEMPLE 20 :

Synthèse de l'acide N-[N-(*S*)-α-méthylbenzylamino (4-hydroxyphénylimino)méthyl]-2-aminoéthanoïque :

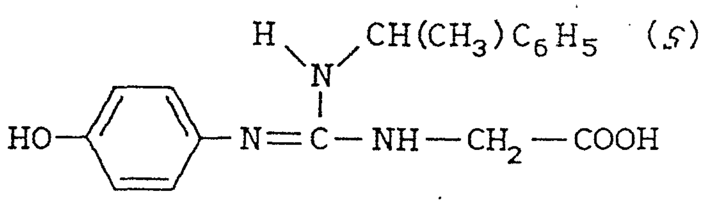

Ce composé est obtenu à partir de la glycine et de la N-(*S*)-α-méthylbenzyl-N'-(4-hydroxyphényl)-S-méthylisothiourée, en suivant le protocole expérimental décrit dans l'exemple 2 (rendement 23 %, point de fusion 160 °C).

Le pouvoir édulcorant de ce composé correspond approximativement, sur une base pondérale, à 400 (quatre cents) fois celui du saccharose (par comparaison avec une solution de saccharose à 2 %).

EXEMPLE 21 :

Synthèse de l'acide N-[N-(S)-α-méthylbenzylamino (4-méthylmercaptophénylimino)méthyl]-2-aminoéthanoïque :

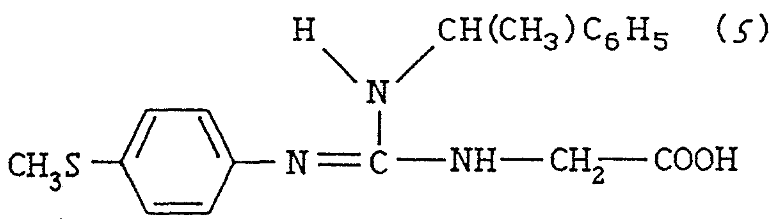

Ce composé est obtenu à partir de la glycine et de la N-(*S*)-α-méthylbenzyl-N'-(4-méthylmercaptophényl)-S-méthylisothiourée, en suivant le protocole expérimental décrit dans l'exemple 2 (rendement 77 %, point de fusion 171 °C).

Le pouvoir édulcorant de ce composé correspond approximativement, sur une base pondérale, à 1 200 (mille deux cents) fois celui du saccharose (par comparaison avec une solution de saccharose à 2 %).

EXEMPLE 22 :

Synthèse de l'acide N-[N-(*S*)-α-méthylbenzylamino (4-cyano-3-méthylphénylimino)méthyl]-2-aminoéthanoïque :

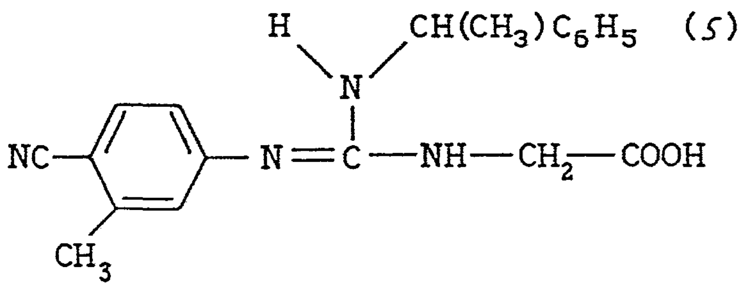

Ce composé est obtenu à partir de la glycine et de la N-(*S*)-α-méthylbenzyl-N'-(4-cyano-3-méthylphényl)-S-méthylisothiourée, en suivant le protocole expérimental décrit dans l'exemple 2 (rendement 46 %, point de fusion 172 °C).

Le pouvoir édulcorant de ce composé correspond approximativement, sur une base pondérale, à 50 000 (cinquante mille) fois celui du saccharose (par comparaison avec une solution de saccharose à 2 %).

EXEMPLE 23 :

Synthèse de l'acide N-[N-(*S*)-α-méthylbenzylamino (3,4-dichlorophénylimino)méthyl]-2-aminoéthanoïque :

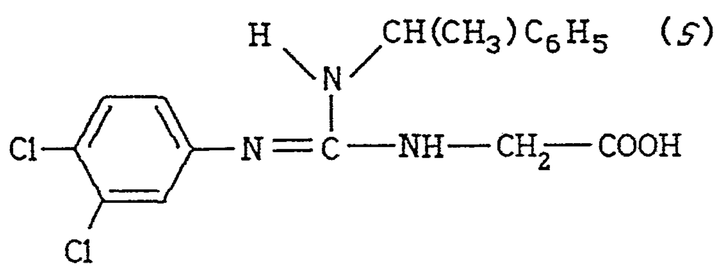

Ce composé est obtenu à partir de la glycine et de la N-(*S*)-α-méthylbenzyl-N'-(3,4-dichlorophényl)-S-méthylisothiourée, en suivant le protocole expérimental décrit dans l'exemple 2 (rendement 20 %, point de fusion 195 °C).

Le pouvoir édulcorant de ce composé correspond approximativement, sur une base pondérale, à 17 000 (dix sept mille) fois celui du saccharose (par comparaison avec une solution de saccharose à 2 %).

EXEMPLE 24 :

Synthèse de l'acide N-[N-(*S*)-α-méthylbenzylamino (3-trifluorométhyl-5-méthoxyphénylimino)méthyl]-2-amino-éthanoïque :

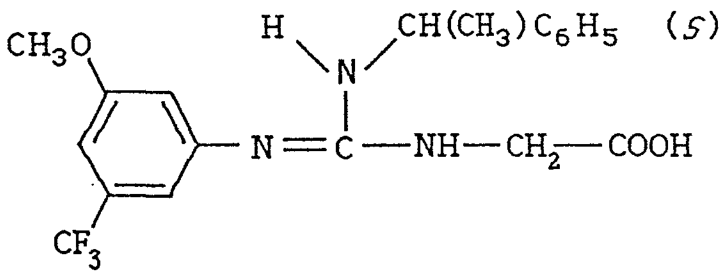

Ce composé est obtenu à partir de la glycine et de la N-(*S*)-α-méthylbenzyl-N'-(3-trifluorométhyl-5-méthoxyphényl)-S-méthylisothiourée, en suivant le protocole expérimental décrit dans l'exemple 2 (rendement 71 %, point de fusion 192 °C).

Le pouvoir édulcorant de ce composé correspond approximativement, sur une base pondérale, à 7 000 (sept mille) fois celui du saccharose (par comparaison avec une solution de saccharose à 2 %).

EXEMPLE 25 :

Synthèse de l'acide N-[N-(*S*)-α-méthylbenzylamino (3,5-diméthylphénylimino)méthyl]-2-aminoéthanoïque :

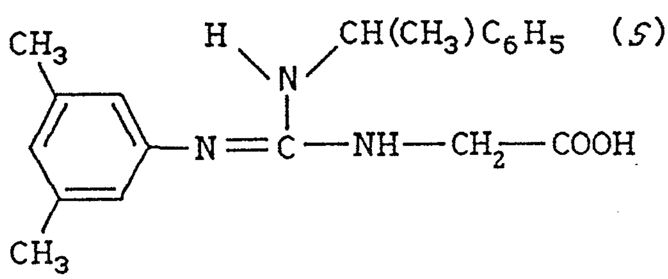

Ce composé est obtenu à partir de la glycine et de la N-(*S*)-α-méthylbenzyl-N'-(3,5-diméthylphényl)-S-méthylisothiourée, en suivant le protocole expérimental décrit dans l'exemple 2 (rendement 67 %, point de fusion 225 °C).

Le pouvoir édulcorant de ce composé correspond approximativement, sur une base pondérale, à 30 000 (trente mille) fois celui du saccharose (par comparaison avec une solution de saccharose à 2 %).

EXEMPLE 26 :

Synthèse de l'acide N-[N-(*S*)-α-méthylbenzylamino (3,5-dichlorophénylimino)méthyl]-2-aminoéthanoïque :

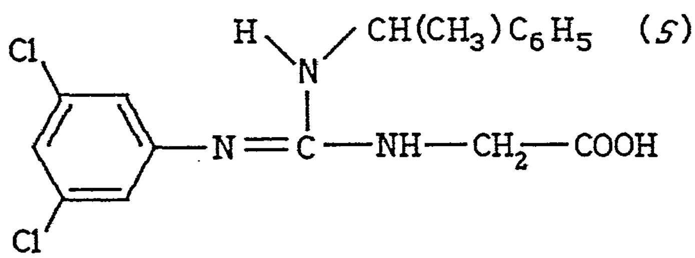

Ce composé est obtenu à partir de la glycine et de la N-(*S*)-α-méthylbenzyl-N'-(3,5-dichlorophényl)-S-méthylisothiourée, en suivant le protocole expérimental décrit dans l'exemple 2 (rendement 46 %, point de fusion 202 °C).

Le pouvoir édulcorant de ce composé correspond approximativement, sur une base pondérale, à 120 000 (cent ving mille) fois celui du saccharose (par comparaison avec une solution de saccharose à 2 %), à 90 000 (quatre vingt dix mille) fois par rapport à une solution à 5 %, et à 50 000 (cinquante mille) fois par rapport à une solution à 10 %.

EXEMPLE 27 :

Synthèse de l'acide N-[N-(*S*)-α-méthylbenzylamino (3,5-difluorophénylimino)méthyl]-2-aminoéthanoïque :

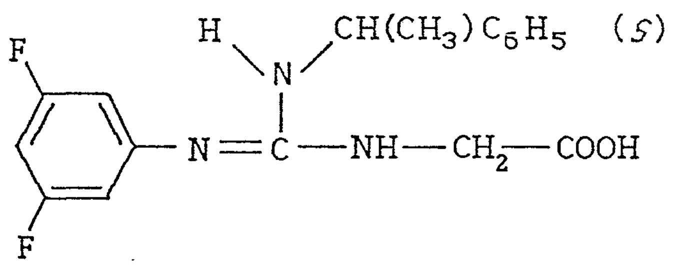

Ce composé est obtenu à partir de la glycine et de la N-(*S*)-α-méthylbenzyl-N'-(3,5-difluorophényl)-S-méthylisothiourée, en suivant le protocole expérimental décrit dans l'exemple 2 (rendement 66 %, point de fusion 212 °C).

Le pouvoir édulcorant de ce composé correspond approximativement, sur une base pondérale, à 15 000 (quinze mille) fois celui du saccharose (par comparaison avec une solution de saccharose à 2 %).

EXEMPLE 28 :

Synthèse de l'acide N-[N-(*S*)-α-méthylbenzylamino (3,4,5-trichlorophénylimino)méthyl]-2-aminoéthanoïque :

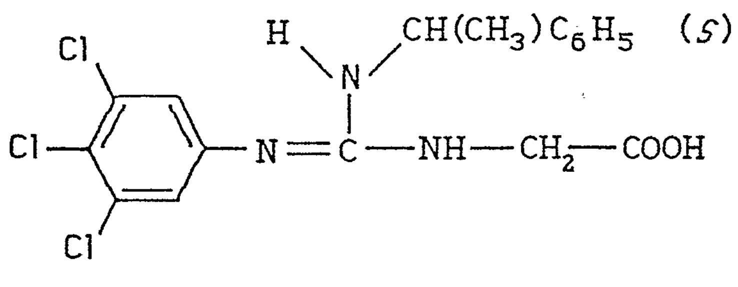

Ce composé est obtenu à partir de la glycine et de la N-(*S*)-α-méthylbenzyl-N'-(3,4,5-trichlorophényl)-S-méthylisothiourée, en suivant le protocole expérimental décrit dans l'exemple 2 (rendement 69 %, point de fusion 208 °C).

Le pouvoir édulcorant de ce composé correspond approximativement, sur une base pondérale, à 35 000 (trente cinq mille) fois celui du saccharose (par comparaison avec une solution de saccharose à 2 %).

EXEMPLE 29 :

Synthèse de l'acide N-[2-nitro(3,5-dichloro phénylamino)éthényl]-2-aminoéthanoïque :

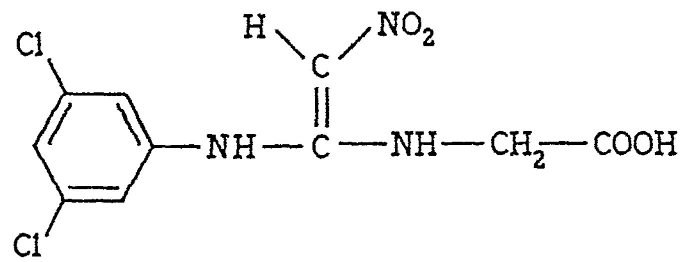

5 g (30 mmol) de 1-nitro-2,2-(méthylthio)éthène et 4,91 g (30 mmol) de 3,5-dichloroaniline sont mélangés dans 50 $cm^3$ d'éthanol à 95 %. La solution est chauffée au reflux durant 6 heures. Après refroidissement, le précipité formé est filtré, lavé par l'éther éthylique (3 x 20 $cm^3$). 5,3 g (rendement 63 %) de 1-nitro-2-(3,5-dichlorophénylamino)-2-(méthylthio)éthène (point de fusion 114 °C) sont ainsi obtenus :

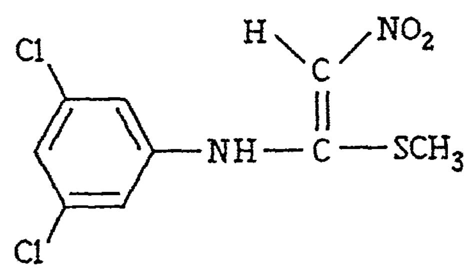

1,34 g (17,9 mmol) de glycine, 5 g (17,9 mmol) de 1-nitro-2-(3,5-dichlorophénylamino)-2-(méthylthio)éthène et 2,5 $cm^3$ (17,9 mmol) de triéthylamine sont mélangés dans 50 $cm^3$ d'éthanol-eau (5-1). La solution est chauffée au reflux durant 2 heures. Après concentration sous vide, le résidu obtenu est dissous dans 60 $cm^3$ d'une solution 1N d'hydroxyde de sodium. La solution résultante est lavée par l'acétate d'éthyle (4 x 20 $cm^3$) puis acidifiée par une solution de chlorure d'hydrogène 6N jusqu'à l'obtention d'un pH voisin de 7.

Le solide obtenu est filtré, lavé par l'eau (2 x 2 $cm^3$) et séché sous vide. 1,4 g (rendement 26 %) d'acide N-[2-nitro(3,5-dichlorophénylamino)éthényl]-2-aminoéthanoïque (point de fusion 207 °C) est obtenu.

Le pouvoir édulcorant du composé correspond approximativement, sur une base pondérale, à 400 (quatre cents) fois celui du saccharose (par comparaison avec une solution de saccharose à 2 %)

EXEMPLE 30 :

Synthèse de l'acide N-[2,2-dicyano(3,5-dichloro phénylamino)éthényl]-2-aminoéthanoïque :

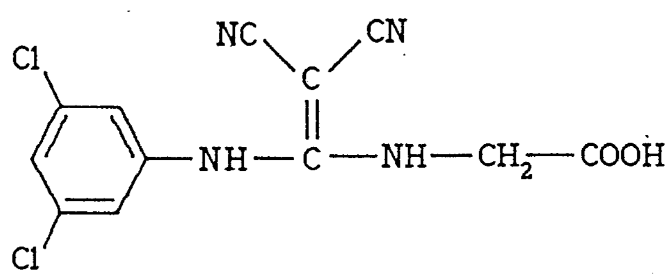

1,64 g d'hydrure de sodium (en dispersion à 50 % dans la paraffine liquide) est ajouté, en plusieurs fractions, à une solution, refroidie à 0 °C, de 2,26 g (34 mmol) de malononitrile dissous dans 25 $cm^3$ de diméthylformamide. La solution est ensuite maintenue

10 minutes à 10 °C. 7 g (34 mmol) de 3,5-dichlorophényl isothiocyanate dissous dans 20 $cm^3$ de diméthylformamide sont ajoutés à la solution. Le mélange réactionnel est maintenu durant 15 minutes à 20 °C, puis concentré à sec sous vide. Le résidu, après trituration à chaud dans du chloroforme (5 x 50 $cm^3$), est séché sous vide. 6 g du solide ainsi obtenu et 2,84 g de diméthyl sulfate sont dissous dans 200 $cm^3$ d'éthanol à 95 % et laissés en contact durant 2 heures à 20 °C. Après élimination de l'éthanol et lavage du solide restant par de l'eau (4 x 50 $cm^3$), 4,3 g (rendement 74 %) de 1,1-dicyano-2-(3,5-dichlorophénylamino)-2-(méthylthio)-éthène sont obtenus après séchage :

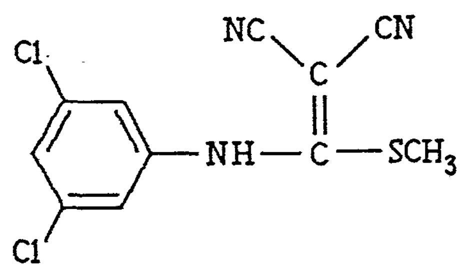

1,58 g (21,1 mmol) de glycine, 4 g (21,1 mmol) du composé précédemment obtenu et 2,7 g (21,1 mmol) de N,N-diisopropyléthylamine sont mélangés dans 200 $cm^3$ d'éthanol. Le mélange réactionnel est chauffé à 70 °C durant 24 heures, puis est concentré à sec sous vide. Le résidu obtenu est dissous dans 200 $cm^3$ d'une solution aqueuse de carbonate de sodium à 2 %. La solution est lavée par l'éther éthylique (3 x 50 $cm^3$), puis acidifié par une solution d'HCl 3N. Le précipité pâteux obtenu est séparé par décantation. Après purification par chromatographie, 0,4 g (rendement 10 %) d'acide N-[2,2-dicyano(3,5-dichlorophénylamino)éthényl]-2-amino-éthanoïque (point de fusion 103 °C) est obtenu.

Le pouvoir édulcorant du composé correspond approximativement, sur une base pondérale, à 30 (trente) fois celui du saccharose (par comparaison avec une solution de saccharose à 2 %).

EXEMPLE 31 :

Synthèse de l'acide N-[N-méthyl-N-benzylamino (3,5-dichlorophénylimino)méthyl]-2-aminoéthanoïque :

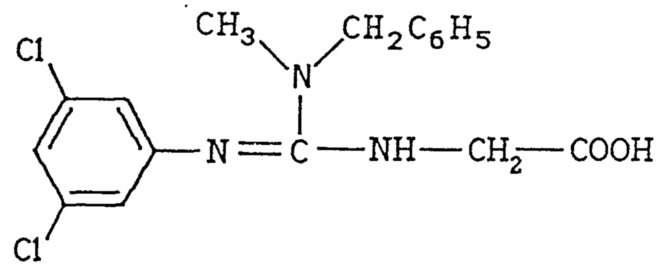

Ce composé est obtenu à partir de la glycine et de la N-méthyl-N-benzyl-N'-3,5-dichlorophényl-S-méthyl isothiourée, en suivant le protocole expérimental décrit dans l'exemple 2 (rendement 10 %, point de fusion 153 °C).

Le pouvoir édulcorant de ce composé correspond approximativement, sur une base pondérale, à 20 000 (vingt mille) fois celui du saccharose (par comparaison avec une solution de saccharose à 2 %).

EXEMPLE 32 :

Synthèse de l'acide N-[N-méthyl-N-1-méthyléthyl-amino(4-cyanophénylimino)méthyl]-2-aminoéthanoïque :

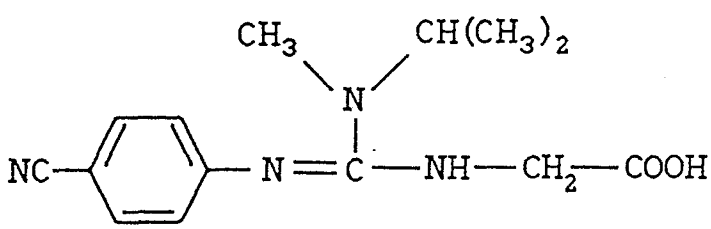

Ce composé est obtenu à partir de la glycine et de la N-méthyl-N-1-méthyléthyl-N'-(4-cyanophényl)-S-méthylisothiourée, en suivant le protocole expérimental décrit dans l'exemple 2 (rendement 5 %, point de fusion 120 °C).

Le pouvoir édulcorant de ce composé correspond approximativement, sur une base pondérale, à 80 (quatre vingt) fois celui du saccharose (par comparaison avec une solution de saccharose à 2 %).

EXEMPLE 33 :

Synthèse de l'acide N-[N-méthyl-N-benzylamino(4-cyanophénylimino)méthyl]-2-aminoéthanoïque :

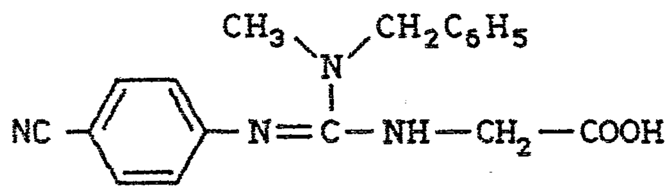

Ce composé est obtenu à partir de la glycine et de la N-méthyl-N-benzyl-N'-(4-cyanophényl)-S-méthylisothiourée, en suivant le protocole expérimental décrit dans l'exemple 2 (rendement 22 %, point de fusion 133 °C).

Le pouvoir édulcorant de ce composé correspond approximativement, sur une base pondérale, à 3 000 (trois mille) fois celui du saccharose par rapport à une solution de saccharose à 2 %, à 1 800 (mille huit cents) par rapport à une solution de saccharose à 5 %, à 1 200 (mille deux cents) par rapport à une solution de saccharose à 10 %.

EXEMPLE 34 :

Synthèse de l'acide N-[N-éthyl-N-benzylamino (4-cyanophénylimino)méthyl]-2-aminoéthanoïque :

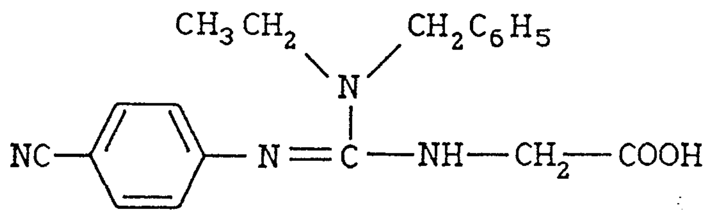

Ce composé est obtenu à partir de la glycine et de la N-éthyl-N-benzyl-N'-(4-cyanophényl)-S-méthylisothiourée, en suivant le protocole expérimental décrit dans l'exemple 2 (rendement 5 %, point de fusion 147 °C).

Le pouvoir édulcorant de ce composé correspond approximativement, sur une base pondérale, à 3 000 (trois mille) fois celui du saccharose (par rapport à une solution de saccharose à 2 %).

EXEMPLE 35 :

Synthèse de l'acide N-[N-éthylamino(4-cyanophényl imino)méthyl]-2-aminoéthanoïque :

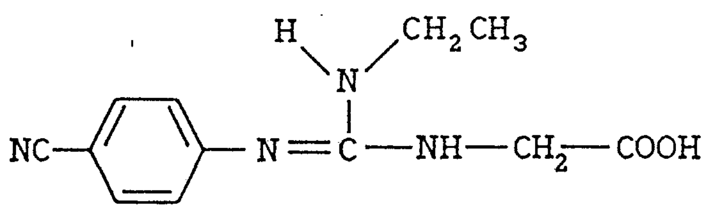

Ce composé est obtenu à partir de la glycine et de la N-éthyl-N'-(4-cyanophényl)-S-méthylisothiourée, en suivant le protocole expérimental décrit dans l'exemple 2 (rendement 18 %, point de fusion 185 °C).

Le pouvoir édulcorant de ce composé correspond approximativement, sur une base pondérale, à 350 (trois cent cinquante) fois celui du saccharose (par rapport à une solution de saccharose à 2 %).

EXEMPLE 36 :

Synthèse de l'acide N-[N-hexylamino(4-cyanophényl imino)méthyl]-2-aminoéthanoïque :

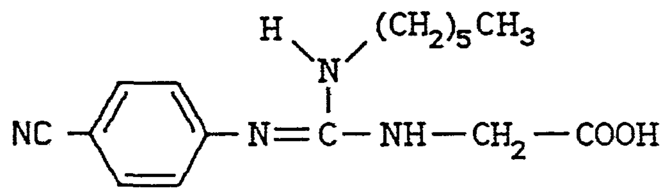

Ce composé est obtenu à partir de la glycine et de la N-hexyl-N'-(4-cyanophényl)-S-méthylisothiourée, en suivant le protocole expérimental décrit dans l'exemple 2 (rendement 17 %, point de fusion 159 °C).

Le pouvoir édulcorant de ce composé correspond approximativement, sur une base pondérale, à 6 000 (six mille) fois celui du saccharose par rapport à une solution de saccharose à 2 %, à 4 300 (quatre mille trois cents) fois par rapport à une solution de saccharose à 5 %, à 1 400 (mille quatre cents) fois par rapport à une solution de saccharose à 10 %.

EXEMPLE 37 :

Synthèse de l'acide N-[N-(3-méthylbutyl)amino (4-cyanophénylimino)méthyl]-2-aminoéthanoïque :

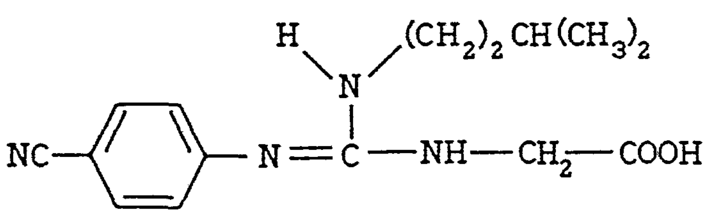

Ce composé est obtenu à partir de la glycine et de la N-(3-méthylbutyl)-N'-(4-cyanophényl)-S-méthylisothiourée, en suivant le protocole expérimental décrit

dans l'exemple 2 (rendement 16 %; point de fusion 192 °C).

Le pouvoir édulcorant de ce composé correspond approximativement, sur une base pondérale, à 200 (deux cents) fois celui du saccharose (par rapport à une solution de saccharose à 2 %).

EXEMPLE 38 :

Synthèse de l'acide N-[N-(1,1-diméthylpropyl) amino(4-cyanophénylimino)méthyl]-2-aminoéthanoïque :

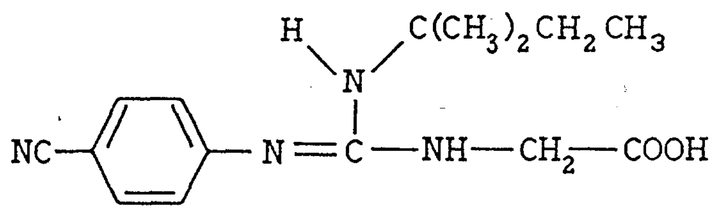

Ce composé est obtenu à partir de la glycine et de la N-(1,1-diméthylpropyl)-N'-(4-cyanophényl)-S-méthylisothiourée, en suivant le protocole expérimental décrit dans l'exemple 2 (rendement 16 %; point de fusion 210 °C).

Le pouvoir édulcorant de ce composé correspond approximativement, sur une base pondérale, à 300 (trois cents) fois celui du saccharose (par rapport à une solution de saccharose à 2 %).

EXEMPLE 39 :

Synthèse de l'acide N-[N-(1,1,3,3-tétraméthylbut-yl)amino(4-cyanophénylimino)méthyl]-2-aminoéthanoïque :

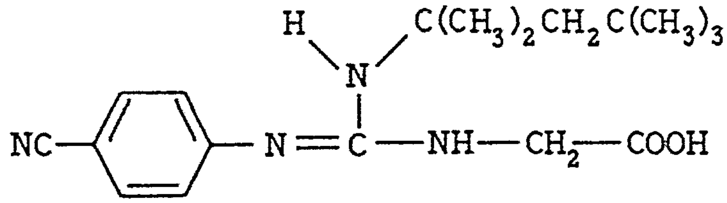

Ce composé est obtenu à partir de la glycine et de la N-(1,1,3,3-tétraméthylbutyl)-N'-(4-cyanophényl)-S-méthylisothiourée, en suivant le protocole expérimental décrit dans l'exemple 2 (rendement 43 %; point de fusion 189 °C).

Le pouvoir édulcorant de ce composé correspond approximativement, sur une base pondérale, à 1 200 (mille deux cents) fois celui du saccharose (par rapport à une solution de saccharose à 2 %).

EXEMPLE 40 :

Synthèse de l'acide N-[N-phénylamino(phénylimino)méthyl]-2-aminoéthanoïque :

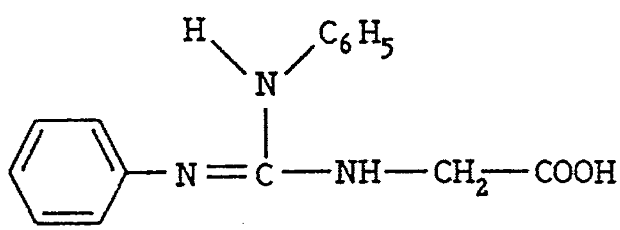

Ce composé est obtenu à partir de la glycine et de la N,N'-diphényl-S-méthylisothiourée, en suivant le protocole expérimental décrit dans l'exemple 2 (rendement 41 %; point de fusion 214 °C).

Le pouvoir édulcorant de ce composé correspond approximativement, sur une base pondérale, à 500 (cinq cents) fois celui du saccharose (par rapport à une solution de saccharose à 2 %).

EXEMPLE 41 :

Synthèse de l'acide N-[N-phénylamino(4-cyano phénylimino)méthyl]-2-aminoéthanoïque :

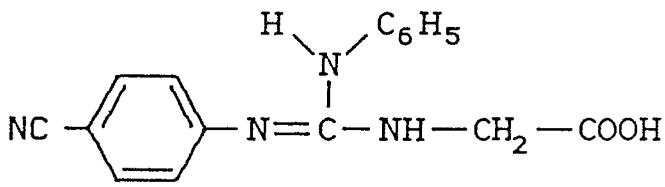

Ce composé est obtenu à partir de la glycine et de la N-phényl-N'-(4-cyanophényl)isothiourée en suivant le protocole expérimental décrit dans l'exemple 2 (rendement 33 %, point de fusion 142 °C).

Le pouvoir édulcorant de ce composé correspond approximativement, sur une base pondérale, à 4 000 (quatre mille) fois celui du saccharose par rapport à une solution de saccharose à 2 %, à 1 800 (mille huit cents) fois par rapport à une solution de saccharose à 5 %, à 650 (six cent cinquante) fois par rapport à une solution de saccharose à 10 %.

EXEMPLE 42 :

Synthèse de l'acide N-[N-cyclohexylamino(phényl imino)méthyl]-2-aminoéthanoïque :

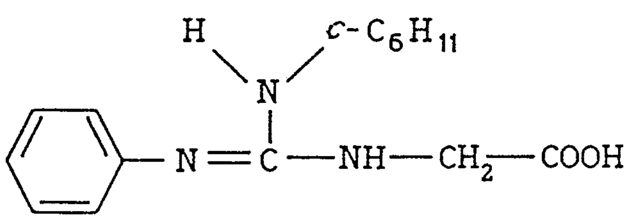

Etape 1 : Synthèse du N-cyclohexyl-N'-phényl carbodiimide :

Une solution contenant 2,0 g (8,55 mmol) de N-cyclohexyl-N'-phénylthiourée, 2,9 g (11,1 mmol) de triphényl phosphine et 1,16 $cm^3$ (12 mmol) de tétrachlorure de carbone dans 12 $cm^3$ de chlorure de

méthylène, est traitée avec 1,19 $cm^3$ (8,55 mmol) de triéthylamine. La solution est chauffée durant 2 heures, le précipité formé est éliminé par filtration et le filtrat est concentré à sec. Le résidu obtenu est extrait par l'hexane puis filtré. Les filtrats sont alors concentrés à sec, ce qui permet d'obtenir 1,7 g (rendement 100 %) du carbodiimide désiré sous forme d'huile.

Etape 2 : Synthèse de l'acide N-[N-cyclohexyl amino(phénylimino)méthyl]-2-aminoéthanoïque :

Une suspension de 1,6 g (8 mmol) du composé précédent et de 1,34 g (8 mmol) de glycine *t*-butyl ester hydrochlorure dans 20 $cm^3$ d'acétonitrile est chauffée au reflux durant 12 heures. La solution obtenue est alors concentrée à sec sous vide et le résidu huileux est trituré dans l'éther éthylique, ce qui permet d'obtenir, sous forme solide, 2,7 g (rendement 93%) de *t*-butyl N-[N-cyclohexylamino(phénylimino)méthyl]-2-aminoéthanoate.

Une solution de 2 g (5,45 mmol) du composé précédent dans 8 $cm^3$ d'acide acétique pur est agitée durant 1 heure à 20 °C en présence de 7,8 $cm^3$ (54,4 mmol) d'une solution 6,98 M de chlorure d'hydrogène dans le dioxane. Après concentration à sec sous vide et trituration du résidu dans l'éther éthylique, on obtient 1,6 g (rendement 94 %, point de fusion 160-165 °C) du composé désiré sous forme d'hydrochlorure.

Le pouvoir édulcorant de ce composé correspond approximativement, sur une base pondérale, à 100 (cent) fois celui du saccharose (par rapport à une solution de saccharose à 2 %).

EXEMPLE 43 :

Synthèse de l'acide N-[N-cyclohexylamino(4-cyano phénylimino)méthyl]-2-aminoéthanoïque :

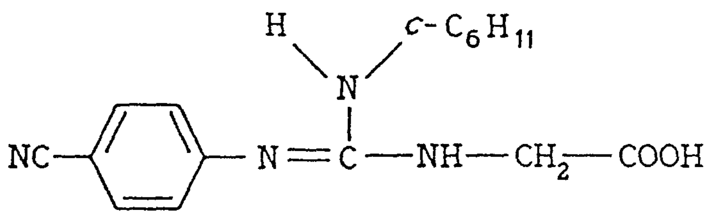

Ce composé est obtenu à partir de la glycine et de la N-cyclohexyl-N'-(4-cyanophényl)-S-méthylisothiourée, en suivant le protocole expérimental décrit dans l'exemple 2 (rendement 58 %, point de fusion 214 °C).

Le pouvoir édulcorant de ce composé correspond approximativement, sur une base pondérale, à 12 000 (douze mille) fois celui du saccharose par rapport à une solution de saccharose à 2 %, à 10 500 (dix mille cinq cents) fois par rapport à une solution de saccharose à 5 %, à 8 500 (huit mille cinq cents) fois par rapport à une solution de saccharose à 10 %.

EXEMPLE 44 :

Synthèse de l'acide N-[N-cycloheptylamino (3,5-dichlorophénylimino)méthyl]-2-aminoéthanoïque :

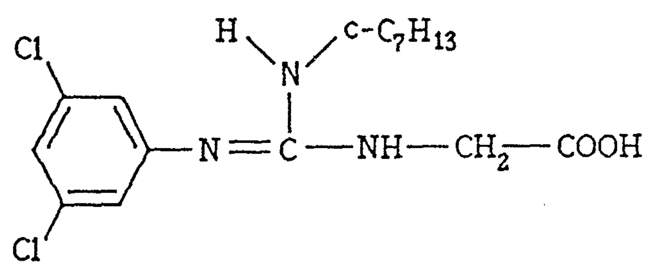

Ce composé est obtenu à partir de la glycine et de la N-cycloheptyl-N'-(3,5-dichlorophényl)-S-méthyl isothiourée, en suivant le protocole expérimental décrit dans l'exemple 2 (rendement 87 %, point de fusion 202 °C).

Le pouvoir édulcorant de ce composé correspond approximativement, sur une base pondérale, à 20 000 (vingt mille) fois celui du saccharose (par comparaison avec une solution de saccharose à 2 %).

EXEMPLE 45 :

Synthèse de l'acide N-[N-cycloheptylamino(4-cyano phénylimino)méthyl]-2-aminoéthanoïque :

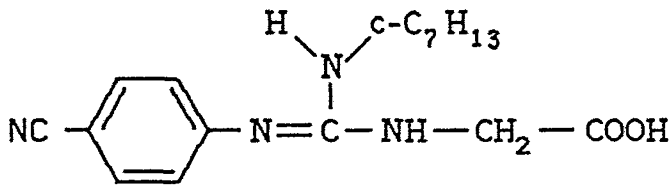

Ce composé est obtenu à partir de la glycine et de la N-cycloheptyl-N'-(4-cyanophényl)-S-méthylisothiourée, en suivant le protocole expérimental décrit dans l'exemple 2 (rendement 16 %, point de fusion 220 °C).

Le pouvoir édulcorant de ce composé correspond approximativement, sur une base pondérale, à 60 000 (soixante mille) fois celui du saccharose (par comparaison avec une solution de saccharose à 2 %).

EXEMPLE 46 :

Synthèse de l'acide N-[N-cyclooctylamino (3,5-dichlorophénylimino)méthyl]-2-aminoéthanoïque :

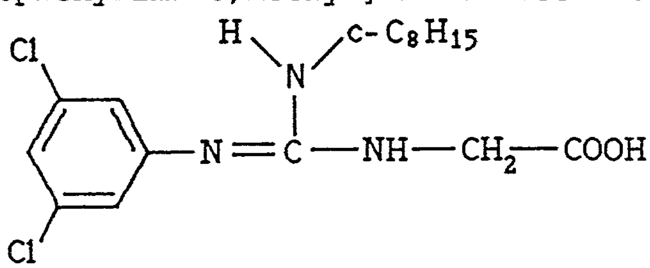

Ce composé est obtenu à partir de la glycine et de la N-cyclooctyl-N'-(3,5-dichlorophényl)-S-méthyl

isothiourée, en suivant le protocole expérimental décrit dans l'exemple 2 (rendement 61 %, point de fusion 199 °C).

Le pouvoir édulcorant de ce composé correspond approximativement, sur une base pondérale, à 60 000 (soixante mille) fois celui du saccharose (par comparaison avec une solution de saccharose à 2 %).

EXEMPLE 47 :

Synthèse de l'acide N-[N-cyclooctylamino-(4-cyanophénylimino)méthyl]-2-aminoéthanoïque :

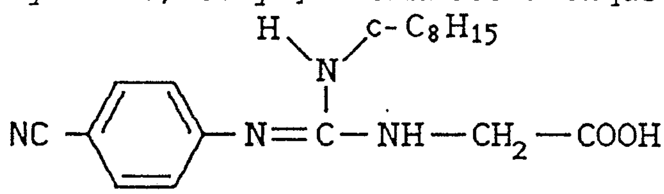

Ce composé est obtenu à partir de la glycine et de la N-cyclooctyl-N'-(4-cyanophényl)-S-méthylisothiourée, en suivant le protocole expérimental décrit dans l'exemple 2 (rendement 80 %, point de fusion 228 °C).

Les sels de sodium ou de potassium de ce composé peuvent être obtenus après dissolution de 1 g du produit dans 3 $cm^3$ d'une solution d'hydroxyde de sodium 1N ou d'hydroxyde de potassium 1N suivie d'une concentration à sec.

Le pouvoir édulcorant de ces composés correspond approximativement, sur une base pondérale, à 170 000 (cent soixante dix mille) fois celui du saccharose par comparaison avec une solution de saccharose à 2 %, à 130 000 (cent trente mille) fois par comparaison avec une solution de saccharose à 5 %, à 100 000 (cent mille) fois par comparaison avec une solution de saccharose à 10 %.

EXEMPLE 48 :

Synthèse de l'acide N-[N-cyclooctylamino(4-cyano-3-méthylphénylimino)méthyl]-2-aminoéthanoïque :

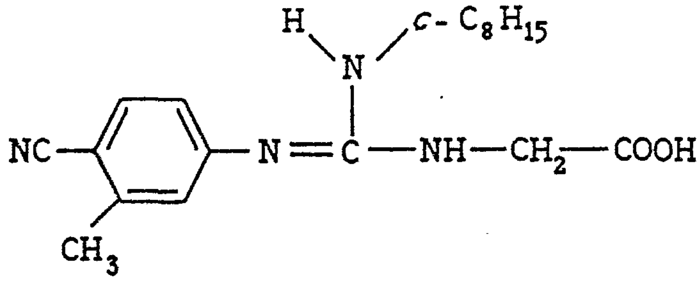

Ce composé est obtenu à partir de la glycine et de la N-cyclooctyl-N'-(4-cyano-3-méthylphényl)-S-méthylisothiourée en suivant le protocole expérimental décrit dans l'exemple 2 (rendement 30 %, point de fusion 155 °C).

Le pouvoir édulcorant de ce composé correspond approximativement, sur une base pondérale, à 80 000 (quatre vingt mille) fois celui du saccharose (par comparaison avec une solution de saccharose à 2 %).

EXEMPLE 49 :

Synthèse de l'acide N-[N-cyclooctylamino(3-chloro-4-cyanophénylimino)méthyl]-2-aminoéthanoïque :

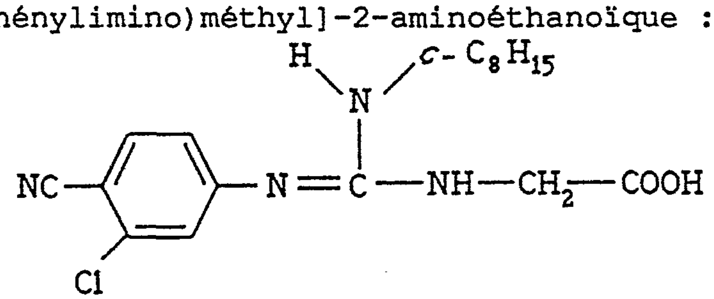

Ce composé est obtenu à partir de la glycine et de la N-cyclooctyl-N'-(3-chloro-4-cyanophényl)-S-méthylisothiourée en suivant le protocole expérimental décrit dans l'exemple 2 (rendement 68 %, point de fusion 156 °C).

Le pouvoir édulcorant de ce composé correspond approximativement, sur une base pondérale, à 100 000 (cent mille) fois celui du saccharose (par comparaison avec une solution de saccharose à 2 %).

EXEMPLE 50 :

Synthèse de l'acide N-[N-cyclononylamino(4-cyano phénylimino)méthyl]-2-aminoéthanoïque :

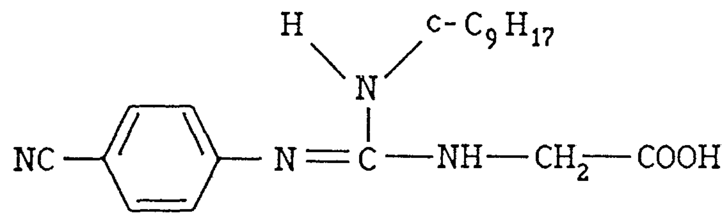

Ce composé est obtenu à partir de la glycine et de la N-cyclononyl-N'-(4-cyanophényl)-S-méthylisothiourée en suivant le protocole expérimental décrit dans l'exemple 2 (rendement 70 %, point de fusion 195 °C).

Le pouvoir édulcorant de ce composé correspond approximativement, sur une base pondérale, à 200 000 (deux cent mille) fois celui du saccharose (par comparaison avec une solution de saccharose à 2 %).

EXEMPLE 51 :

Synthèse de l'acide N-[N-benzylamino(3,5-dichloro phénylimino)méthyl]-2-aminoéthanoïque :

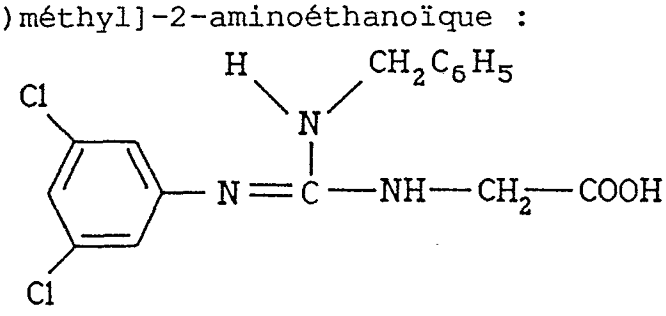

Ce composé est obtenu à partir de la glycine et de la N-benzyl-N'-(3,5-dichlorophényl)-S-méthylisothiourée, en suivant le protocole expérimental décrit dans l'exemple 2 (rendement 35 %, point de fusion 182 °C).

Le pouvoir édulcorant de ce composé correspond approximativement, sur une base pondérale, à 80 000 (quatre vingt mille) fois celui du saccharose (par comparaison avec une solution de saccharose à 2 %).

EXEMPLE 52 :

Synthèse de l'acide N-[N-benzylamino(4-cyano phénylimino)méthyl]-2-aminoéthanoïque :

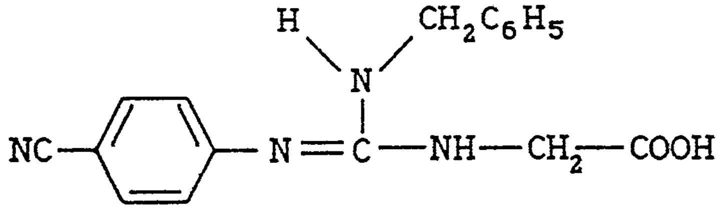

Ce composé est obtenu à partir de la glycine et de la N-benzyl-N'-(4-cyanophényl)-S-méthylisothiourée, en suivant le protocole expérimental décrit dans l'exemple 2 (rendement 31 %, point de fusion 149° C).

Le pouvoir édulcorant de ce composé correspond approximativement, sur une base pondérale, à 30 000 (trente mille) fois celui du saccharose par comparaison avec une solution de saccharose à 2 %, à 27 000 (vingt sept mille) fois par comparaison avec une solution de saccharose à 5 %, à 22 000 (vingt deux mille) fois par comparaison avec une solution de saccharose à 10 %.

EXEMPLE 53 :

Synthèse de l'acide N-[N-phényléthylamino(4-cyanophénylimino)méthyl]-2-aminoéthanoïque :

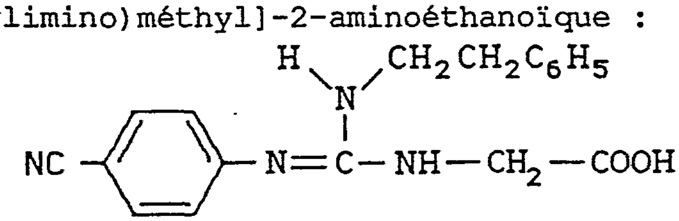

Ce composé est obtenu à partir de la glycine et de la N-phényléthyl-N'-(4-cyanophényl)-S-méthylisothiourée, en suivant le protocole expérimental décrit dans l'exemple 2 (rendement 52 %, point de fusion 129 °C).

Le pouvoir édulcorant de ce composé correspond approximativement, sur une base pondérale, à 8 500 (huit mille cinq cents) fois celui du saccharose par comparaison à une solution de saccharose à 2 %, à 6 100 (six mille cent) fois par comparaison à une solution de saccharose à 5 %, à 4 200 (quatre mille deux cents) fois par comparaison à une solution de saccharose à 10 %.

EXEMPLE 54 :

Synthèse de l'acide N-[N-(*R*)-α-méthylbenzylamino (4-cyanophénylimino)méthyl]-2-aminoéthanoïque :

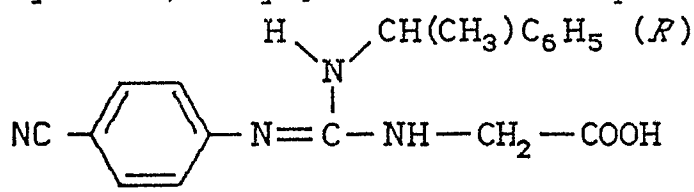

Ce composé est obtenu à partir de la glycine et de la N-(*R*)-α-méthylbenzyl-N'-(4-cyanophényl)-S-méthylisothiourée, en suivant le protocole expérimental décrit dans l'exemple 2 [rendement 71 %, $[\alpha]_D^{20}$ -112,5° (c = 1, HCl 0,5 N), point de fusion 189 °C].

Le pouvoir édulcorant de ce composé correspond approximativement, sur une base pondérale, à 9 000 (neuf mille) fois celui du saccharose par comparaison à une solution de saccharose à 2 %, à 6 600 (six mille six cents) fois par comparaison à une solution de saccharose à 5 %, à 2 500 (deux mille cinq cents) fois par comparaison à une solution de saccharose à 10 %.

EXEMPLE 55 :

Synthèse de l'acide N-[N-cyclohexylméthylamino (phénylimino)méthyl]-2-aminoéthanoïque :

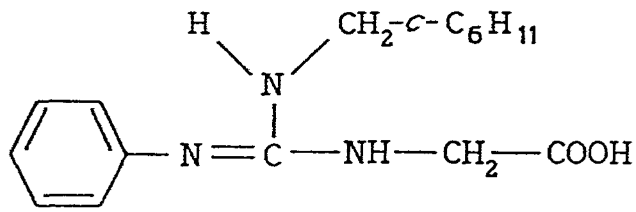

Ce composé est obtenu à partir de la glycine et de la N-cyclohexylméthyl-N'-phényl-S-méthylisothiourée, en suivant le protocole expérimental décrit dans l'exemple 2 (rendement 33 %, point de fusion 185 °C).

Le pouvoir édulcorant de ce composé correspond approximativement, sur une base pondérale, à 3 300 (trois mille trois cents) fois celui du saccharose par comparaison à une solution de saccharose à 2 %, à 2 500 (deux mille cinq cents) fois par comparaison à une solution de saccharose à 5 %, à 2 000 (deux mille) fois par comparaison à une solution de saccharose à 10 %.

<u>EXEMPLE 56</u> :

Synthèse de l'acide N-[N-cyclohexylméthylamino (3-cyanophénylimino)méthyl]-2-aminoéthanoïque :

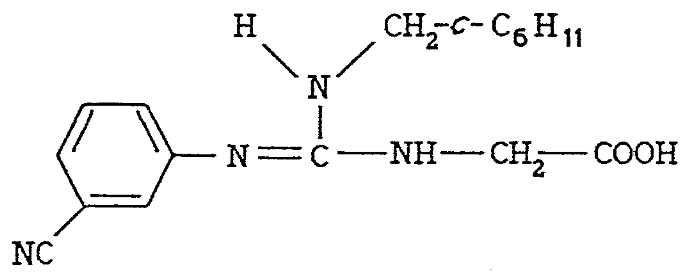

Etape 1 : Synthèse de la N-(3-cyanophényl)-N'-t-butyloxycarbonylméthyl thiourée :

On ajoute, à 0 °C, 2,8 $cm^3$ (20 mmol) de triéthylamine et 3,2 g (20 mmol) de 3-cyanophényl isothiocyanate, à une solution de 3,35 g (20 mmol) de glycine *t*-butyl ester dans 150 $cm^3$ de dichlorométhane. Le mélange est agité 12 heures à 20 °C avant d'être concentré sous vide puis repris par un mélange eau-éther éthylique (300-300). La phase éthérée est séparée, lavée par une solution 1N de chlorure d'hydrogène (200 $cm^3$), puis par une solution saturée de chlorure de sodium.

Après séchage sur sulfate de magnésium et concentration sous vide, on obtient 5,17 g (rendement 89 %) d'un solide jaune dont le point de fusion est de 131-132 °C.

Etape 2 : Synthèse de l'acide N-[N-cyclohexyl méthylamino(3-cyanophénylimino)méthyl]-2-aminoéthanoïque:

On ajoute 2,23 g (10,82 mmol) de dicyclohexyl carbodiimide, puis 1,4 $cm^3$ (1,1 équivalent) de cyclohexylméthylamine à une solution de 3 g (10,3 mmol) de la thiourée précédente dans 70 $cm^3$ d'acétate d'éthyle. Le mélange est chauffé, sous atmosphère d'azote, durant 72 heures au reflux, puis est concentré jusqu'à 20 $cm^3$ par évaporation. Le solide obtenu après refroidissement est éliminé par filtration, et le filtrat est concentré sous vide. L'huile obtenue est purifiée par chromatographie sur gel de silice (éluant méthanol-acétate d'éthyle : 5-95). On obtient 2,07 g (rendement 54 %) d'une huile légèrement jaune.

0,4 g (1,08 mmol) de l'ester *t*-butylique ainsi obtenu est dissous dans 1,6 $cm^3$ d'acide acétique pur puis traité par addition, goutte à goutte, de 1,6 $cm^3$ (10,81 mmol) d'une solution 6,82 M de chlorure d'hydrogène dans le dioxane. Après 2 heures à température ambiante, la solution est concentrée sous vide et l'huile obtenue est mise en contact, durant 24 heures, avec 100 $cm^3$ d'éther éthylique. On obtient après filtration, 0,31 g (rendement 82 %, point de fusion 80-83 °C) du produit désiré sous forme d'hydrochlorure.

Le pouvoir édulcorant de ce composé correspond approximativement, sur une base pondérale, à 5 000 (cinq mille) fois celui du saccharose (par comparaison avec une solution de saccharose à 2 %).

<u>EXEMPLE 57</u> :

Synthèse de l'acide N-[N-cyclohexylméthylamino (3,5-dichlorophénylimino)méthyl]-2-aminoéthanoïque :

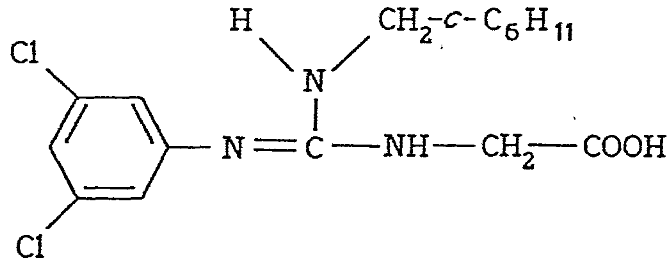

Ce composé est obtenu à partir de la glycine et de la N-cyclohexylméthyl-N'-(3,5-dichlorophényl)-S-méthylisothiourée, en suivant le protocole expérimental décrit dans l'exemple 2 (rendement 50 %, point de fusion 186 °C).

Le pouvoir édulcorant du composé correspond approximativement, sur une base pondérale, à 35 000 (trente cinq mille) fois celui du saccharose (par comparaison avec une solution de saccharose à 2 %)

EXEMPLE 58 :

Synthèse de l'acide N-[N-cyclohexylméthylamino(4-chlorophénylimino)méthyl]-2-aminoéthanoïque :

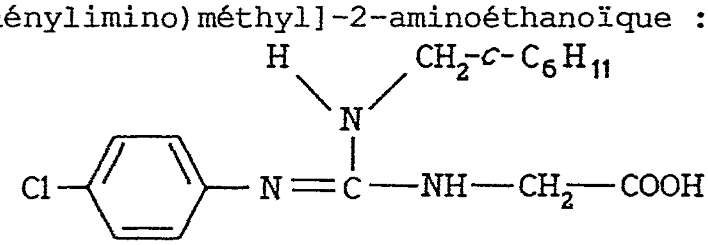

Ce composé est obtenu à partir de la glycine et de la N-cyclohexylméthyl-N'-(4-chlorophényl)-S-méthylisothiourée, en suivant le protocole expérimental décrit dans l'exemple 2 (rendement 33 %, point de fusion 183 °C).

Le pouvoir édulcorant de ce composé correspond approximativement, sur une base pondérale, à 3 500 (trois mille cinq cents) fois celui du saccharose par comparaison à une solution de saccharose à 2 %, à 2 700 (deux mille sept cents) fois par comparaison à une solution de saccharose à 5 %, à 1 500 (mille cinq cents) fois par comparaison à une solution de saccharose à 10 %.

EXEMPLE 59 :

Synthèse de l'acide N-[N-cyclohexylméthylamino(4-cyanophénylimino)méthyl]-2-aminoéthanoïque:

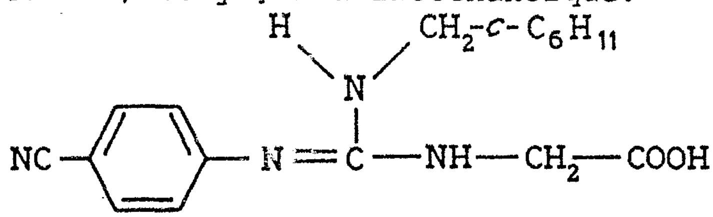

Ce composé est obtenu à partir de la glycine et de la N-cyclohexylméthyl-N'-(4-cyanophényl)-S-méthylisothiourée, en suivant le protocole expérimental décrit dans l'exemple 2 (rendement 48 %, point de fusion 180 °C).

Le pouvoir édulcorant de ce composé correspond approximativement, sur une base pondérale, à 35 000 (trente cinq mille) fois celui du saccharose par comparaison à une solution de saccharose à 2 %, à 28 000 (vingt huit mille) fois par comparaison à une solution de saccharose à 5 %, à 17 000 (dix sept mille) fois par comparaison à une solution de saccharose à 10 %.

EXEMPLE 60 :

Synthèse de l'acide N-[N-cyclohexylméthylamino(4-méthoxycarbonylphénylimino)méthyl]-2-aminoéthanoïque :

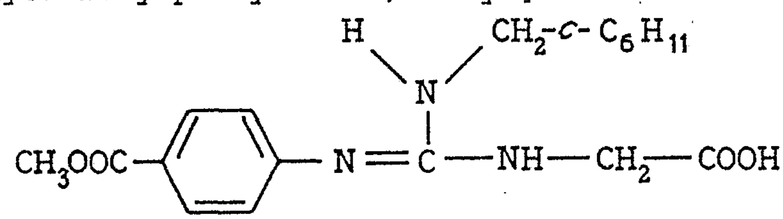

Ce composé est obtenu à partir de la glycine et de la N-cyclohexylméthyl-N'-(4-méthoxycarbonylphényl)-S-méthylisothiourée, en suivant le protocole expérimental décrit dans l'exemple 2 (rendement 57 %, point de fusion 182 °C).

Le pouvoir édulcorant de ce composé correspond approximativement, sur une base pondérale, à 1 300 (mille

trois cents) fois celui du saccharose par comparaison à une solution de saccharose à 2 %, à 900 (neuf cents) fois par comparaison à une solution de saccharose à 5 %, et à 500 (cinq cents) par comparaison à une solution de saccharose à 10 %.

EXEMPLE 61 :

Synthèse de l'acide N-[N-cyclohexylméthylamino(4-carboxyphénylimino)méthyl]-2-aminoéthanoïque :

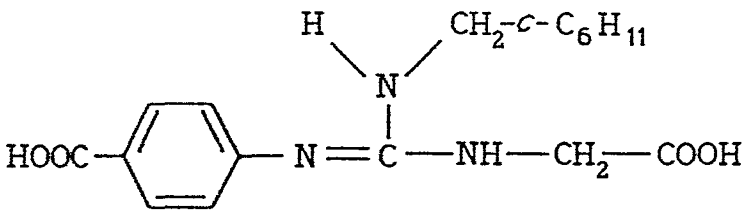

Ce composé est obtenu (rendement 79 %, point de fusion 164 °C) par saponification, par une solution d'hydroxyde de sodium 1 N, de l'acide N-[N-cyclohexyl-méthylamino(4-méthoxycarbonylphénylimino)méthyl]-2-amino-éthanoïque (point de fusion 163 °C) qui a été lui-même préparé à partir de la glycine et de la N-cyclohexylméthyl-N'-(4-méthoxycarbonylphényl)-S-méthylisothiourée en suivant le protocole décrit dans l'exemple 2.

Le pouvoir édulcorant du composé correspond approximativement, sur une base pondérale, à 50 (cinquante) fois celui du saccharose (par comparaison avec une solution de saccharose à 2 %).

EXEMPLE 62 :

Synthèse de l'acide N-[N-cyclohexylméthylamino(4-nitrophénylimino)méthyl]-2-aminoéthanoïque :

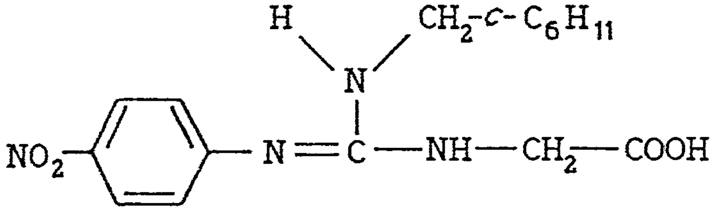

Ce composé est obtenu à partir de la glycine et de la N-cyclohexylméthyl-N'-(4-nitrophényl)-S-méthylisothiourée, en suivant le protocole expérimental décrit dans l'exemple 2 (rendement 39 %, point de fusion 203 °C).

Le pouvoir édulcorant de ce composé correspond approximativement, sur une base pondérale, à 8 000 (huit mille) fois celui du saccharose par comparaison à une solution de saccharose à 2 %, à 7 500 (sept mille cinq cents) fois par comparaison à une solution de saccharose à 5 %, à 4 000 (quatre mille) fois par comparaison à une solution de saccharose à 10 %.

EXEMPLE 63 :

Synthèse de l'acide N-[N-(*S*)-1-cyclohexyléthyl amino(3,5-dichlorophénylimino)méthyl]-2-aminoéthanoïque :

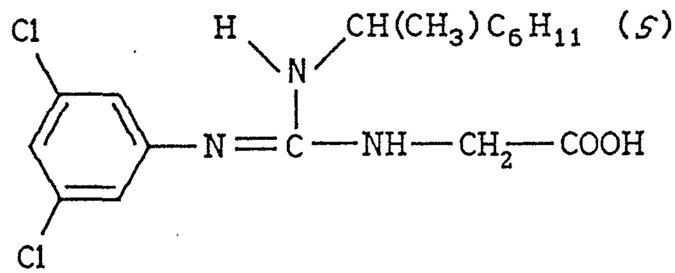

Ce composé est obtenu à partir de la glycine et de la N-(*S*)-1-cyclohexyléthyl-N'-(3,5-dichlorophényl)-S-méthylisothiourée, en suivant le protocole expérimental décrit dans l'exemple 2 (rendement 80 %, point de fusion 150 °C).

Le pouvoir édulcorant de ce composé correspond approximativement, sur une base pondérale, à 70 000 (soixante dix mille) fois celui du saccharose (par comparaison avec une solution de saccharose à 2 %).

EXEMPLE 64 :

Synthèse de l'acide N-[N-(2-méthylphényl)amino (4-cyanophénylimino)méthyl]-2-aminoéthanoïque :

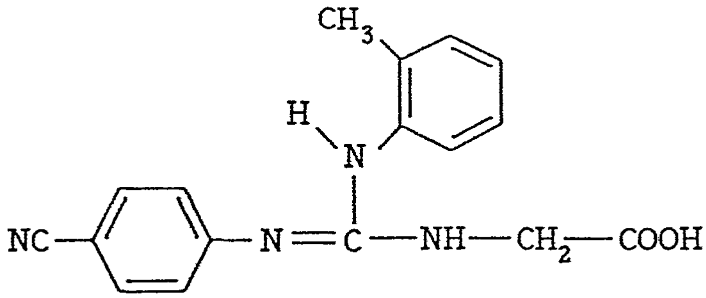

Ce composé est obtenu à partir de la glycine et de la N-2-(méthylphényl)-N'-(4-cyanophényl)-S-méthylisothiourée, en suivant le protocole expérimental décrit dans l'exemple 2 (rendement 69%, point de fusion 165 °C).

Le pouvoir édulcorant de ce composé correspond approximativement, sur une base pondérale, à 5 000 (cinq mille) fois celui du saccharose (par comparaison avec une solution de saccharose à 2 %).

EXEMPLE 65 :

Synthèse de l'acide N-[N-(3-méthylphényl)amino(4-cyanophénylimino)méthyl]-2-aminoéthanoïque :

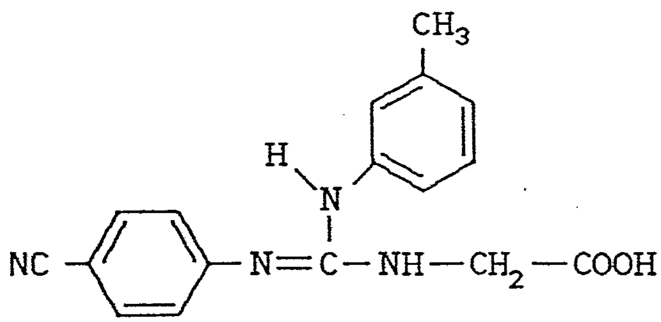

Ce composé est obtenu à partir de la glycine et de la N-(3-méthylphényl)-N'-(4-cyanophényl)-S-méthylisothiourée, en suivant le protocole expérimental décrit dans l'exemple 2 (rendement 69 %, point de fusion 220 °C).

Le pouvoir édulcorant de ce composé correspond approximativement, sur une base pondérale, à 9 000 (neuf mille) fois celui du saccharose (par comparaison avec une solution de saccharose à 2 %).

EXEMPLE 66 :

Synthèse de l'acide N-[N-(4-méthylphényl)amino(4-cyanophénylimino)méthyl]-2-aminoéthanoïque :

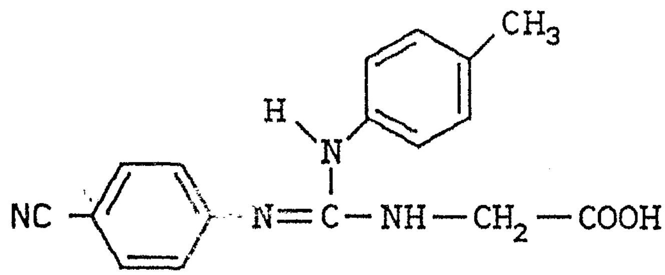

Ce composé est obtenu à partir de la glycine et de la N-(4-méthylphényl)-N'-(4-cyanophényl)-S-méthylisothiourée en suivant le protocole expérimental décrit dans l'exemple 2 (rendement 45 %, point de fusion 216 °C).

Le pouvoir édulcorant de ce composé correspond approximativement, sur une base pondérale, à 7 000 (sept mille) fois celui du saccharose (par comparaison avec une solution de saccharose à 2 %).

EXEMPLE 67 :

Synthèse de l'acide N-[N-(1-méthylcyclohexyl)-amino(4-cyanophénylimino)méthyl]-2-aminoéthanoïque :

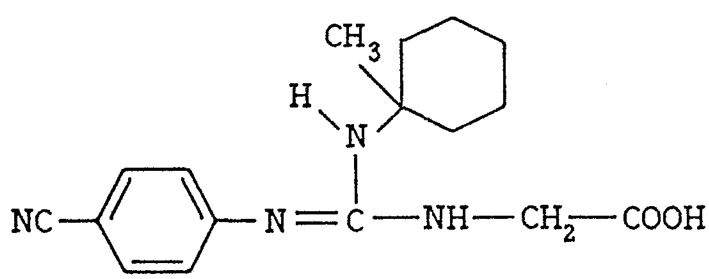

Etape 1 : Synthèse de la N-(1-méthylcyclohexyl)-N'-(4-cyanophényl) thiourée :

A une solution, refroidie à 0 °C, de 10,26 g (64,05 mmol) de 4-cyanophényl isothiocyanate dans 250 $cm^3$ d'acétate d'éthyle, sont ajoutés, 7,59 g (67,1 mmol) de 1-méthylcyclohexylamine. La solution est ensuite

agitée durant une nuit à température ambiante. Le précipité formé est filtré puis lavé par l'éther éthylique. Le filtrat est concentré à sec sous vide et le résidu est trituré dans l'éther éthylique (50 $cm^3$) pour conduire à l'obtention d'une seconde portion de thiourée. On obtient finalement 14,43 g (rendement 82,5 %) de la thiourée désirée.

Etape 2 : Synthèse de l'acide N-[N-(1-méthyl cyclohexyl)amino(4-cyanophénylimino)méthyl]-2-aminoéthanoïque sous forme d'hydrochlorure :

Une suspension de 1,39 g (5,08 mmol) de N-(1-méthylcyclohexyl)-N'-(4-cyanophényl) thiourée, de 1 g (7,63 mmol) de glycine *tert*-butyl ester, de 1,57 g (7,63 mmol) de dicyclohexylcarbodiimide et de 0,05 $cm^3$ (0,34 mmol) de triéthylamine dans 10 $cm^3$ d'acétate d'éthyle, est agitée durant 4 jours à température ambiante. La dicyclohexylthiourée formée est éliminée par filtration et le filtrat est concentré à sec. Le résidu obtenu est purifié par chromatographie (colonne ø 50 mm, éluant $CH_2Cl_2$- $CH_3OH$ 95-5/$CH_2Cl_2$ contenant 0,6 % de $NH_4OH$), puis recristallisé dans l'hexane. On obtient 1,43 g (rendement 76 %) de *tert*-butyl N-[N-(1-méthylcyclohexyl)amino(4-cyanophénylimino)méthyl-2-aminoéthanoate.

A 1,9 $cm^3$ d'une solution dans le dioxane de HCl 6,98 N (13 mmol) et de 3,8 $cm^3$ d'acide acétique, est ajouté 0,5 g (1,35 mmol) du composé précédemment obtenu. La solution est agitée durant 40 minutes et la réaction est arrêtée par addition de 100 $cm^3$ d'éther éthylique, ce qui provoque la formation d'une huile qui cristallise après plusieurs heures d'agitation. Le solide est séparé par filtration, puis est lavé par l'éther éthylique. Après séchage, on obtient 0,425 g (rendement 90 %) du produit désiré.

Etape 3 : Synthèse de l'acide N-[N-(1-méthyl cyclohexyl)amino(4-cyanophénylimino)méthyl]-2-aminoéthanoïque :

A une suspension de 0,508 g (1,45 mmol) de l'hydrochlorure précédemment obtenu dans un mélange eau-méthanol (25-5), est ajouté 0,475 g (11,9 mmol) d'hydroxyde de sodium. Le mélange est filtré et le méthanol est éliminé sous vide. La solution est neutralisée à pH 7 par addition d'une solution de chlorure d'hydrogène à 10 %, puis est filtrée après une nuit sous agitation. On obtient 0,31 g (rendement 68 %) du dérivé désiré.

Le pouvoir édulcorant de ce composé correspond approximativement, sur une base pondérale, à 100 (cent) fois celui du saccharose (par comparaison avec une solution de saccharose à 2 %).

EXEMPLE 68 :

Synthèse de l'acide N-[N-1-naphtylamino (3,5-dichlorophénylimino)méthyl]-2-aminoéthanoïque :

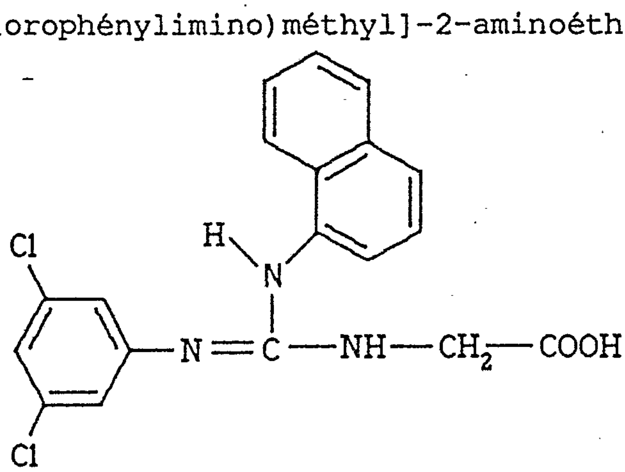

Ce composé est obtenu à partir de la glycine et de la N-1-naphtyl-N'-(3,5-dichlorophényl)-S-méthyl isothiourée, en suivant le protocole expérimental décrit

dans l'exemple 2 (rendement 78 %, point de fusion 210 °C).

Le pouvoir édulcorant de ce composé correspond approximativement, sur une base pondérale, à 30 000 (trente mille) fois celui du saccharose (par comparaison avec une solution de saccharose à 2 %).

EXEMPLE 69 :

Synthèse de l'acide N-[N-1-naphtylamino(4-cyano phénylimino)méthyl]-2-aminoéthanoïque :

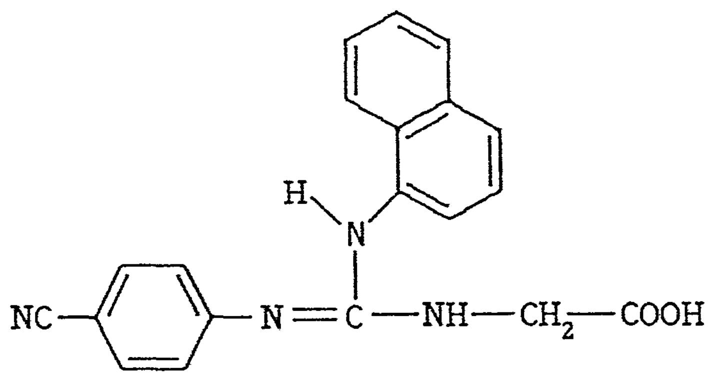

Ce composé est obtenu à partir de la glycine et de la N-1-naphtyl-N'-(4-cyanophényl)-S-méthylisothiourée, en suivant le protocole expérimental décrit dans l'exemple 2 (rendement 44 %, point de fusion 178 °C).

Le pouvoir édulcorant de ce composé correspond approximativement, sur une base pondérale, à 60 000 (soixante mille) fois celui du saccharose (par comparaison avec une solution de saccharose à 2 %).

EXEMPLE 70 :

Synthèse de l'acide N-[N-(1-indanyl)amino(4-cyano phénylimino)méthyl]-2-aminoéthanoïque :

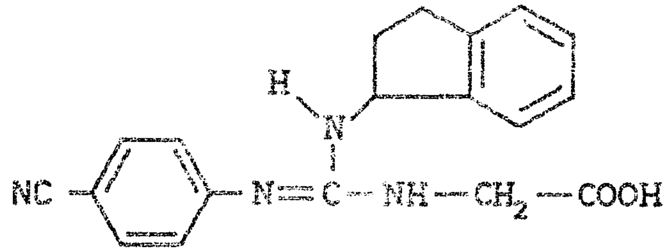

Ce composé est obtenu à partir de la glycine et de la N-(1-indanyl)-N'-(4-cyanophényl)-S-méthylisothiourée, en suivant le protocole expérimental décrit dans l'exemple 2 (rendement 62 %, point de fusion 176 °C).

Le pouvoir édulcorant de ce composé correspond approximativement, sur une base pondérale, à 5 000 (cinq mille) fois celui du saccharose (par comparaison avec une solution de saccharose à 2 %).

EXEMPLE 71 :

Synthèse de l'acide N-[N-(1-adamantyl)amino(4-cyanophénylimino)méthyl]-2-aminoéthanoïque :

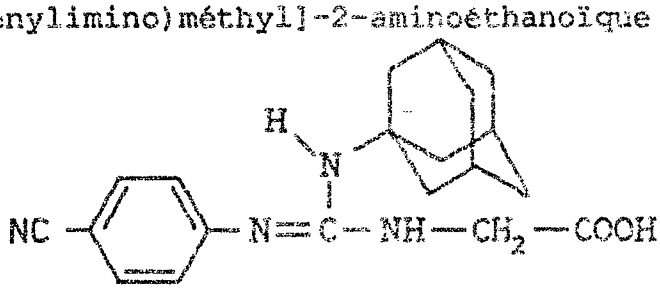

Ce composé est obtenu à partir de la glycine et de la N-(1-adamantyl)-N'-(4-cyanophényl)-S-méthylisothiourée, en suivant le protocole expérimental décrit dans l'exemple 2 (rendement 55 %, point de fusion 232 °C).

Le pouvoir édulcorant de ce composé correspond approximativement, sur une base pondérale, à 3 500 (trois mille cinq cents) fois celui du saccharose (par comparaison avec une solution de saccharose à 2 %).

EXEMPLE 72 :

Synthèse de l'acide N-[N-(1-adamantylméthyl) amino(4-cyanophénylimino)méthyl]-2-aminoéthanoïque :

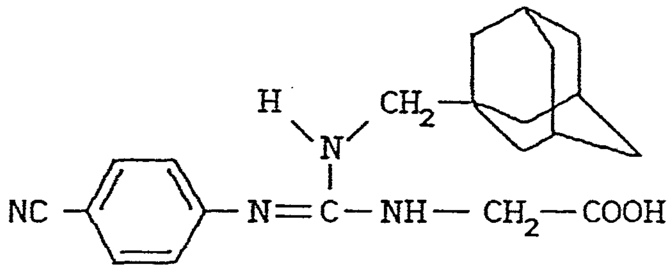

Ce composé est obtenu à partir de la glycine et de la N-(1-adamantylméthyl)-N'-(4-cyanophényl)-S-méthylisothiourée, en suivant le protocole expérimental décrit dans l'exemple 2 (rendement 62 %, point de fusion 169 °C).

Le pouvoir édulcorant de ce composé correspond approximativement, sur une base pondérale, à 23 000 (vingt trois mille) fois celui du saccharose (par comparaison avec une solution de saccharose à 2 %).

EXEMPLE 73 :

Synthèse de l'acide N-[cyanoimino(3-chlorophényl amino)méthyl]-2-aminoéthanoïque :

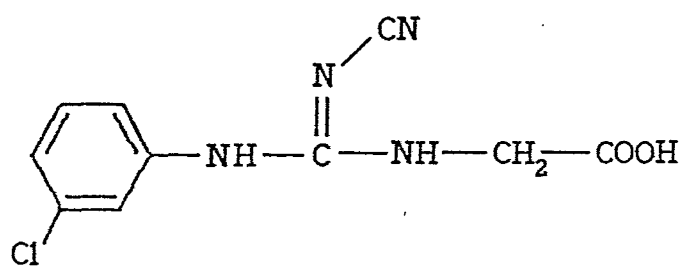

10 g (59 mmol) de 3-chlorophényl isothiocyanate et de 4,15 g (65 mmol) de monosodium cyanamide sont mélangés dans 100 $cm^3$ d'éthanol absolu et sont maintenus durant 2 heures à l'ébullition. Après élimination du solvant, le résidu solide obtenu est lavé par 100 $cm^3$

d'éther éthylique. Ce composé est alors mis en suspension dans une solution d'iodure de méthyle ($4,8\ cm^3$, soit 77 mmol) dans $50\ cm^3$ d'éthanol. Le mélange est laissé 20 heures à température ambiante. Le précipité final est filtré, lavé par $2 \times 50\ cm^3$ d'eau et par $2 \times 50\ cm^3$ d'éthanol, puis est séché sous vide. 10,1 g (rendement 83 %) de N-(3-chlorophényl)-N'-cyano-S-méthylisothiourée (point de fusion 167 °C) sont obtenus :

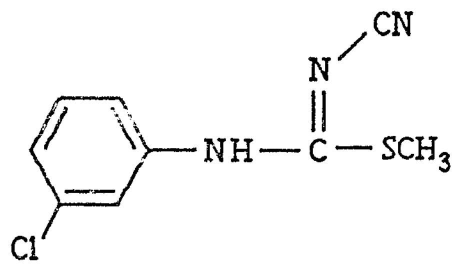

3 g (39,9 mmol) de glycine et 1,5 g (37,2 mmol) d'hydroxyde de sodium sont mélangés dans $10\ cm^3$ d'eau et sont ajoutés à 6 g (26,5 mmol) de N-(3-chlorophényl)-N'-cyano-S-méthylisothiourée en solution dans $40\ cm^3$ d'éthanol à 95 %. Le mélange est chauffé au reflux durant 1 heure. Aprés refroidissement, le précipité formé est filtré, puis lavé par $2 \times 50\ cm^3$ d'éther éthylique. Le solide obtenu est dissous dans $50\ cm^3$ d'une solution d'hydroxyde de sodium 1N. La solution résultante est lavée par du dichlorométhane ($3 \times 10\ cm^3$), puis acidifiée par une solution de HCl 3N jusqu'à l'obtention d'un pH voisin de 2. Le solide blanc formé est filtré, puis lavé par l'eau ($2 \times 10\ cm^3$) et séché sous vide. 6 g (rendement 89 %) d'acide N-[cyanoimino(3-chlorophénylamino)méthyl]-2-amino éthanoïque (point de fusion 109 °C) sont obtenus.

Le pouvoir édulcorant de ce composé correspond approximativement, sur une base pondérale, à 80 (quatre vingt) fois celui du saccharose (par comparaison avec une solution de saccharose à 2 %).

EXEMPLE 74 :

Synthèse de l'acide N-[N-(N-méthyl-N-phénylamino)(4-cyanophénylimino)méthyl]-2-aminoéthanoïque :

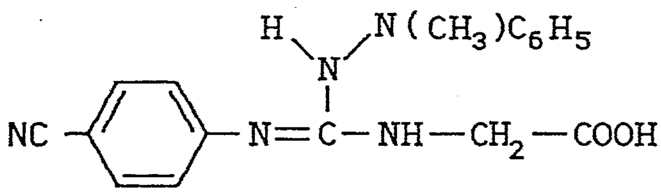

Ce composé est obtenu à partir de la glycine et de la N-(N-méthyl-N-phénylamino)-N'-(4-cyanophényl)-S-méthylisothiourée, en suivant le protocole expérimental décrit dans l'exemple 2 (rendement 13 %, point de fusion 202 °C).

Le pouvoir édulcorant de ce composé correspond approximativement, sur une base pondérale, à 18 000 (dix huit mille) fois celui du saccharose (par comparaison avec une solution de saccharose à 2 %).

EXEMPLE 75 :

Synthèse de l'acide N-[N-(3,4-méthylènedioxyphényl)amino(4-cyanophénylimino)méthyl]-2-aminoéthanoïque :

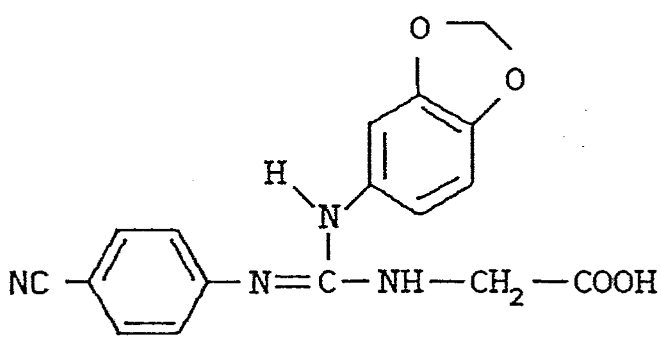

Ce composé est obtenu à partir de la glycine et de la N-(3,4-méthylènedioxyphényl)-N'-(4-cyanophényl)-S-méthylisothiourée, en suivant le protocole expérimental

décrit dans l'exemple 2 (rendement 10 %, point de fusion 166 °C).

Le pouvoir édulcorant de ce composé correspond approximativement, sur une base pondérale, à 5 000 (cinq mille) fois celui du saccharose (par comparaison avec une solution à 2 %).

EXEMPLE 76 :

Synthèse de l'acide N-[N-morpholinoamino(3,5-dichlorophénylimino)méthyl]-2-aminoéthanoïque :

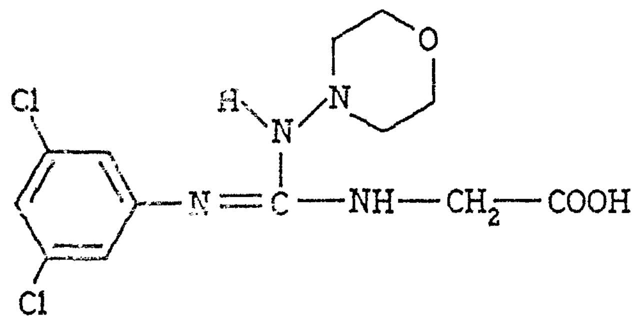

Ce composé est obtenu à partir de la glycine et de la N-morpholino-N'-(3,5-dichlorophényl)-S-méthylisothiourée, en suivant le protocole expérimental décrit dans l'exemple 2 (rendement 20 %, point de fusion 215 °C).

Le pouvoir édulcorant de ce composé correspond approximativement, sur une base pondérale, à 500 (cinq cents) fois celui du saccharose (par comparaison avec une solution de saccharose à 2 %).

EXEMPLE 77 :

Synthèse de l'acide N-[N-morpholinoamino(4-cyano-phénylimino)méthyl]-2-aminoéthanoïque :

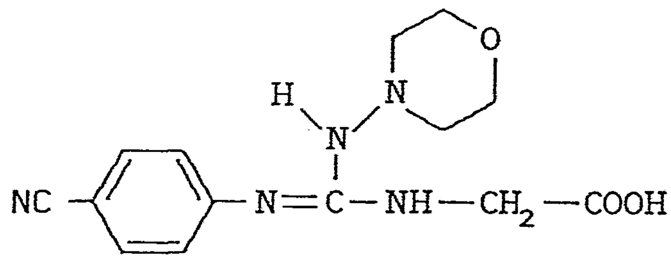

Ce composé est obtenu à partir de la glycine et de la N-morpholino-N'-(4-cyanophényl)-S-méthylisothiourée, en suivant le protocole expérimental décrit dans l'exemple 2 (rendement 11 %, point de fusion 205 °C).

Le pouvoir édulcorant de ce composé correspond approximativement, sur une base pondérale , à 950 (neuf cent cinquante) fois celui du saccharose (par comparaison avec une solution de saccharose à 2 %).

EXEMPLE 78 :

Synthèse de l'acide N-[N-(2,2,4,4-tétraméthylthia cyclobut-3-yl)amino(4-cyanophénylimino)méthyl]-2-amino-éthanoïque :

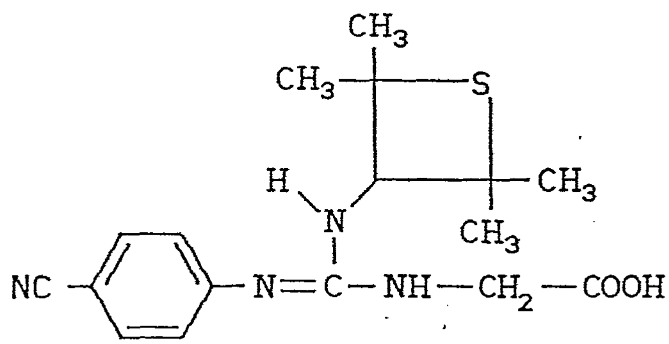

Ce composé est obtenu à partir de la glycine et de la N-(2,2,4,4-tétraméthylthiacyclobut-3-yl)-N'-(4-cyanophényl)-S-méthylisothiourée, en suivant le protocole expérimental décrit dans l'exemple 2 (rendement 50 %, point de fusion 237 °C).

Le pouvoir édulcorant de ce composé correspond approximativement, sur une base pondérale, à 700 (sept cents) fois celui du saccharose (par comparaison avec une solution de saccharose à 2 %).

EXEMPLE 79 :

Synthèse de l'acide N-[phénylsulfonylimino (3-chlorophénylamino)méthyl]-2-aminoéthanoïque :

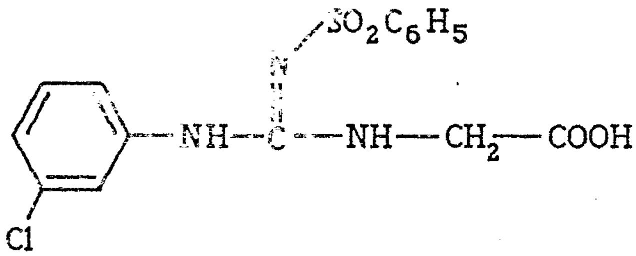

6 g (35,4 mmol) de 3-chlorophényl isothiocyanate et 6,1 g (38,9 mmol) de benzènesulfonamide sont dissous dans 50 $cm^3$ d'acétone. 1,55 g (38,9 mmol) d'hydroxyde de sodium est dissous dans 3 $cm^3$ d'eau. Les deux solutions sont mélangées. Le mélange est maintenu à température ambiante durant 2 heures. Le précipité formé est filtré, lavé par l'acétone et l'éther éthylique (2 x 20 $cm^3$). 11,3 g d'un résidu sont obtenus (rendement 100 %). Le résidu est dissous dans 50 $cm^3$ d'éthanol à 95 % contenant 2,88 $cm^3$ d'iodure de méthyle (46 mmol). Après 24 heures à température ambiante, la solution est évaporée à sec. Le résidu est lavé par l'éther éthylique (3 x 20 $cm^3$) puis séché sous vide. 8,75 g (rendement 80 %) de N-(3-chlorophényl)-N'-phénylsulfonyl-S-méthylisothiourée (point de fusion 116 °C) sont obtenus :

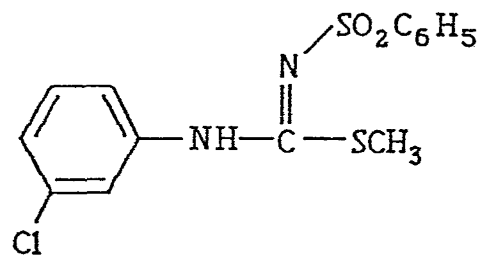

2,3 g (30,7 mmol) de glycine, 1,15 g (28,6 mmol) d'hydroxyde de sodium dans 10 $cm^3$ d'eau et 6,4 g (20,5 mmol) de N-(3-chlorophényl)-N'-phénylsulfonyl-S-méthyl isothiourée sont mélangés dans 50 $cm^3$ d'éthanol à 95 %. Le mélange est chauffé au reflux pendant 9 heures. Après concentration sous vide, le résidu obtenu est dissous dans 50 $cm^3$ d'une solution 1N d'hydroxyde de sodium. La solution est lavée par le dichlorométhane (3 x 20 $cm^3$) et l'acétate d'éthyle (2 x 20 $cm^3$), puis acidifiée par addition d'une solution 6N de chlorure d'hydrogène jusqu'à obtention d'un pH voisin de 3.

Après refroidissement, le précipité obtenu est filtré, lavé par l'eau (2 x 5 $cm^3$) et séché sous vide. 5,6 g (rendement 75 %) d'acide N-[phénylsulfonylimino (3-chlorophénylamino)méthyl]-2-aminoéthanoïque (point de fusion 218 °C) sont obtenus.

Le pouvoir édulcorant de ce composé correspond approximativement, sur une base pondérale, à 17 000 (dix sept mille) fois celui du saccharose (par comparaison avec une solution de saccharose à 2 %).

EXEMPLE 80 :

Synthèse de l'acide N-[N-(3-chlorophényl)amino(4-cyanophénylimino)méthyl]-2-aminoéthanoïque :

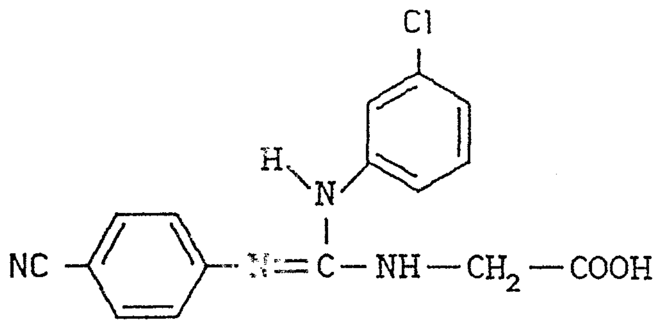

Ce composé est obtenu à partir de la glycine et la N-(3-chlorophényl)-N'-(4-cyanophényl)-S-méthylisothiourée, en suivant le protocole expérimental décrit dans l'exemple 2 (rendement 89 %, point de fusion 198 °C).

Le pouvoir édulcorant de ce composé correspond approximativement, sur une bas pondérale, à 10 000 (dix mille) fois celui du saccharose (par comparaison avec une solution de saccharose à 2 %).

EXEMPLE 81 :

Synthèse de l'acide N-[N-3,5-dichlorophénylamino(3,5-dichlorophénylimino)méthyl]-2-aminoéthanoïque :

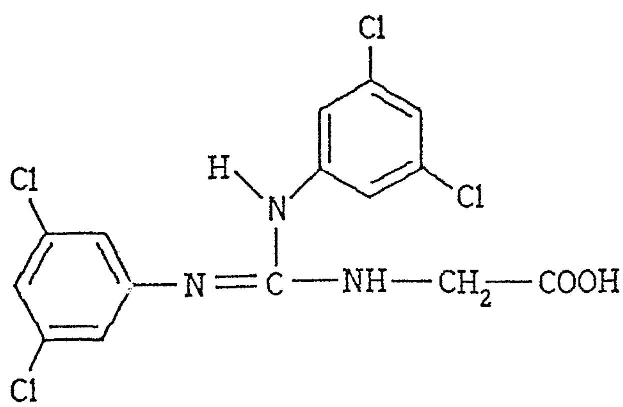

Ce composé est obtenu à partir de la glycine et de la N,N'-bis(3,5-dichlorophényl)-S-méthylisothiourée,

en suivant le protocole expérimental décrit dans l'exemple 2 (rendement 35 %, point de fusion 177 °C).

Le pouvoir édulcorant de ce composé correspond approximativement, sur une base pondérale, à 1 000 (mille) fois celui du saccharose (par comparaison avec une solution de saccharose à 2 %).

EXEMPLE 82 :

Synthèse de l'acide N-[N,N-(tétraméthylène)amino (4-cyanophénylimino)méthyl]-2-aminoéthanoïque :

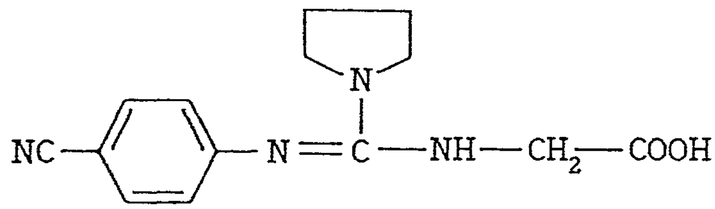

Etape 1 : Synthèse de la N-(4-cyanophényl)-N'-*t*-butyloxycarbonylméthyl thiourée :

On ajoute 10,7 g (82 mmol) de glycine *t*-butyl ester à une solution de 13 g (82 mmol) de 4-cyanophényl isothiocyanate dissous dans 100 $cm^3$ d'acétonitrile. Après 12 heures d'agitation, le précipité formé est filtré puis lavé par l'éther éthylique pour conduire à 18,7 g (rendement 78 %) de la thiourée désirée.

Etape 2 : Synthèse de la N-4-cyanophényl-N'-*t*-butyloxycarbonylméthyl-S-méthylisothiourée :

On agite 12 heures à 20 °C un mélange de 2,6 g (8,9 mmol) de la thiourée précédente et de 1,6 g (11,4 mmol) d'iodure de méthyle dans 50 $cm^3$ d'acétone. Le mélange est concentré à sec, et le résidu est filtré après trituration dans l'éther éthylique. L'hydroiodure obtenu est dissous dans une solution 1 N d'hydroxyde de sodium, et le mélange est extrait par le dichlorométhane.

Après séchage sur du sulfate de sodium anhydre et concentration à sec, on obtient 2,3 g (rendement 82 %) du composé désiré.

Etape 3 : Synthèse de l'acide N-[N,N-(tétraméthylène)amino(4-cyanophénylimino)méthyl]-2-aminoéthanoïque sous forme d'hydrochlorure :

On chauffe à 70 °C durant 24 heures un mélange de 6,5 g (21 mmol) du composé précédent et de 2,62 $cm^3$ (31 mmol) de pyrrolidine dans 100 $cm^3$ d'éthanol absolu. On ajoute à nouveau 1,3 $cm^3$ (15 mmol) de pyrrolidine et on maintient le chauffage durant 4 heures. Le mélange réactionnel est concentré à sec sous vide et le résidu est purifié par chromatographie sur colonne de gel de silice (éluant méthanol-dichlorométhane-hydroxyde d'ammonium : 7 - 92,5 - 0,5). On obtient 2,8 g (rendement 40 %) de *t*-butyl N-[N,N-(tétraméthylène)amino(4-cyanophénylimino)méthyl]-2-aminoéthanoate.

On agite durant 1 heure une solution de 1 g (3,13 mmol) de l'ester précédent dans 11 $cm^3$ d'acide acétique et 4,5 $cm^3$ d'une solution dans le dioxane de HCl 6,98 N.

Le mélange réactionnel est versé dans 1 L d'éther éthylique, le précipité obtenu est filtré, dissous dans l'eau et la solution obtenue est lyophilisée. On obtient 0,66 g (rendement 66 %) du composé désiré sous forme d'hydrochlorure.

Le pouvoir édulcorant de ce composé correspond approximativement, sur une base pondérale, à 100 (cent) fois celui du saccharose (par comparaison avec une solution de saccharose à 2 %).

EXEMPLE 83 :

Synthèse de l'acide N-[N,N-(pentaméthylène)amino(4-cyanophénylimino)méthyl]-2-aminoéthanoïque :

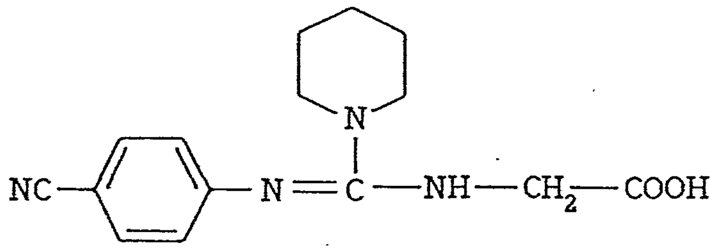

On chauffe à 50 °C durant 24 heures un mélange de 2,91 g (10 mmol) de N-(4-cyanophényl)-N'-*t*-butyloxy carbonylméthylthiourée, 2,3 g (11 mmol) de dicyclohexyl carbodiimide dissoute dans 50 $cm^3$ d'acétate d'éthyle et 2 $cm^3$ (20 mmol) de pipéridine. Après refroidissement, la dicyclohexylthiourée est éliminée et le filtrat est concentré à sec sous vide, ce qui permet d'obtenir 4 g d'une huile de couleur rouge. Après purification sur colonne de gel de silice (méthanol-dichlorométhane : 7,5-92,5), on obtient 1 g (rendement 33 %) de *t*-butyl N-[N,N-(pentaméthylène)amino(4-cyanophénylimino)méthyl]-2-aminoéthanoate.

Après élimination du groupe protecteur *t*-butylique suivant le protocole précédemment décrit, on obtient 0,71 g (rendement 95 %) du composé désiré sous forme d'hydrochlorure.

Le pouvoir édulcorant de ce composé correspond approximativement, sur une base pondérale, à 200 (deux cents) fois celui du saccharose (par comparaison avec une solution de saccharose à 2 %).

<u>EXEMPLE 84</u> :

Synthèse de l'acide N-[benzylimino(N-méthyl-N-phénylamino)méthyl]-2-aminoéthanoïque :

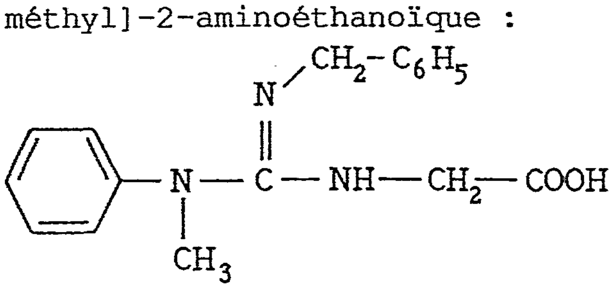

Ce composé est obtenu à partir de la N-benzyl-N'-méthyl-N'-phényl-S-méthylisothiourée, en suivant le protocole expérimental décrit dans l'exemple 2 (rendement 3 %, point de fusion 138 °C).

Le pouvoir édulcorant de ce composé correspond approximativement, sur une base pondérale, à 200 (deux cents) fois celui du saccharose (par comparaison avec une solution de saccharose à 2 %).

EXEMPLE 85 :

Synthèse de l'acide (*R*)-N-[N-cyclohexylamino(4-cyanophénylimino)méthyl]-2-aminopropanoïque :

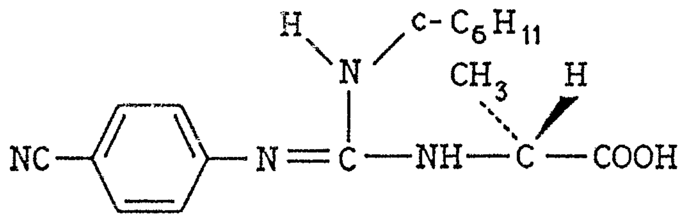

Ce composé est obtenu à partir de la D-alanine et de la N-cyclohexyl-N'-(4-cyanophényl)-S-méthylisothiourée, en suivant le protocole expérimental décrit dans l'exemple 2 (rendement 15 %, point de fusion 177 °C).

Le pouvoir édulcorant de ce composé correspond approximativement, sur une base pondérale, à 100 (cent) fois celui du saccharose (par comparaison avec une solution de saccharose à 2 %).

EXEMPLE 86 :

Synthèse de l'acide (*R*)-N-[N-(*S*)-α-méthylbenzyl amino(3,5-dichlorophénylimino)méthyl]-2-aminopropanoïque:

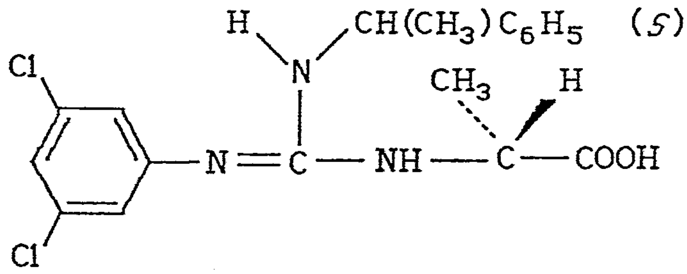

Ce composé est obtenu à partir de la D-alanine et de la N-(*S*)-α-méthylbenzyl-N'-(3,5-dichlorophényl)-S-méthylisothiourée, en suivant le protocole expérimental décrit dans l'exemple 2 (rendement 77 %, point de fusion 177 °C).

Le pouvoir édulcorant de ce composé correspond approximativement, sur une base pondérale, à 120 (cent vingt) fois celui du saccharose (par comparaison avec une solution de saccharose à 2 %).

<u>EXEMPLE 87</u> :

Synthèse de l'acide (*R*)-N-[N-cyclooctylamino(4-cyanophénylimino)méthyl]-2-aminopropanoïque :

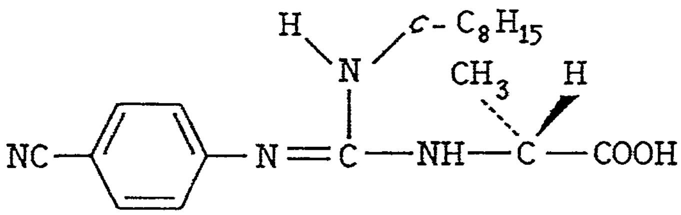

Ce composé est obtenu à partir de la D-alanine et de la N-cyclooctyl-N'-(4-cyanophényl)-S-méthylisothiourée en suivant le protocole expérimental décrit dans l'exemple 2 (rendement 70 %, point de fusion 144 °C).

Le pouvoir édulcorant de ce composé correspond approximativement, sur une base pondérale, à 900 (neuf cents) fois celui du saccharose (par comparaison avec une solution de saccharose à 2 %).

EXEMPLE 88 :

Synthèse de l'acide N-[N-méthyl-N-benzylamino (3,5-diméthylphénylimino)méthyl]-2-aminoéthanoïque :

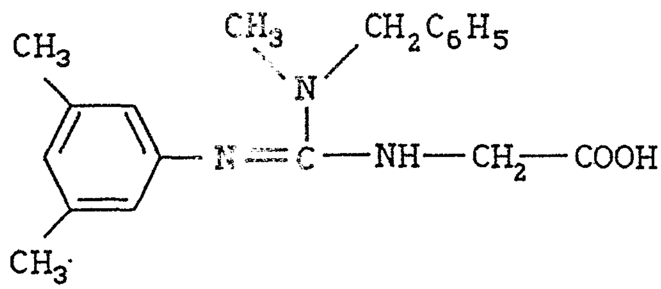

Ce composé est obtenu à partir de la glycine et de la N-méthyl-N-benzyl-N'-(3,5-diméthylphényl)-S-méthylisothiourée en suivant le protocole expérimental décrit dans l'exemple 2 (rendement 22 %, point de fusion 141 °C).

Le pouvoir édulcorant de ce composé correspond approximativement, sur une base pondérale, à 5 000 (cinq mille) fois celui du saccharose (par comparaison avec une solution de saccharose à 2 %).

Les pouvoirs édulcorants obtenus avec les différents composés cités dans les Exemples 1 à 88 sont récapitulés dans le tableau II ci-après ; les pouvoirs édulcorants ainsi donnés ont été évalués, sur une base pondérale, par rapport à une solution de saccharose à 2 %.

TABLEAU II

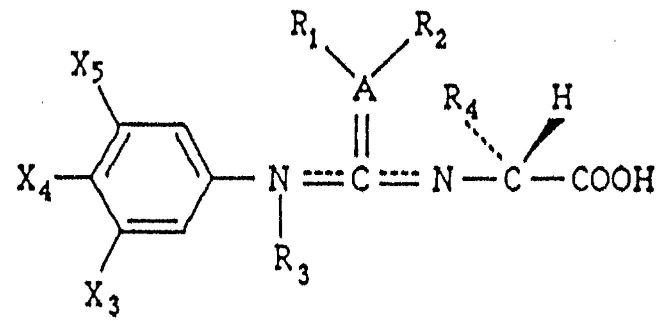

| Composé | $X_3$ | $X_4$ | $X_5$ | A | $R_1$ | $R_2$ | $R_3$ | $R_4$ | Pouvoir édulcorant |
|---|---|---|---|---|---|---|---|---|---|
| 1 | H | H | H | N | H | (S)$CH(CH_3)C_6H_5$ | | H | 5 000 |
| 2 | Br | H | H | N | H | (S)$CH(CH_3)C_6H_5$ | | H | 25 000 |
| 3 | $CF_3$ | H | H | N | H | (S)$CH(CH_3)C_6H_5$ | | H | 7 500 |
| 4 | $CH_3$ | H | H | N | H | (S)$CH(CH_3)C_6H_5$ | | H | 12 000 |
| 5 | $C_2H_5$ | H | H | N | H | (S)$CH(CH_3)C_6H_5$ | | H | 4 500 |
| 6 | Cl | H | H | N | H | (S)$CH(CH_3)C_6H_5$ | | H | 30 000 |
| 7 | CN | H | H | N | H | (S)$CH(CH_3)C_6H_5$ | | H | 5 500 |
| 8 | $NO_2$ | H | H | N | H | (S)$CH(CH_3)C_6H_5$ | | H | 6 000 |
| 9 | $OCH_3$ | H | H | N | H | (S)$CH(CH_3)C_6H_5$ | | H | 4 500 |
| 10 | $SCH_3$ | H | H | N | H | (S)$CH(CH_3)C_6H_5$ | | H | 5 500 |
| 11 | H | $CF_3$ | H | N | H | (S)$CH(CH_3)C_6H_5$ | | H | 500 |
| 12 | H | $CH_3$ | H | N | H | (S)$CH(CH_3)C_6H_5$ | | H | 800 |

| Composé | $X_3$ | $X_4$ | $X_5$ | A | $R_1$ | $R_2$ | $R_3$ | $R_4$ | Pouvoir édulcorant |
|---|---|---|---|---|---|---|---|---|---|
| 13 | H | Cl | H | N | H | (S)$CH(CH_3)C_6H_5$ | | H | 3 000 |
| 14 | H | CN | H | N | H | (S)$CH(CH_3)C_6H_5$ | | H | 28 000 |
| 15 | H | $COCH_3$ | H | N | H | (S)$CH(CH_3)C_6H_5$ | | H | 800 |
| 16 | H | $COOCH_3$ | H | N | H | (S)$CH(CH_3)C_6H_5$ | | H | 700 |
| 17 | H | F | H | N | H | (S)$CH(CH_3)C_6H_5$ | | H | 4 000 |
| 18 | H | $NO_2$ | H | N | H | (S)$CH(CH_3)C_6H_5$ | | H | 7 000 |
| 19 | H | $OCH_3$ | H | N | H | (S)$CH(CH_3)C_6H_5$ | | H | 2 100 |
| 20 | H | OH | H | N | H | (S)$CH(CH_3)C_6H_5$ | | H | 400 |
| 21 | H | $SCH_3$ | H | N | H | (S)$CH(CH_3)C_6H_5$ | | H | 1 200 |
| 22 | $CH_3$ | CN | H | N | H | (S)$CH(CH_3)C_6H_5$ | | H | 50 000 |
| 23 | Cl | Cl | H | N | H | (S)$CH(CH_3)C_6H_5$ | | H | 17 000 |
| 24 | $CF_3$ | H | $OCH_3$ | N | H | (S)$CH(CH_3)C_6H_5$ | | H | 7 000 |
| 25 | $CH_3$ | H | $CH_3$ | N | H | (S)$CH(CH_3)C_6H_5$ | | H | 30 000 |
| 26 | Cl | H | Cl | N | H | (S)$CH(CH_3)C_6H_5$ | | H | 120 000 |
| 27 | F | H | F | N | H | (S)$CH(CH_3)C_6H_5$ | | H | 15 000 |
| 28 | Cl | Cl | Cl | N | H | (S)$CH(CH_3)C_6H_5$ | | H | 35 000 |
| 29 | Cl | H | Cl | C | H | $NO_2$ | H | H | 400 |

| Composé | $X_3$ | $X_4$ | $X_5$ | A' | $R_1$ | $R_2$ | $R_3$ | $R_4$ | Pouvoir édulcorant |
|---|---|---|---|---|---|---|---|---|---|
| 30 | Cl | H | Cl | C | CN | CN | H | H | 30 |
| 31 | Cl | H | Cl | N | $CH_3$ | $CH_2C_6H_5$ | | H | 20 000 |
| 32 | H | CN | H | N | $CH_3$ | $CH(CH_3)_2$ | | H | 80 |
| 33 | H | CN | H | N | $CH_3$ | $CH_2C_6H_5$ | | H | 3 000 |
| 34 | H | CN | H | N | $C_2H_5$ | $CH_2C_6H_5$ | | H | 3 000 |
| 35 | H | CN | H | N | H | $CH_2CH_3$ | | H | 350 |
| 36 | H | CN | H | N | H | $(CH_2)_5CH_3$ | | H | 6 000 |
| 37 | H | CN | H | N | H | $(CH_2)_2CH(CH_3)_2$ | | H | 200 |
| 38 | H | CN | H | N | H | $C(CH_3)_2CH_2CH_3$ | | H | 300 |
| 39 | H | CN | H | N | H | $C(CH_3)_2CH_2C(CH_3)_3$ | | H | 1 200 |
| 40 | H | H | H | N | H | $C_6H_5$ | | H | 500 |
| 41 | H | CN | H | N | H | $C_6H_5$ | | H | 4 000 |
| 42 | H | H | H | N | H | $c\text{-}C_6H_{11}$ | | H | 100 |
| 43 | H | CN | H | N | H | $c\text{-}C_6H_{11}$ | | H | 12 000 |
| 44 | Cl | H | Cl | N | H | $c\text{-}C_7H_{13}$ | | H | 20 000 |
| 45 | H | CN | H | N | H | $c\text{-}C_7H_{13}$ | | H | 60 000 |
| 46 | Cl | H | Cl | N | H | $c\text{-}C_8H_{15}$ | | H | 60 000 |

| Composé | $X_3$ | $X_4$ | $X_5$ | A | $R_1$ | $R_2$ | $R_3$ | $R_4$ | Pouvoir édulcorant |
|---|---|---|---|---|---|---|---|---|---|
| 47 | H | CN | H | N | H | $c-C_8H_{15}$ | | H | 170 000 |
| 48 | $CH_3$ | CN | H | N | H | $c-C_8H_{15}$ | | H | 80 000 |
| 49 | Cl | CN | H | N | H | $c-C_8H_{15}$ | | H | 100 000 |
| 50 | H | CN | H | N | H | $c-C_9H_{17}$ | | H | 200 000 |
| 51 | Cl | H | Cl | N | H | $CH_2C_6H_5$ | | H | 80 000 |
| 52 | H | CN | H | N | H | $CH_2C_6H_5$ | | H | 30 000 |
| 53 | H | CN | H | N | H | $CH_2CH_2C_6H_5$ | | H | 8 500 |
| 54 | H | CN | H | N | H | (R)$CH(CH_3)C_6H_5$ | | H | 9 000 |
| 55 | H | H | H | N | H | $CH_2-c-C_6H_{11}$ | | H | 3 300 |
| 56 | CN | H | H | N | H | $CH_2-c-C_6H_{11}$ | | H | 5 000 |
| 57 | Cl | H | Cl | N | H | $CH_2-c-C_6H_{11}$ | | H | 35 000 |
| 58 | H | Cl | H | N | H | $CH_2-c-C_6H_{11}$ | | H | 3 500 |
| 59 | H | CN | H | N | H | $CH_2-c-C_6H_{11}$ | | H | 35 000 |
| 60 | H | $COOCH_3$ | H | N | H | $CH_2-c-C_6H_{11}$ | | H | 1 300 |
| 61 | H | COOH | H | N | H | $CH_2-c-C_6H_{11}$ | | H | 50 |
| 62 | H | $NO_2$ | H | N | H | $CH_2-c-C_6H_{11}$ | | H | 8 000 |
| 63 | Cl | H | Cl | N | H | (S)$CH(CH_3)-c-C_6H_{11}$ | | H | 70 000 |

| Composé | $X_3$ | $X_4$ | $X_5$ | A | $R_1$ | $R_2$ | $R_3$ | $R_4$ | Pouvoir édulcorant |
|---|---|---|---|---|---|---|---|---|---|
| 64 | H | CN | H | N | H | $2-CH_3-C_6H_4$ | | H | 5 000 |
| 65 | H | CN | H | N | H | $3-CH_3-C_6H_4$ | | H | 9 000 |
| 66 | H | CN | H | N | H | $4-CH_3-C_6H_4$ | | H | 7 000 |
| 67 | H | CN | H | N | H | $1-CH_3-c-C_6H_{10}$ | | H | 100 |
| 68 | Cl | H | Cl | N | H | 1-naphthyl | | H | 30 000 |
| 69 | H | CN | H | N | H | 1-naphthyl | | H | 60 000 |
| 70 | H | CN | H | N | H | 1-indanyl | | H | 5 000 |
| 71 | H | CN | H | N | H | 1-adamantyl | | H | 3 500 |
| 72 | H | CN | H | N | H | $CH_2$-1-adamantyl | | H | 23 000 |
| 73 | Cl | H | H | N | | CN | H | H | 80 |
| 74 | H | CN | H | N | H | $N(CH_3)C_6H_5$ | | H | 18 000 |
| 75 | H | CN | H | N | H | $3,4-CH_2$-dioxy-$C_6H_3$ | | H | 5 000 |
| 76 | Cl | H | Cl | N | H | morpholino | | H | 500 |
| 77 | H | CN | H | N | H | morpholino | | H | 950 |
| 78 | H | CN | H | N | H | 2,2,4,4-tetra$CH_3$-thia-c-but-3-yl | | H | 700 |
| 79 | Cl | H | H | N | | $SO_2C_6H_5$ | H | H | 17 000 |
| 80 | H | CN | H | N | H | $3-Cl-C_6H_4$ | | H | 10 000 |

| Composé | $X_3$ | $X_4$ | $X_5$ | A | $R_1$ | $R_2$ | $R_3$ | $R_4$ | Pouvoir édulcorant |
|---|---|---|---|---|---|---|---|---|---|
| 81 | Cl | H | Cl | N | H | 3,5-diCl-$C_6H_3$ | | H | 1 000 |
| 82 | H | CN | H | N | $CH_2(CH_2)_2CH_2$ | | | H | 100 |
| 83 | H | CN | H | N | $CH_2(CH_2)_3CH_2$ | | | H | 200 |
| 84 | H | H | H | N | | $CH_2C_6H_5$ | $CH_3$ | H | 200 |
| 85 | H | CN | H | N | H | c-$C_6H_{11}$ | | $CH_3$ | 100 |
| 86 | Cl | H | Cl | N | H | (S)$CH(CH_3)C_6H_5$ | | $CH_3$ | 120 |
| 87 | H | CN | H | N | H | c-$C_8H_{15}$ | | $CH_3$ | 900 |
| 88 | $CH_3$ | H | $CH_3$ | N | $CH_3$ | $CH_2C_6H_5$ | | H | 5 000 |

REVENDICATIONS

1. Agents édulcorants caractérisés en ce qu'ils répondent à la formule générale :

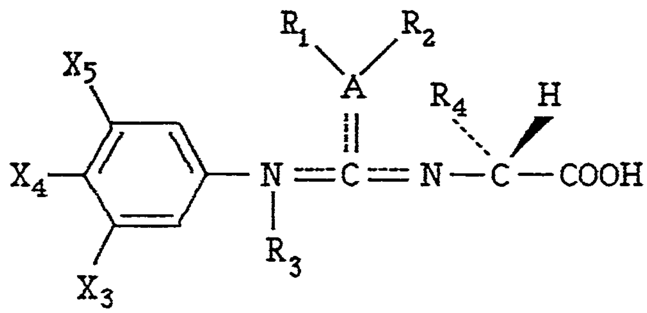

en incluant les formes tautomères et les sels physiologiquement acceptables,

- dans laquelle $X_3$, $X_4$ et $X_5$ sont choisis dans le groupe comprenant :

H,
Br,
$CF_3$,
$CF_2CF_3$,
$CH_2CF_3$,
$C_1$-$C_4$ alkyl,
$CH=NOCH_3$,
$CH=NOH$,
CHO,
$CH_2OCH_3$,
$CH_2OH$,
Cl,
CN,
$COCF_3$,
$COC_1$-$C_3$ alkyl,

$CONH_2$,
$CONHC_1-C_3$ alkyl,
$CON(C_1-C_3$ alkyl$)_2$,
$COOC_1-C_3$ alkyl,
COOH,
F,
I,
$NH_2$,
$NHC_1-C_3$ alkyl,
$N(C_1-C_3$ alkyl$)_2$,
NHCHO,
$NHCOCH_3$,
$NHCONH_2$,
$NHSO_2CH_3$,
$NO_2$,
$OC_1-C_3$ alkyl,
$OCOCH_3$,
OH,
$SC_1-C_3$ alkyl,
$SOC_1-C_3$ alkyl,
$SO_2C_1-C_3$ alkyl,
$SO_2NH_2$,
$SO_2NHC_1-C_3$ alkyl,
$SO_2N(C_1-C_3$ alkyl$)_2$ et
$SO_3H$ ;

- dans laquelle A est choisi dans le groupe comprenant N et C ;

- dans laquelle $R_1$ est un atome d'hydrogène ou un groupe hydrocarboné ou un groupe hydrocarboné modifié, ayant jusqu'à 4 atomes de carbone, saturé ou insaturé, acyclique, cyclique ou mixte, et dans lequel, dans le

groupe hydrocarboné modifié :

. 1 à 2 atomes de carbone peuvent être remplacés par 1 à 2 hétéroatomes, identiques ou différents, choisis dans le groupe comprenant les atomes d'azote, oxygène, soufre, chlore, brome et iode,

. et 1 à 3 atomes d'hydrogène peuvent être remplacés par 1 à 3 atomes de fluor ;

- dans laquelle $R_2$ est un groupe hydrocarboné ou un groupe hydrocarboné modifié, de 2 à 13 atomes de carbone, saturé ou insaturé, acyclique, cyclique ou mixte, et dans lequel, dans le groupe hydrocarboné modifié :

. 1 à 4 atomes de carbone peuvent être remplacés par 1 à 4 hétéroatomes, identiques ou différents, choisis dans le groupe comprenant les atomes d'azote, oxygène, soufre, chlore, brome et iode,

. et 1 à 5 atomes d'hydrogène peuvent être remplacés par 1 à 5 atomes de fluor ;

- dans laquelle $R_1$ et $R_2$ peuvent être fusionnés ;

- dans laquelle $R_3$ est choisi dans le groupe comprenant un atome d'hydrogène ou un groupe alkyle $C_1$-$C_3$ ;

- dans laquelle $R_4$ est choisi dans le groupe comprenant :

H et

$CH_3$ ;

à condition, quand $X_3$, $X_5$, $R_3$ et $R_4$ sont des atomes d'hydrogène,

. et quand $X_4$ est CN ou $NO_2$, que $R_1$ et $R_2$ ne soient pas CN ou $OCH_3$,

. et quand $X_4$ est H, $CF_3$, CHO, Cl, CN, $COCH_3$, F ou $NO_2$, que $R_1$ ne soit pas $NO_2$ ou $SOC_1-C_2$ alkyl et que $R_2$ ne soit pas $NO_2$, SOR, $SO_2R$, $SO_2NHR$ et $SO_2N(R)_2$, R étant un groupe alkyle, cycloalkyle ou aryle ayant jusqu'à 10 atomes de carbone, 1 ou 2 de ces atomes de carbone pouvant être remplacés par 1 ou 2 atomes de soufre ou d'oxygène.

2. Agents édulcorants selon la revendication 1, caractérisés en ce que $X_3$ et $X_5$ sont choisis dans le groupe constitué par :

H
Br,
$CF_3$,
$CH_3$,
Cl et
F.

3. Agents édulcorants selon l'une des revendications 1 et 2, caractérisés en ce que $X_4$ est choisi dans le groupe constitué par :

H,
CN,
$COOCH_3$,
F et
$NO_2$.

4. Agents édulcorants selon la revendication 1, caractérisés en ce que A est un atome d'azote.

5. Agents édulcorants selon l'une des revendications 1 à 4, caractérisés en ce que $R_1$ est choisi dans le groupe constitué par H et $CH_3$.

6. Agents édulcorants selon l'une des revendications 1 à 5, caractérisés en ce que $R_2$ est choisi dans le groupe constitué par:

$C_2$-$C_{13}$ alk(én)(yn)yl normal,
$C_3$-$C_{13}$ alk(én)(yn)yl ramifié,
$C_3$-$C_{13}$ cycloalk(én)yl,
$C_4$-$C_{13}$ alk(én)yl cycloalk(én)yl,
$C_4$-$C_{13}$ cycloalk(én)yl alk(én)yl,
$C_5$-$C_{13}$ alk(én)yl cycloalk(én)yl alk(én)yl,
$C_7$-$C_{13}$ alk(én)yl bicycloalk(én)yl,
$C_7$-$C_{13}$ bicycloalk(én)yl fusionné,
$C_8$-$C_{13}$ alk(én)yl bicycloalk(én)yl fusionné,
$C_8$-$C_{13}$ bicycloalk(én)yl fusionné alk(én)yl,
$C_9$-$C_{13}$ alk(én)yl bicycloalk(én)yl fusionné alk(én)yl,
$C_{10}$-$C_{13}$ tricycloalk(én)yl fusionné,
$C_{11}$-$C_{13}$ alk(én)yl tricycloalk(én)yl fusionné,
$C_{11}$-$C_{13}$ tricycloalk(én)yl fusionné alk(én)yl,
et $C_{13}$ alk(én)yl tricycloalk(én)yl fusionné alk(én)yl.

7. Agents édulcorants selon la revendication 6, caractérisés en ce que $R_2$ est un groupe hydrocarboné modifié dans lequel :

. jusqu'à 4 atomes de carbone peuvent être remplacés par des hétéroatomes, identiques ou différents, choisis dans le groupe constitué par :

S pour remplacer C ou $CH_2$,

N pour remplacer CH,

NH et O pour remplacer $CH_2$, et

Cl, Br et I pour remplacer $CH_3$ ;

. et jusqu'à 5 atomes d'hydrogène peuvent être remplacés par des atomes de fluor.

8. Agents édulcorants selon l'une des revendications 1 à 7, caractérisés en ce que $R_3$ est choisi dans le groupe constitué par H et $CH_3$.

9. Agents édulcorants selon l'une des revendications 1 à 8, caractérisés en ce que $R_4$ est un atome d'hydrogène.

10. Agents édulcorants selon la revendication 6, caractérisés en ce que $R_2$ est choisi dans le groupe constitué par :

*n*-$C_5H_{11}$, *n*-$C_6H_{13}$, *n*-$C_7H_{15}$,

$CH(CH_3)(CH_2)_2CH_3$, $CH(CH_3)(CH_2)_3CH_3$, $(CH_2)_3CH(CH_3)_2$, $(CH_2)_4CH(CH_3)_2$, $(CH_2)_5CH(CH_3)_2$,

$C_6H_5$, $C_6$-$C_{10}$ cycloalkyl,

$CH_2C_6H_5$, $CH_2$-*c*-$C_6H_{11}$, $CH(CH_3)C_6H_5$, $CH(CH_3)$-*c*-$C_6H_{11}$, $(CH_2)_2C_6H_5$, $(CH_2)_2$-*c*-$C_6H_{11}$, $CH(CH_3)CH_2C_6H_5$, $CH(CH_3)CH_2$-*c*-$C_6H_{11}$,

$C_6H_4(CH_3)$, $C_6$-$C_{10}$ cycloalkyl$(CH_3)$,

$CH_2C_6H_4(CH_3)$, $CH_2$-*c*-$C_6H_{10}(CH_3)$, $CH(CH_3)C_6H_4(CH_3)$, $CH(CH_3)$-*c*-$C_6H_{10}(CH_3)$,

$(CH_2)_2CH(c\text{-}C_3H_5)_2$, $(CH_2)_3CH(c\text{-}C_3H_5)_2$, $CH(CH_3)CH_2CH(c\text{-}C_3H_5)_2$, $CH(CH_3)(CH_2)_2CH(c\text{-}C_3H_5)_2$,

naphtyl, 5,6,7,8-tétrahydronaphtyl, perhydronaphtyl, indényl, indanyl,

naphtyl$(CH_3)$, 5,6,7,8-tétrahydronaphtyl$(CH_3)$, perhydronaphtyl$(CH_3)$, indényl$(CH_3)$, indanyl$(CH_3)$, fenchyl,

$CH_2$-naphtyl, $CH_2$-5,6,7,8-tétrahydronaphtyl, $CH_2$-perhydronaphtyl, $CH_2$-indényl, $CH_2$-indanyl,

$CH_2$-naphtyl$(CH_3)$, $CH_2$-5,6,7,8-tétrahydronaphtyl-$(CH_3)$, $CH_2$-perhydronaphtyl$(CH_3)$, $CH_2$-indényl$(CH_3)$, $CH_2$-indanyl$(CH_3)$,

adamantyl,

$CH_2$-adamantyl, $CH(CH_3)$adamantyl,

$CH_2$-adamantyl$(CH_3)$.

11. Agents édulcorants selon la revendication 7, caractérisés en ce que le groupe hydrocarboné modifié $R_2$ est choisi dans le groupe constitué par :

$N(CH_3)C_6H_5$, pyridinyl, pipéridyl, homopipéridyl, indolyl, indolinyl, isoindolinyl, quinolyl, isoquinolyl, pyrazinyl, pyrimidyl, indazolyl, quinoxalinyl, quinazolinyl, purinyl,

$OCH_2C_6H_5$, pyranyl, benzofuranyl, méthoxyphényl, méthyloxycarbonylphényl, 3,4-méthylènedioxyphényl, morpholinyl, benzoxazolyl, éthanamidophényl, nitrile, nitro,

thiophényl, benzothiophényl, 2,2,4,4-tétraméthylthiacyclobut-3-yl, thiazolyl, isothiazolyl, $SO_2C_6H_5$, $SO_2C_6H_{11}$, $SO_2C_7H_{13}$,

chlorophényl,

fluorophényl, trifluorométhylphényl.

12. Agents édulcorants selon la revendication 1, caractérisés en ce qu'ils répondent à la formule :

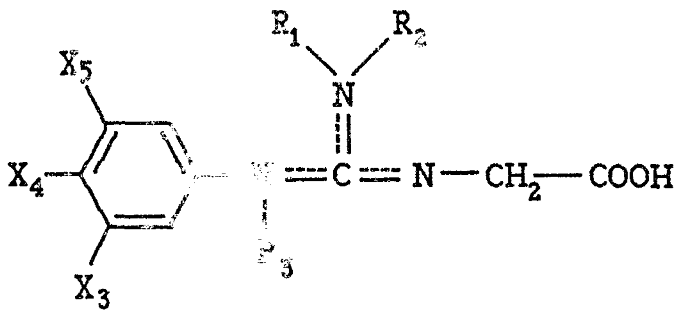

dans laquelle :

- $X_3$ et $X_5$ sont un atome d'hydrogène et $X_4$ est CN,
- $X_3$ et $X_5$ sont Cl, $CF_3$, $CH_3$ et F, et $X_4$ est H ou CN,
- $R_1$ est H ou $CH_3$,
- $R_2$ est choisi dans le groupe constitué par $CH(CH_3)C_6H_5$, $CH_2C_6H_5$, $CH(CH_3)$-c-$C_6H_{11}$, c-$C_6H_{11}$, c-$C_7H_{13}$, c-$C_8H_{15}$, c-$C_9H_{17}$, c-$C_{10}H_{19}$, $SO_2C_6H_5$ et $SO_2C_7H_{13}$,
- $R_3$ est H.

13. Agents édulcorants selon la revendication 1, caractérisés en ce que ces composés sont salifiés par des acides ou des bases inorganiques ou organiques physiologiquement acceptables.

14. Agents édulcorants selon la revendication 13, caractérisés en ce que ces composés sont salifiés sous forme d'hydrochlorure, de sels de sodium, potassium, ammonium, calcium et magnésium.

15. Procédé pour édulcorer les aliments, boissons, confiseries, pâtisseries, chewing gum, produits d'hygiène, cosmétiques, articles de toilette, produits pharmaceutiques et vétérinaires, caractérisé en ce qu'il consiste à ajouter à ces produits une quantité adéquate d'au moins un agent édulcorant selon l'une des revendications 1 à 14.

16. Préparations édulcorées suivant le procédé de la revendication 15.

17. Compositions édulcorantes caractérisées en ce qu'elles comprennent une quantité adéquate d'un ou plusieurs agents édulcorants selon l'une des revendications 1 à 14 et un support ou agent de charge choisi dans le groupe comprenant le polydextrose, l'amidon, les maltodextrines, la cellulose, la méthylcellulose, la carboxyméthylcellulose, l'hydroxyméthylcellulose, la cellulose microcristalline, l'alginate de sodium, les pectines, les gommes, le lactose, le maltose, le glucose, la leucine, le glycérol, le mannitol, le sorbitol, le bicarbonate de sodium et les acides phosphorique, citrique, tartrique, fumarique, benzoïque, sorbique et propionique, et leurs sels de sodium, potassium et calcium, et leurs mélanges.

18. Compositions édulcorantes caractérisées en ce qu'elles comprennent un agent édulcorant selon l'une des revendications 1 à 14 et au moins un autre agent édulcorant, l'autre agent édulcorant étant choisi dans le groupe comprenant le saccharose, le sirop de maïs, le fructose, l'aspartame, l'alitame, la néohespéridine dihydrochalcone, l'isomaltulose hydrogéné, le stévioside, les sucres L, la glycyrrhizine, le xylitol, l'acésulfame-K, la saccharine et ses sels de sodium, potassium ou calcium, l'acide cyclamique et ses sels de sodium, potassium ou calcium, le trichlorogalactosucrose, la monelline, la thaumatine et leurs mélanges.

REVENDICATIONS POUR

L'AUTRICHE

L'ESPAGNE

LA GRECE

REVENDICATIONS

1. Agents édulcorants caractérisés en ce qu'ils répondent à la formule générale :

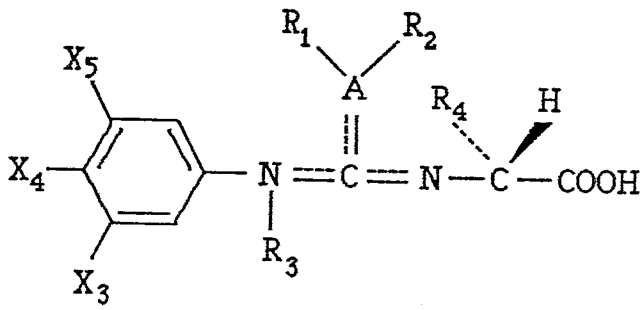

en incluant les formes tautomères et les sels physiologiquement acceptables,

- dans laquelle $X_3$, $X_4$ et $X_5$ sont choisis dans le groupe comprenant :

H,
Br,
$CF_3$,
$CF_2CF_3$,
$CH_2CF_3$,
$C_1$-$C_4$ alkyl,
$CH=NOCH_3$,
$CH=NOH$,
CHO,
$CH_2OCH_3$,
$CH_2OH$,
Cl,
CN,
$COCF_3$,
$COC_1$-$C_3$ alkyl,

$CONH_2$,
$CONHC_1-C_3$ alkyl,
$CON(C_1-C_3$ alkyl$)_2$,
$COOC_1-C_3$ alkyl,
COOH,
F,
I,
$NH_2$,
$NHC_1-C_3$ alkyl,
$N(C_1-C_3$ alkyl$)_2$,
NHCHO,
$NHCOCH_3$,
$NHCONH_2$,
$NHSO_2CH_3$,
$NO_2$,
$OC_1-C_3$ alkyl,
$OCOCH_3$,
OH,
$SC_1-C_3$ alkyl,
$SOC_1-C_3$ alkyl,
$SO_2C_1-C_3$ alkyl,
$SO_2NH_2$,
$SO_2NHC_1-C_3$ alkyl,
$SO_2N(C_1-C_3$ alkyl$)_2$ et
$SO_3H$ ;

- dans laquelle A est choisi dans le groupe comprenant N et C ;

- dans laquelle $R_1$ est un atome d'hydrogène ou un groupe hydrocarboné ou un groupe hydrocarboné modifié, ayant jusqu'à 4 atomes de carbone, saturé ou insaturé, acyclique, cyclique ou mixte, et dans lequel, dans le

groupe hydrocarboné modifié :

. 1 à 2 atomes de carbone peuvent être remplacés par 1 à 2 hétéroatomes, identiques ou différents, choisis dans le groupe comprenant les atomes d'azote, oxygène, soufre, chlore, brome et iode,

. et 1 à 3 atomes d'hydrogène peuvent être remplacés par 1 à 3 atomes de fluor ;

- dans laquelle $R_2$ est un groupe hydrocarboné ou un groupe hydrocarboné modifié, de 2 à 13 atomes de carbone, saturé ou insaturé, acyclique, cyclique ou mixte, et dans lequel, dans le groupe hydrocarboné modifié :

. 1 à 4 atomes de carbone peuvent être remplacés par 1 à 4 hétéroatomes, identiques ou différents, choisis dans le groupe comprenant les atomes d'azote, oxygène, soufre, chlore, brome et iode,

. et 1 à 5 atomes d'hydrogène peuvent être remplacés par 1 à 5 atomes de fluor ;

- dans laquelle $R_1$ et $R_2$ peuvent être fusionnés ;

- dans laquelle $R_3$ est choisi dans le groupe comprenant un atome d'hydrogène ou un groupe alkyle $C_1$-$C_3$ ;

- dans laquelle $R_4$ est choisi dans le groupe comprenant :

H et

$CH_3$ ;

à condition, quand $X_3$, $X_5$, $R_3$ et $R_4$ sont des atomes d'hydrogène,

. et quand $X_4$ est CN ou $NO_2$, que $R_1$ et $R_2$ ne soient pas CN ou $OCH_3$,

. et quand $X_4$ est H, $CF_3$, CHO, Cl, CN, $COCH_3$, F ou $NO_2$, que $R_1$ ne soit pas $NO_2$ ou $SOC_1$-$C_2$ alkyl et que $R_2$ ne soit pas $NO_2$, SOR, $SO_2R$, $SO_2NHR$ et $SO_2N(R)_2$, R étant un groupe alkyle, cycloalkyle ou aryle ayant jusqu'à 10 atomes de carbone, 1 ou 2 de ces atomes de carbone pouvant être remplacés par 1 ou 2 atomes de soufre ou d'oxygène.

2. Agents édulcorants selon la revendication 1, caractérisés :

- en ce que $X_3$ et $X_5$ sont choisis dans le groupe constitué par :

H
Br,
$CF_3$,
$CH_3$,
Cl et
F ;

- et en ce que $X_4$ est choisi dans le groupe constitué par :

H,
CN,
$COOCH_3$,
F et
$NO_2$.

3. Agents édulcorants selon la revendication 1, caractérisés en ce que A est un atome d'azote.

4. Agents édulcorants selon l'une des revendications 1 à 3, caractérisés en ce que $R_1$ et $R_3$ sont choisis dans le groupe constitué par H et $CH_3$, et en ce que $R_4$ est un atome d'hydrogène.

5. Agents édulcorants selon l'une des revendications 1 à 4, caractérisés en ce que $R_2$ est choisi dans le groupe constitué par:

$C_2$-$C_{13}$ alk(én)(yn)yl normal,
$C_3$-$C_{13}$ alk(én)yl ramifié,
$C_3$-$C_{13}$ cycloalk(én)yl,
$C_4$-$C_{13}$ alk(én)yl cycloalk(én)yl,
$C_4$-$C_{13}$ cycloalk(én)yl alk(én)yl,
$C_5$-$C_{13}$ alk(én)yl cycloalk(én)yl alk(én)yl,
$C_7$-$C_{13}$ alk(én)yl bicycloalk(én)yl,
$C_7$-$C_{13}$ bicycloalk(én)yl fusionné,
$C_8$-$C_{13}$ alk(én)yl bicycloalk(én)yl fusionné,
$C_8$-$C_{13}$ bicycloalk(én)yl fusionné alk(én)yl,
$C_9$-$C_{13}$ alk(én)yl bicycloalk(én)yl fusionné alk(én)yl,
$C_{10}$-$C_{13}$ tricycloalk(én)yl fusionné,
$C_{11}$-$C_{13}$ alk(én)yl tricycloalk(én)yl fusionné,
$C_{11}$-$C_{13}$ tricycloalk(én)yl fusionné alk(én)yl,
et $C_{13}$ alk(én)yl tricycloalk(én)yl fusionné alk(én)yl.

6. Agents édulcorants selon la revendication 5, caractérisés en ce que $R_2$ est un groupe hydrocarboné modifié dans lequel :

. jusqu'à 4 atomes de carbone peuvent être remplacés par des hétéroatomes, identiques ou différents, choisis dans le groupe constitué par :

S pour remplacer C ou $CH_2$,

N pour remplacer CH,

NH et O pour remplacer $CH_2$, et

Cl, Br et I pour remplacer $CH_3$ ;

. et jusqu'à 5 atomes d'hydrogène peuvent être remplacés par des atomes de fluor.

7. Agents édulcorants selon les revendications 5 et 6 caractérisés :

- en ce que le groupe hydrocarboné $R_2$ est choisi dans le groupe constitué par :

$n$-$C_5H_{11}$, $n$-$C_6H_{13}$, $n$-$C_7H_{15}$,

$CH(CH_3)(CH_2)_2CH_3$, $CH(CH_3)(CH_2)_3CH_3$, $(CH_2)_3CH(CH_3)_2$, $(CH_2)_4CH(CH_3)_2$, $(CH_2)_5CH(CH_3)_2$,

$C_6H_5$, $C_6$-$C_{10}$ cycloalkyl,

$CH_2C_6H_5$, $CH_2$-$c$-$C_6H_{11}$, $CH(CH_3)C_6H_5$, $CH(CH_3)$-$c$-$C_6H_{11}$, $(CH_2)_2C_6H_5$, $(CH_2)_2$-$c$-$C_6H_{11}$, $CH(CH_3)CH_2C_6H_5$, $CH(CH_3)CH_2$-$c$-$C_6H_{11}$,

$C_6H_4(CH_3)$, $C_6$-$C_{10}$ cycloalkyl$(CH_3)$,

$CH_2C_6H_4(CH_3)$, $CH_2$-$c$-$C_6H_{10}(CH_3)$, $CH(CH_3)C_6H_4(CH_3)$, $CH(CH_3)$-$c$-$C_6H_{10}(CH_3)$,

$(CH_2)_2CH(c$-$C_3H_5)_2$, $(CH_2)_3CH(c$-$C_3H_5)_2$, $CH(CH_3)CH_2CH$-$(c$-$C_3H_5)_2$, $CH(CH_3)(CH_2)_2CH(c$-$C_3H_5)_2$,

naphtyl, 5,6,7,8-tétrahydronaphtyl, perhydronaphtyl, indényl, indanyl,

naphtyl($CH_3$), 5,6,7,8-tétrahydronaphtyl($CH_3$), perhydronaphtyl($CH_3$), indényl($CH_3$), indanyl($CH_3$), fenchyl,

$CH_2$-naphtyl, $CH_2$-5,6,7,8-tétrahydronaphtyl, $CH_2$-perhydronaphtyl, $CH_2$-indényl, $CH_2$-indanyl,

$CH_2$-naphtyl($CH_3$), $CH_2$-5,6,7,8-tétrahydronaphtyl-($CH_3$), $CH_2$-perhydronaphtyl($CH_3$), $CH_2$-indényl($CH_3$), $CH_2$-indanyl($CH_3$),

adamantyl,

$CH_2$-adamantyl, $CH(CH_3)$adamantyl,

$CH_2$-adamantyl($CH_3$);

- et en ce que le groupe hydrocarboné modifié $R_2$ est choisi dans le groupe constitué par :

$N(CH_3)C_6H_5$, pyridinyl, pipéridyl, homopipéridyl, indolyl, indolinyl, isoindolinyl, quinolyl, isoquinolyl, pyrazinyl, pyrimidyl, indazolyl, quinoxalinyl, quinazolinyl, purinyl,

$OCH_2C_6H_5$, pyranyl, benzofuranyl, méthoxyphényl, méthyloxycarbonylphényl, 3,4-méthylènedioxyphényl, morpholinyl, benzoxazolyl, éthanamidophényl, nitrile, nitro,

thiophényl, benzothiophényl, 2,2,4,4-tétraméthyl-thiacyclobut-3-yl, thiazolyl, isothiazolyl, $SO_2C_6H_5$, $SO_2C_6H_{11}$, $SO_2C_7H_{13}$,

chlorophényl,

fluorophényl, trifluorométhylphényl.

8. Agents édulcorants selon la revendication 1, caractérisés en ce qu'ils répondent à la formule :

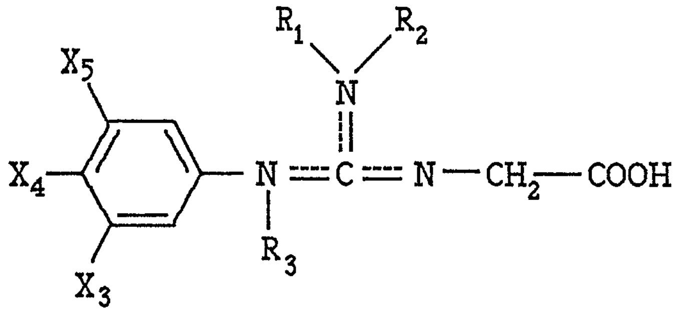

dans laquelle :

- $X_3$ et $X_5$ sont un atome d'hydrogène et $X_4$ est CN,
- $X_3$ et $X_5$ sont Cl, $CF_3$, $CH_3$ et F, et $X_4$ est H ou CN,
- $R_1$ est H ou $CH_3$,
- $R_2$ est choisi dans le groupe constitué par $CH(CH_3)C_6H_5$, $CH_2C_6H_5$, $CH(CH_3)$-*c*-$C_6H_{11}$, *c*-$C_6H_{11}$, *c*-$C_7H_{13}$, *c*-$C_8H_{15}$, *c*-$C_9H_{17}$, *c*-$C_{10}H_{19}$, $SO_2C_6H_5$ et $SO_2C_7H_{13}$,
- $R_3$ est H.

9. Agents édulcorants selon la revendication 1, caractérisés en ce que ces composés sont salifiés par des acides ou des bases inorganiques ou organiques physiologiquement acceptables.

10. Procédé pour édulcorer les aliments, boissons, confiseries, pâtisseries, chewing gum, produits d'hygiène, cosmétiques, articles de toilette, produits pharmaceutiques et vétérinaires, caractérisé en ce qu'il consiste à ajouter à ces produits une quantité adéquate d'au moins un agent édulcorant selon l'une des revendications 1 à 9.

11. Compositions édulcorantes caractérisées en ce qu'elles comprennent une quantité adéquate d'un ou plusieurs agents édulcorants selon l'une des revendications 1 à 9 et un support ou agent de charge choisi dans le groupe comprenant le polydextrose, l'amidon, les maltodextrines, la cellulose, la méthylcellulose, la carboxyméthylcellulose, l'hydroxy-méthylcellulose, la cellulose microcristalline, l'alginate de sodium, les pectines, les gommes, le lactose, le maltose, le glucose, la leucine, le glycérol, le mannitol, le sorbitol, le bicarbonate de sodium et les acides phosphorique, citrique, tartrique, fumarique, benzoïque, sorbique et propionique, et leurs sels de sodium, potassium et calcium, et leurs mélanges.

12. Compositions édulcorantes caractérisées en ce qu'elles comprennent un agent édulcorant selon l'une des revendications 1 à 9 et au moins un autre agent édulcorant, l'autre agent édulcorant étant choisi dans le groupe comprenant le saccharose, le sirop de maïs, le fructose, l'aspartame, l'alitame, la néohespéridine dihydrochalcone, l'isomaltulose hydrogéné, le stévioside, les sucres L, la glycyrrhizine, le xylitol, l'acésulfame-K, la saccharine et ses sels de sodium, potassium ou calcium, l'acide cyclamique et ses sels de sodium, potassium ou calcium, le trichlorogalactosucrose, la monelline, la thaumatine et leurs mélanges.

13. Procédé pour préparer un agent édulcorant de formule générale :

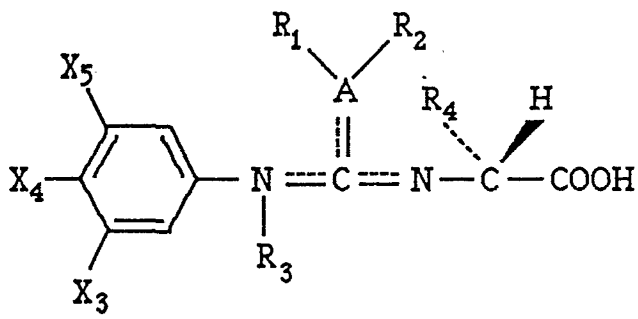

en incluant les formes tautomères et les sels physiologiquement acceptables,

- dans laquelle $X_3$, $X_4$ et $X_5$ sont choisis dans le groupe comprenant :

H,
Br,
$CF_3$,
$CF_2CF_3$,
$CH_2CF_3$,
$C_1$-$C_4$ alkyl,
$CH=NOCH_3$,
CH=NOH,
CHO,
$CH_2OCH_3$,
$CH_2OH$,
Cl,
CN,
$COCF_3$,
$COC_1$-$C_3$ alkyl,
$CONH_2$,
$CONHC_1$-$C_3$ alkyl,

$CON(C_1-C_3\ alkyl)_2$,
$COOC_1-C_3$ alkyl,
COOH,
F,
I,
$NH_2$,
$NHC_1-C_3$ alkyl,
$N(C_1-C_3\ alkyl)_2$,
NHCHO,
$NHCOCH_3$,
$NHCONH_2$,
$NHSO_2CH_3$,
$NO_2$,
$OC_1-C_3$ alkyl,
$OCOCH_3$,
OH,
$SC_1-C_3$ alkyl,
$SOC_1-C_3$ alkyl,
$SO_2C_1-C_3$ alkyl,
$SO_2NH_2$,
$SO_2NHC_1-C_3$ alkyl,
$SO_2N(C_1-C_3\ alkyl)_2$ et
$SO_3H$ ;

- dans laquelle A est choisi dans le groupe comprenant N et C ;

- dans laquelle $R_1$ est un atome d'hydrogène ou un groupe hydrocarboné ou un groupe hydrocarboné modifié, ayant jusqu'à 4 atomes de carbone, saturé ou insaturé, acyclique, cyclique ou mixte, et dans lequel, dans le groupe hydrocarboné modifié :

. 1 à 2 atomes de carbone peuvent être remplacés par 1 à 2 hétéroatomes, identiques ou différents, choisis dans le groupe comprenant les atomes d'azote, oxygène, soufre, chlore, brome et iode,

. et 1 à 3 atomes d'hydrogène peuvent être remplacés par 1 à 3 atomes de fluor ;

- dans laquelle $R_2$ est un groupe hydrocarboné ou un groupe hydrocarboné modifié, de 2 à 13 atomes de carbone, saturé ou insaturé, acyclique, cyclique ou mixte, et dans lequel, dans le groupe hydrocarboné modifié :

. 1 à 4 atomes de carbone peuvent être remplacés par 1 à 4 hétéroatomes, identiques ou différents, choisis dans le groupe comprenant les atomes d'azote, oxygène, soufre, chlore, brome et iode,

. et 1 à 5 atomes d'hydrogène peuvent être remplacés par 1 à 5 atomes de fluor ;

- dans laquelle $R_1$ et $R_2$ peuvent être fusionnés ;

- dans laquelle $R_3$ est choisi dans le groupe comprenant un atome d'hydrogène ou un groupe alkyle $C_1-C_3$ ;

- dans laquelle $R_4$ est choisi dans le groupe comprenant :

H et

$CH_3$ ;

à condition, quand $X_3$, $X_5$, $R_3$ et $R_4$ sont des atomes d'hydrogène,

. et quand $X_4$ est CN ou $NO_2$, que $R_1$ et $R_2$ ne soient pas CN ou $OCH_3$,

. et quand $X_4$ est H, $CF_3$, CHO, Cl, CN, $COCH_3$, F ou $NO_2$, que $R_1$ ne soit pas $NO_2$ ou $SOC_1$-$C_2$ alkyl et que $R_2$ ne soit pas $NO_2$, SOR, $SO_2R$, $SO_2NHR$ et $SO_2N(R)_2$, R étant un groupe alkyle, cycloalkyle ou aryle ayant jusqu'à 10 atomes de carbone, 1 ou 2 de ces atomes de carbone pouvant être remplacés par 1 ou 2 atomes de soufre ou d'oxygène,

<u>caractérisé</u> en ce qu'il consiste à condenser un dérivé thiouréido ou thioamido de formule générale :

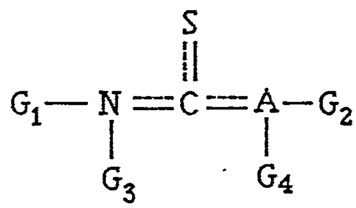

avec un composé de formule générale :

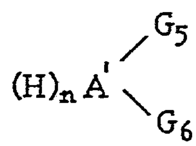

dans lesquelles :

. $G_1$ est $(X_3X_4X_5)C_6H_2$ ou $HOOCCHR_4$,

. $G_2$ est $R_2$ ou $HOOCCHR_4$,

. $G_3$, $G_4$ et $G_5$ sont H, $R_1$ ou $R_3$,

. $G_6$ est $(X_3X_4X_5)C_6H_2$, $R_2$ ou $HOOCCHR_4$,

. A' est N ou C,

. n est égal à 1 quand A' est N, et est égal à 2 quand A' est C ;

et dans lesquelles :

. $G_1$, $G_2$ et $G_6$ ne sont pas simultanément identiques,

. A et A' ne sont pas simultanément un atome de carbone,

. A' est N et $G_3$, $G_4$ et $G_5$ sont H lorsque $G_1$ ou $G_2$ ou $G_6$ sont $HOOCCHR_4$ ;

et dans lesquelles $X_3$, $X_4$ ,$X_5$, $R_1$, $R_2$, $R_3$, $R_4$ et A correspondent aux définitions données précédemment.

14. Procédé selon la revendication 13, caractérisé en ce que :

- $X_3$ et $X_5$ sont choisis dans le groupe constitué par :

H
Br,
$CF_3$,
$CH_3$,
Cl et
F ;

- $X_4$ est choisi dans le groupe constitué par :

H,
CN,
$COOCH_3$,
F et
$NO_2$ ;

- A est un atome d'azote ;

- $R_1$ est choisi dans le groupe constitué par H et $CH_3$ ;

- $R_2$ est choisi dans le groupe constitué par:

$C_2$-$C_{13}$ alk(én)(yn)yl normal,
$C_3$-$C_{13}$ alk(én)yl ramifié,
$C_3$-$C_{13}$ cycloalk(én)yl,
$C_4$-$C_{13}$ alk(én)yl cycloalk(én)yl,

$C_4$-$C_{13}$ cycloalk(én)yl alk(én)yl,
$C_5$-$C_{13}$ alk(én)yl cycloalk(én)yl alk(én)yl,
$C_7$-$C_{13}$ alk(én)yl bicycloalk(én)yl,
$C_7$-$C_{13}$ bicycloalk(én)yl fusionné,
$C_8$-$C_{13}$ alk(én)yl bicycloalk(én)yl fusionné,
$C_8$-$C_{13}$ bicycloalk(én)yl fusionné alk(én)yl,
$C_9$-$C_{13}$ alk(én)yl bicycloalk(én)yl fusionné alk(én)yl,
$C_{10}$-$C_{13}$ tricycloalk(én)yl fusionné,
$C_{11}$-$C_{13}$ alk(én)yl tricycloalk(én)yl fusionné,
$C_{11}$-$C_{13}$ tricycloalk(én)yl fusionné alk(én)yl,
et $C_{13}$ alk(én)yl tricycloalk(én)yl fusionné alk(én)yl ;

dans lesquels, sous la forme d'un groupe hydrocarboné modifié :

. jusqu'à 4 atomes de carbone peuvent être remplacés par des hétéroatomes, identiques ou différents, choisis dans le groupe constitué par :
S pour remplacer C ou $CH_2$,
N pour remplacer CH,
NH, et O pour remplacer $CH_2$, et
Cl, Br et I pour remplacer $CH_3$ ;

. et jusqu'à 5 atomes d'hydrogène peuvent être remplacés par des atomes de fluor ;

- $R_3$ est choisi dans le groupe constitué par H et $CH_3$;

- $R_4$ est un atome d'hydrogène.

15. Procédé selon la revendication 14, caractérisé :

- en ce que le groupe hydrocarboné $R_2$ est choisi dans le groupe constitué par :

*n*-$C_5H_{11}$, *n*-$C_6H_{13}$, *n*-$C_7H_{15}$,

$CH(CH_3)(CH_2)_2CH_3$, $CH(CH_3)(CH_2)_3CH_3$, $(CH_2)_3CH(CH_3)_2$, $(CH_2)_4CH(CH_3)_2$, $(CH_2)_5CH(CH_3)_2$,

$C_6H_5$, $C_6$-$C_{10}$ cycloalkyl,

$CH_2C_6H_5$, $CH_2$-*c*- $C_6H_{11}$, $CH(CH_3)C_6H_5$, $CH(CH_3)$-*c*-$C_6H_{11}$, $(CH_2)_2C_6H_5$, $(CH_2)_2$-*c*-$C_6H_{11}$, $CH(CH_3)CH_2C_6H_5$, $CH(CH_3)CH_2$-*c*-$C_6H_{11}$,

$C_6H_4(CH_3)$, $C_6$-$C_{10}$ cycloalkyl$(CH_3)$,

$CH_2C_6H_4(CH_3)$, $CH_2$-*c*-$C_6H_{10}(CH_3)$, $CH(CH_3)C_6H_4(CH_3)$, $CH(CH_3)$-*c*-$C_6H_{10}(CH_3)$,

$(CH_2)_2CH$(*c*-$C_3H_5)_2$, $(CH_2)_3CH$(*c*-$C_3H_5)_2$, $CH(CH_3)CH_2CH$-(*c*-$C_3H_5)_2$, $CH(CH_3)(CH_2)_2CH$(*c*-$C_3H_5)_2$,

naphtyl, 5,6,7,8-tétrahydronaphtyl, perhydronaphtyl, indényl, indanyl,

naphtyl$(CH_3)$, 5,6,7,8-tétrahydronaphtyl$(CH_3)$, perhydronaphtyl$(CH_3)$, indényl$(CH_3)$, indanyl$(CH_3)$, fenchyl,

$CH_2$-naphtyl, $CH_2$-5,6,7,8-tétrahydronaphtyl, $CH_2$-perhydronaphtyl, $CH_2$-indényl, $CH_2$-indanyl,

$CH_2$-naphtyl$(CH_3)$, $CH_2$-5,6,7,8-tétrahydronaphtyl-$(CH_3)$, $CH_2$-perhydronaphtyl$(CH_3)$, $CH_2$-indényl$(CH_3)$, $CH_2$-indanyl$(CH_3)$,

adamantyl,

$CH_2$-adamantyl, $CH(CH_3)$adamantyl,

$CH_2$-adamantyl$(CH_3)$;

- et en ce que le groupe hydrocarboné modifié $R_2$ est choisi dans le groupe constitué par :

$N(CH_3)C_6H_5$, pyridinyl, pipéridyl, homopipéridyl, indolyl, indolinyl, isoindolinyl, quinolyl, isoquinolyl, pyrazinyl, pyrimidyl, indazolyl, quinoxalinyl, quinazolinyl, purinyl,

$OCH_2C_6H_5$, pyranyl, benzofuranyl, méthoxyphényl, méthyloxycarbonylphényl, 3,4-méthylènedioxyphényl, morpholinyl, benzoxazolyl, éthanamidophényl, nitrile, nitro,

thiophényl, benzothiophényl, 2,2,4,4-tétraméthylthiacyclobut-3-yl, thiazolyl, isothiazolyl, $SO_2C_6H_5$, $SO_2C_6H_{11}$, $SO_2C_7H_{13}$,

chlorophényl,

fluorophényl, trifluorométhylphényl.

16. Procédé selon la revendication 13, caractérisé en ce que l'on prépare un composé de formule générale :

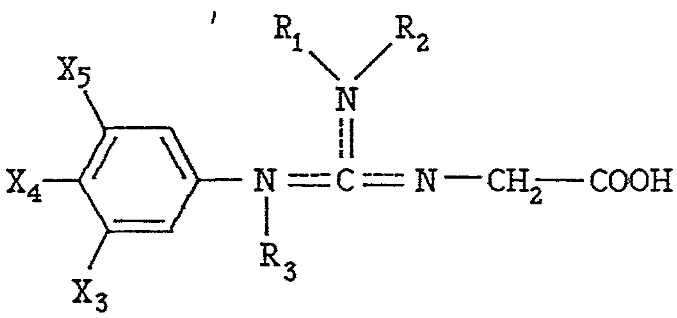

dans laquelle :

- $X_3$ et $X_5$ sont un atome d'hydrogène et $X_4$ est CN,
- $X_3$ et $X_5$ sont Cl, $CF_3$, $CH_3$ et F, et $X_4$ est H ou CN,
- $R_1$ est H ou $CH_3$,
- $R_2$ est choisi dans le groupe constitué par $CH(CH_3)C_6H_5$, $CH_2C_6H_5$, $CH(CH_3)$-$c$-$C_6H_{11}$, $c$-$C_6H_{11}$, $c$-$C_7H_{13}$, $c$-$C_8H_{15}$, $c$-$C_9H_{17}$, $c$-$C_{10}H_{19}$, $SO_2C_6H_5$ et $SO_2C_7H_{13}$,
- $R_3$ est H.

17. Procédé selon la revendication 13, caractérisé en ce qu'il consiste à obtenir le dérivé thiouréido par l'action d'un isothiocyanate sur une amine selon l'une des réactions :

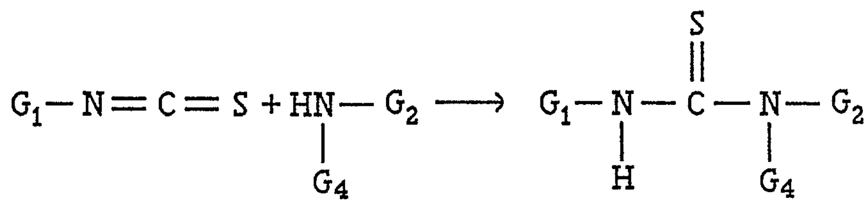

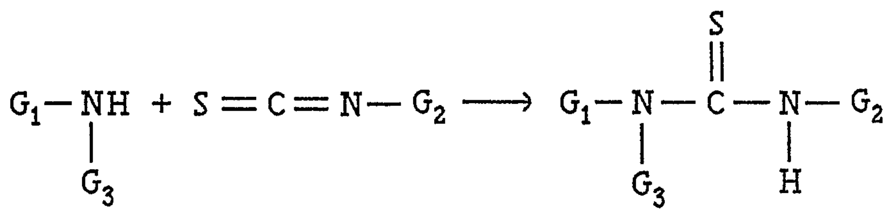

18. Procédé selon la revendication 13, caractérisé en ce qu'il consiste à activer l'atome de soufre du dérivé thiouréido ou thioamido en le transformant en un groupe S-alkyle ou $SO_3H$, ou en le remplaçant par un groupe O-alkyl, O-$SO_2$aryl ou un atome d'halogène.

19. Procédé selon les revendications 13 et 18, caractérisé en ce qu'il consiste à activer le dérivé thiouréido ou thioamido en le transformant en un dérivé S-méthylisothiouréido ou S-méthylisothioamido de formule générale :

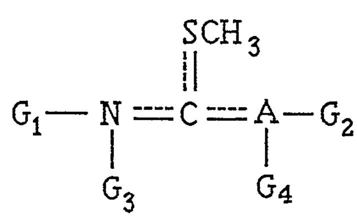

par action d'un agent alkylant choisi dans le groupe comprenant l'iodure de méthyle et le diméthyl sulfate.

20. Procédé selon la revendication 19, caractérisé en ce que le dérivé S-méthylisothiouréido correspond à la formule générale :

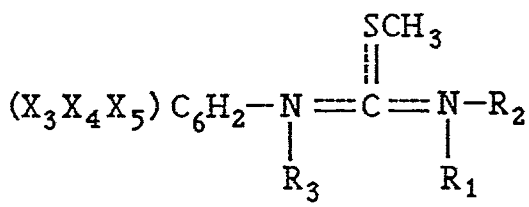

21. Procédé selon la revendication 13, caractérisé en ce qu'il consiste à activer le dérivé thiouréido, lorsque A est N et lorsque $G_3$ et $G_4$ sont un atome d'hydrogène, en le transformant en un intermédiaire carbodiimide :

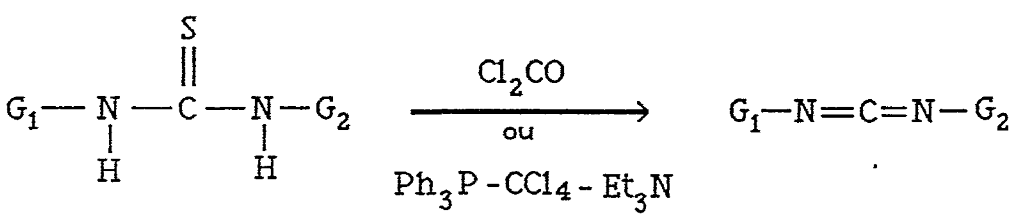


par traitement par le phosgène ou par action d'un mélange équimoléculaire de $(C_6H_5)_3P$ - $CCl_4$ - $(C_2H_5)_3N$.

22. Procédé selon la revendication 13, caractérisé en ce que, lorsque A et A' sont N et qu'au moins l'un des radicaux $G_3$, $G_4$ ou $G_5$ est un atome d'hydrogène, le dérivé thiouréido est condensé avec l'amine par action de dicyclohexylcarbodiimide (DCC) comme agent de couplage, suivant la réaction :